# EUROPÄISCHE PATENTSCHRIFT

(11) **EP 2 736 542 B1**
(45) Veröffentlichungstag und Bekanntmachung des Hinweises auf die Patenterteilung: **06.05.2015**
(21) Anmeldenummer: 12756104.1
(22) Anmeldetag: 31.08.2012
(51) Int. Cl.: A61L 15/18, A61L 15/60, A61F 13/15, A61L 15/32, A61L 15/42

(54) **MEHRSCHICHTIGE WUNDAUFLAGE ENTHALTEND EIN HYDROCOLLOID UND AKTIVKOHLE**
MULTI-LAYERED WOUND DRESSING CONTAINING AN HYDROCOLLOID AND ACTIVATED CARBON
PANSEMENT MULTICOUCHE CONTENANT UN HYDROCOLLOÏDE ET DU CHARBON ACTIF

(30) Priorität: 02.09.2011 DE 102011112102; 08.12.2011 DE 102011120491
(43) Veröffentlichungstag der Anmeldung: 04.06.2014
(73) Patentinhaber: Blücher GmbH, 40699 Erkrath (DE)
(72) Erfinder: VON BLÜCHER, Hasso, 40699 Erkrath (DE); SCHÖNFELD, Raik, 30629 Hannover (DE); PALLASKE, Frank, 09355 Gersdorf (DE)
(74) Vertreter: Von Rohr Patentanwälte Partnerschaft mbB
(86) Internationale Anmeldenummer: PCT/EP2012/003659
(87) Internationale Veröffentlichungsnummer: WO 2013/029796

(56) Entgegenhaltungen:
- DE-U1-202006 016 821
- US-A1- 2005 137 272

## Beschreibung

Die vorliegende Erfindung betrifft das medizinische Gebiet der Wundversorgung bzw. Wundheilung, insbesondere auf Basis einer lokalen Applikation einer Wundauflage.

Insbesondere betrifft die vorliegende Erfindung eine Wundauflage, welche sich vorzugsweise zur therapeutischen Wundversorgung des menschlichen oder tierischen Körpers eignet.

Weiterhin betrifft die vorliegende Erfindung die Verwendung der Wundauflage nach der Erfindung zur therapeutischen Wundversorgung.

Unter einer Wunde wird gemäß medizinischer Definition und im Rahmen der vorliegenden Erfindung eine Unterbrechung des Zusammenhangs von Körpergeweben mit oder ohne Substanzverlust verstanden, wobei eine solche Unterbrechung im Allgemeinen durch mechanische Verletzungen oder physikalisch bedingte Zellschädigungen verursacht wird.

Wunden werden nach ihrem Auslöser in verschiedene Typen klassifiziert. So werden unter einer mechanischen Wunde durch äußere Gewalteinwirkung erzeugte Wunden zusammengefasst, wobei es sich hierbei vor allem um Schnitt- und Stichwunden, Quetsch-, Platz-, Ritz- und Schürfwunden handelt. Eine weitere Form der Wunden bezeichnet thermische Wunden, welche durch Einwirkung von extremer Hitze oder Kälte entstehen. Chemische Wunden hingegen werden durch Einwirkung von Chemikalien, insbesondere durch Verätzung mit Säuren oder Laugen, herbeigeführt. Als strahlenbedingte Wunden werden solche Gewebeunterbrechungen bzw. -zerstörungen bezeichnet, welche unter Einwirkung aktinischer Strahlung, z. B. Ultraviolettstrahlung, und/oder ionisierender Strahlung entstehen.

Darüber hinaus unterscheidet man in Abhängigkeit vom physiologischen Zustand auch zwischen nekrotisierenden, infizierten und/oder chronischen oder akuten Wunden.

Für weitergehende Einzelheiten zum Begriff der Wunde kann auf Pschyrembel-Klinisches Wörterbuch, 257. Auflage, 1994, Verlag de Gruyter, Berlin/New York, Seite 1679, Stichwort "Wunde", verwiesen werden.

Die Wundheilung, d. h. die physiologischen Vorgänge zur Regeneration des zerstörten Gewebes bzw. zum Verschluss der Wunde, erfolgt insbesondere durch die Neubildung von Bindegewebe sowie Kapillaren zur Durchblutung, wobei im Allgemeinen drei Phasen durchlaufen werden. Dieser Prozess kann sich je nach Größe und Tiefe der Wunde über einen Zeitraum von bis zu vier Wochen erstrecken.

In der ersten Phase - auch als Latenz- bzw. Entzündungsphase bezeichnet - innerhalb der ersten Stunden nach eingetretener Verwundung erfolgt zunächst die Exsudation von Körperflüssigkeiten, insbesondere von Blut, zur Befreiung des Wundspalts von Fremdkörpern, Keimen und abgestorbenem Gewebe. Im Anschluss wird durch Gerinnung des ausgetretenen Bluts ein Wundschorf ausgebildet, welcher die Wunde nach außen vor dem Eindringen von Keimen und Fremdkörpern schützt. Nach Bildung des Wundschorfes beginnt die resorptive Phase der Latenzphase, in welcher auch eine katabole Autolyse erfolgt, d. h. Makrophagen wandern in das Wundgewebe ein und phagozytieren das koagulierte Blut im Wundspalt. Möglicherweise eingedrungene Fremdkörper oder Mikroorganismen werden in dieser Phase degradiert, was mit leichten bis mittleren Entzündungssymptomen verbunden sein kann. Zudem beginnt in der resorptiven Phase der Aufbau des basalen Epithels sowie von Granulationsgewebe. Nach etwa einem bis drei Tagen nach Auslösung der Verletzung ist im Allgemeinen die Latenzphase abgeschlossen, und die Latenzphase geht in die zweite Phase, die so genannte Proliferations- bzw. Granulationsphase, über, welche im Allgemeinen vom vierten bis zum siebten Tag nach der Verletzung andauert. Dabei beginnt die anabole Reparation, welche insbesondere die Bildung von Kollagen durch Fibroblasten bezeichnet. In der Reparations- bzw. Epithelisierungsphase, welche etwa ab dem achten Tag nach Entstehung der Wunde beginnt, entsteht endgültiges Narbengewebe, und das Plattenepithel der Haut erneuert sich. Das entstehende Narbengewebe besitzt weder Talg- noch Schweißdrüsen und erscheint weiß-perlmuttartig auf der Haut. Im Gegensatz zu unversehrtem Gewebe ist das Kollagen im Narbengewebe nicht mehr komplex verknüpft, sondern parallel angeordnet.

Für weitergehende Informationen zum Begriff der Wundheilung kann auf Pschyrembel - Klinisches Wörterbuch, 257. Auflage, 1994, Verlag de Gruyter, Berlin/New York, Seite 1670, Stichwort "Wundheilung" verwiesen werden.

Im Stand der Technik sind zahlreiche medizinische Artikel bzw. Gegenstände und Therapiemaßnahmen bekannt, welche einer Unterstützung oder Beschleunigung der Wundheilung dienen. Dennoch kommt es oft zu Komplikationen oder zu einer erschwerten Wundheilung, insbesondere wenn die Wunde sehr großflächig ist oder aber zahlreiche Gewebeschichten betroffen sind.

Eine relativ häufig auftretende Komplikation bei der Wundheilung stellen durch Bakterien, Pilze oder Viren ausgelöste Wundinfektionen dar, welche insbesondere auf eine mangelnde Wundhygiene oder das vermehrte Auftreten von Keimen, wie es in Krankenhäusern oftmals der Fall ist, zurückzuführen sind. Durch Kontamination mit verschiedenen Mikroorganismen kann es zu insbesondere bakteriellen Infektionen der Wunde kommen, wobei infolge der Infektion klassische Zeichen einer lokalen Entzündung auftreten, wie Schmerzen, Schwellungen, Rötungen und Überwärmung. Im schlimmsten Fall kann es jedoch auch infolge einer phlegmonösen - d. h. flächendeckenden - Ausbreitung zu einer Allgemeininfektion bzw. lebensbedrohlichen Sepsis mit hohem Fieber und Schüttelfrost kommen. Bei der Erzeugung von Wundinfektionen spielen insbesondere die so genannten Krankenhaus- bzw. Hospitalkeime, wie *Proteus mirabilis, Pseudomonas aeruginosa, Staphylococcus epidermidis, Staphylococcus aureus* und *Escherichia coli,* eine bedeutende Rolle. Besonders problematisch an solchen Infektionen mit Hospitalkeimen sind die von den jeweiligen Stämmen im Laufe der Zeit erworbenen zahlreichen Antibiotikaresistenzen, infolge derer sich auftretende Infektionen nur äußerst schlecht behandeln lassen, vor allem bei Patienten mit ohnehin geschwächtem Immunsystem. Insbesondere von *Staphylococcus aureus* existieren zahlreiche Stämme, welche gegenüber sämtlichen auf dem Markt erhältlichen beta-Lactam-Antibiotika, wie Methicillin und Oxacillin, sowie diversen weiteren Antibiotika-Klassen, wie Glykopeptid-Antibiotika, Sulfonamiden, Chinolonen oder Tetrazyklinen, Resistenzen besitzen. Bei Infektionen mit derartigen Keimen muss folglich eine von der Verabreichung von Antibiotika unabhängige Therapie zur Vermeidung einer systemischen Ausbreitung des Erregers im Körper erfolgen. An solchen Therapiekonzepten besteht im Stand der Technik jedoch noch ein gravierender Mangel, so dass die Todesrate durch multiresistente Hospitalkeime die durch saisonale Influenza bedingte Mortalitätsrate übersteigt.

Ein weiteres Problem bei der Wundheilung kann die Bildung von Nekrosen darstellen, wobei es hierbei zum pathologischen Absterben von Zellen am lebenden Organismus kommt. Im Falle von Nekrosen erfordert eine erfolgreiche Therapie meist ein Debridement, welches die Exzision des abgestorbenen Gewebes bezeichnet und der Anregung der Wundheilung sowie der Vermeidung der Ausbreitung einer Wundinfektion dient. Ein Debridement kann sowohl chirurgisch z. B. mittels Skalpell und Ringkürette als auch enzymatisch, autolytisch oder biochirurgisch erfolgen. Eine solche Behandlung ist jedoch für den Patienten meist mit starken Schmerzen verbunden, insbesondere im Falle eines chirurgischen Debridements.

Besonders intensive und sorgfältige Therapiemaßnahmen sind erforderlich, wenn eine akute Wunde in eine chronische Wunde übergeht. Eine Wunde gilt dann als chronisch, wenn ihre Heilung innerhalb eines Zeitraums von vier bis acht Wochen nach der Entstehung nicht abgeschlossen ist. Chronische Wunden entstehen meistens nicht zufällig, sondern treten häufig im Zusammenhang mit Krankheitsbildern, welche mit geschwächtem Immunsystem oder mangelhafter Durchblutung einhergehen, auf. Zu den mit einer schlechten Durchblutung vor allem der Beine verbundenen Krankheiten zählen insbesondere Diabetes mellitus Typ 2, chronisch-venöse Insuffizienz oder die periphere arterielle Verschlusskrankheit, welche auch als die so genannte "Schaufensterkrankheit" bekannt ist. Bei den zuvor genannten Krankheiten kann sich bereits aus sehr kleinen Wunden oder Druckstellen eine großflächige, schlecht heilende und infizierte bzw. nekrotisierende chronische Wunde entwickeln. Insbesondere bei Infektion solcher Wunden mit Mikroorganismen, beispielsweise den zuvor genannten Hospitalkeimen, kann es zum vollständigen Absterben von Haut, Unterhaut und der Muskelfaszie kommen, was im schlimmsten Fall eine Amputation der betroffenen Gliedmaßen erforderlich macht. Besonders häufig kommt es im Zusammenhang mit Durchblutungsstörungen zur Entstehung des diabetischen Fußsyndroms, einer nektrotisierenden Fasziitis oder eines *Ulcus cruris.* Auch eine Immunschwäche, beispielsweise bei HIV-infizierten Patienten, kann die Entstehung chronischer Wunden begünstigen, da einerseits die Infektionsgefahr als solche erhöht ist und andererseits die Neubildung von Gewebe zum Verschluss der Wunden nur verzögert erfolgt. Auch die als Dekubitus bezeichneten Druckgeschwüre, wie sie meist bei bettlägerigen Patienten aufgrund von falscher Lagerung entstehen, fallen unter die chronischen Wunden, da die Dauer ihrer Abheilung ebenfalls über einen Zeitraum von vier Wochen hinausgeht und besonders sorgfältige und ausdauernde Therapiemaßnahmen erfordert.

Die Wundversorgung bzw. Wundbehandlung verfolgt im Allgemeinen das Ziel, eine Wundinfektion zu verhindern und eine rasche und funktionsgerechte Wundheilung zu gewährleisten. Dabei hängt es vom Schweregrad, insbesondere der Tiefe und Fläche, der Wunde ab, wie intensiv und durch welche Maßnahmen die Wundheilung unterstützt werden muss.

Bereits im Jahre 1979 wurden von dem amerikanischen Mediziner T. D. Turner verschiedene, allgemein anerkannte Qualitätskriterien für den idealen Wundverband aufgestellt, welche auch heute noch ihre Gültigkeit haben.

Dennoch sind die aus dem Stand der Technik bekannten Ansätze zur Wundversorgung bzw. zur Beschleunigung der Wundheilung oftmals unzureichend, da sie im Hinblick auf die allgemein anerkannten Qualitätskriterien für Wundauflagen vielfach nicht zufriedenstellend sind bzw. keinen ausreichenden Therapieerfolg ermöglichen.

In der EP 2 322 232 A 2 wird eine mehrschichtige Wundauflage beschrieben, welche auf einem polysaccharidhaltigen Gel sowie einer zusätzlichen Schicht auf Basis eines weiteren bioverträglichen Materials basiert. Solche Wundauflagen auf Basis von Gelen sind jedoch mit dem Nachteil verbunden, dass durch den an sich schon hohen Feuchtigkeitsgehalt des Gels nur eine verringerte Aufnahme überschüssiger Sekrete erfolgen kann. Insbesondere weisen solche Wundauflagen vor allem aber auch keine ausreichende bakteriostatische bzw. bakterizide Wirkung auf, um einen effizienten Kontaminationsschutz gegenüber Mikroorganismen zu gewährleisten.

Weiterhin beschreibt die DE 10 2007 030 931 A1 eine Wundauflage, welche unter anderem eine desinfizierende bzw. dekontaminierende oder proteasehemmende Substanz aufweist. Mit einer Wundauflage dieses Typs lässt sich jedoch keine ausreichende Geruchsadsorption erzielen. Vor allem aber ist der Schutz vor Kontaminationen aufgrund der nur geringen bakteriostatischen Wirkung nicht hinreichend, um effizient Infektionen vorzubeugen.

Des Weiteren beschreibt die DE 10 2006 050 806 A1 Wundauflagen, welche ein kohlenstoffhaltiges Material umfassen, welches zur Verbesserung der bakteriostatischen Wirkung mit Edelmetallen, insbesondere Silber, imprägniert ist. Gegenüber den zuvor beschriebenen Wundauflagen lässt sich hiermit zwar ein besserer Schutz vor Kontaminationen erzielen. Allerdings besteht durch die eingesetzten Edelmetalle eine signifikant erhöhte Gefahr von Nebenwirkungen, da viele Edelmetalle, insbesondere auch Silber, zellgängig sind und im Verdacht stehen, an den Erkrankungen Alzheimer und Parkinson beteiligt zu sein, da sie die Isolationsproteine an den Nerven schädigen können. Zudem wirkt das edelmetallimprägnierte kohlenstoffhaltige Material zwar antimikrobiell, jedoch kann hierdurch die Wundheilung nicht nachhaltig beschleunigt werden.

Darüber hinaus betrifft die DE 38 75 217 T2 entsprechend der EP 0 311 364 B1 einen Wundverband, welcher eine Aktivkohleschicht umfasst, bei der mindestens 10 % des Gesamtporenvolumens der Aktivkohle durch Mesoporen gebildet sein sollen, wobei der genannte Wundverband steril und in einem bakterienfesten Umschlag enthalten sein soll. Weiterhin ist die Aktivkohle vorteilhafterweise mit einem antimikrobiellen Mittel, wie Silber, imprägniert. Die Nachteile silberimprägnierter Aktivkohle wurden zuvor diskutiert. Darüber hinaus ist eine vorwiegend mesoporöse Aktivkohle nicht geeignet, Mikroorganismen dauerhaft zu immobilisieren, so dass die bakteriostatische Wirkung nicht immer ausreichend ist. Darüber hinaus besitzt dieser Wundverband keine wundheilungsfördernde Wirkung.

Die US 2005/0137272 A1 betrifft getrockneten Schaum auf Basis von gelbildenden Polymeren, welche aus der Gruppe von Alginaten, Pektinen, Carrageenanen, Glykolalginaten sowie deren Mischungen ausgewählt sind und welche mittels eines polyvalenten Kations quervernetzt werden können, sowie gegebenenfalls einem wasserlöslichen Weichmacher. Die Schäume sind weich und biegsam und weisen darüber hinaus eine hohe Absorptionsfähigkeit auf. Sie können unter anderem als Wundverbandmaterial, für Zellkulturen und bioabsorbierbare Implantate eingesetzt werden.

Somit besteht ein Bedarf an Auflagen zur Wundversorgung, welche einen verbesserten Kontaminationsschutz, eine verbesserte Geruchsadsorption zur Entfernung unangenehmer Gerüche sowie eine bessere Aufnahme von überschüssigen Sekreten, insbesondere Wundwasser, besitzen.

Daher liegt der vorliegenden Erfindung die Aufgabe zugrunde, eine Wundauflage zum Einsatz in der Wundversorgung insbesondere des menschlichen und tierischen Körpers bereitzustellen, welche die zuvor geschilderten Probleme im Stand der Technik zumindest teilweise vermeidet oder aber zumindest verringert bzw. abschwächt.

Insbesondere liegt der vorliegenden Erfindung die Aufgabe zugrunde, eine Wundauflage bereitzustellen, welche eine optimierte Wundversorgung ermöglicht, insbesondere auf Basis der Bereitstellung eines verbesserten Wundmilieus.

Zur Lösung der zuvor geschilderten Aufgabe schlägt die vorliegende Erfindung-gemäß einem **ersten** Erfindungsaspekt - eine Wundauflage mit mehrschichtigem Aufbau gemäß Anspruch 1 vor; weitere vorteilhafte Ausgestaltungen dieses Erfindungsaspekts sind Gegenstand der diesbezüglichen Unteransprüche.

Weiterer Gegenstand der vorliegenden Erfindung - gemäß einem **zweiten** Erfindungsaspekt - ist zudem die Verwendung einer erfindungsgemäßen Wundauflage zur therapeutischen Wundversorgung gemäß dem unabhängigen Verwendungsanspruch; weitere vorteilhafte Ausgestaltungen dieses Erfindungsaspekts sind Gegenstand der diesbezüglichen Unteransprüche.

Es versteht sich von selbst, dass besondere Ausgestaltungen und Ausführungsformen, welche nur im Zusammenhang mit einem Erfindungsaspekt beschrieben sind, auch in Bezug auf die anderen Erfindungsaspekte entsprechend gelten, ohne dass dies ausdrücklich beschrieben ist.

Bei allen nachstehend genannten relativen bzw. prozentualen, insbesondere gewichtsbezogenen Mengenangaben ist zu beachten, dass diese Angaben im Rahmen der erfindungsgemäßen Zusammensetzung vom Fachmann derart auszuwählen bzw. zu kombinieren sind, dass in der Summe - gegebenenfalls unter Einbeziehung weiterer Komponenten bzw. Inhaltsstoffe bzw. Zusatzstoffe bzw. Bestandteile, insbesondere wie nachfolgend definiert - stets 100 % bzw. 100 Gew.-% resultieren. Dies versteht sich für den Fachmann aber von selbst.

Im Übrigen gilt, dass der Fachmann anwendungsbezogen oder einzelfallbedingt von den nachfolgend ausgeführten Mengenangaben abweichen kann, ohne dass er den Rahmen der vorliegenden Erfindung verlässt.

Weiterhin versteht es sich von selbst, dass bei allen nachfolgend genannten Werte- bzw. Parameterangaben diese Werte bzw. Parameter nach dem Fachmann an sich geläufigen bzw. standardisierten Methoden oder aber nach explizit angegebenen Methoden bestimmt werden.

Dies vorausgeschickt, wird die vorliegende Erfindung nachfolgend im Detail beschrieben.

Gegenstand der vorliegenden Erfindung - gemäß einem **ersten** Aspekt der vorliegenden Erfindung - ist somit eine Wundauflage, welche zur therapeutischen Wundversorgung geeignet ist, wobei die Wundauflage einen mehrschichtigen Aufbau aufweist, wobei der mehrschichtige Aufbau mindestens eine mindestens ein Hydrokolloid, vorzugsweise Kollagen, enthaltende Schicht ("Hydrokolloidschicht" oder "Kollagenschicht") und mindestens eine Aktivkohle enthaltende Schicht ("Aktivkohleschicht") umfasst,
wobei die Aktivkohle enthaltende Schicht eine kugelförmige Aktivkohle umfasst und wobei die Aktivkohle der Aktivkohle enthaltenden Schicht einen durch Mikroporen mit Porendurchmessern von ≤ 200 nm (20 Å) gebildeten Mikroporenvolumenanteil, bezogen auf das Gesamtporenvolumen der Aktivkohle, von mindestens 60 % aufweist.

Überraschenderweise wurde im Rahmen der vorliegenden Erfindung von der Anmelderin gefunden, dass sich durch die erfindungsgemäße Wundauflage mit der erfindungsgemäßen Kombination auf Basis einer Hydrokolloidschicht, insbesondere einer Kollagenschicht, einerseits sowie einer Aktivkohleschicht andererseits der Heilungsprozess von Wunden allgemein, aber insbesondere auch die Heilung von kompliziert zu therapierenden Wunden, wie infizierten bzw. chronischen bzw. nekrotisierenden Wunden, signifikant beschleunigen lässt.

Was den Begriff "Wundauflage" anbelangt, wie er im Rahmen der vorliegenden Erfindung verwendet wird, so bezeichnet dieser im Allgemeinen insbesondere Auflagen zur topischen Applikation auf äußere Wunden, um das Eindringen von Fremdkörpern in die Wunde zu verhindern und um Blut und Wundsekret aufzunehmen. Synonym können erfindungsgemäß auch Begriffe wie "Wundpflaster", "Wundverband" oder "Wundabdeckung" verwendet werden.

Bei der in der Wundauflage enthaltenen Aktivkohle handelt es sich insbesondere um eine bakteriostatische bzw. antimikrobielle Komponente, welche das Wachstum von Bakterien hemmt und so die Ausbreitung von Bakterien in der zu behandelnden Wunde effizient unterbindet. Insbesondere kommt erfindungsgemäß eine Aktivkohle mit spezieller biostatischer bzw. biozider, insbesondere antimikrobieller Wirkung, welche das Wachstum von Mikroorganismen, insbesondere von Bakterien, in der Wunde effizient unterbindet, zum Einsatz. Insbesondere bewirkt die erfindungsgemäß eingesetzte Aktivkohle, insbesondere bei hohem Mikroporenanteil, dass die Mikroorganismen dauerhaft gebunden bzw. immobilisiert werden (was letzten Endes im Allgemeinen zu deren Abtötung führt, da die Immobilisierung sowohl der Mikroorganismen selbst als auch die möglicher Nährstoffe an der Aktivkohle eine ausreichende Nährstoffversorgung unterbindet).

Darüber hinaus kann Aktivkohle große Mengen an Wundwasser aufnehmen bzw. binden, so dass die Bildung von Staunässe in der Wunde verhindert wird. Weiterhin ermöglicht die Aktivkohle die Adsorption von unangenehmen Geruchsstoffen, wie sie insbesondere bei großflächigen und nekrotisierenden Gewebsunterbrechungen auftreten.

Auch werden durch die Aktivkohle topische und die Wundheilung mitunter hemmende bzw. sogar toxische Abbauprodukte, wie sie einerseits durch die mit der Wundheilung einhergehenden Stoffwechselprodukte entstehen und andererseits infolge von Wundinfektionen auftreten, aufgenommen und unschädlich gemacht. Weiterhin kann die Aktivkohle auch als Feuchtigkeitspuffer dienen: Überschüssiges Wasser bzw. überschüssige Feuchtigkeit können zwischengespeichert bzw. gepuffert und bedarfsweise wieder abgegeben werden, so dass ein ideales Umgebungsmilieu für den Heilungsprozess der Wunde gewährleistet ist, bei dem einerseits bei sehr guter Belüftung der Wunde einem Austrocknen der Wunde, andererseits aber auch einer übermäßig feuchten Umgebung entgegengewirkt wird; auf diese Weise wird eine ideale Feuchtigkeitsumgebung für den Heilungsprozess bereitgestellt. Des Weiteren ist die Aktivkohle mit keinerlei Nebenwirkungen verbunden und ist insbesondere toxikologisch vollkommen unbedenklich.

Auch der in der Wundauflage enthaltenen Hydrokolloidschicht, insbesondere Kollagenschicht, kommt hinsichtlich des Wundheilungsprozesses bzw. der Wundversorgung in den verschiedenen Phasen der Wundheilung eine besondere Bedeutung zu:
Bei der Hydrokolloidschicht handelt es sich insbesondere um eine wundabdeckende Schicht, wobei dieser Begriff dahingehend zu verstehen ist, dass es sich hierbei um eine im Trage- und/oder Anwendungszustand der zu behandelnden Wunde zugewandte Schicht handelt. Insbesondere liegt - im Applikations- bzw. Anwendungszustand der erfindungsgemäßen Wundauflage - die wundabdeckende Schicht zumindest im Wesentlichen vollflächig auf der zu behandelnden Wunde auf bzw. steht zumindest im Wesentlichen vollflächig mit der zu behandelnden Wunde in Kontakt. Sie ist somit wesentlicher Bestandteil der Wundauflage zur primären Aufnahme von Wundwasser einerseits sowie zum Schutz der Wunde vor mechanischer Einwirkung andererseits.

Erfindungsgemäß werden durch die Hydrokolloidschicht, insbesondere die Kollagenschicht, Vorgänge der körpereigenen physiologischen Wundheilung gefördert. Denn durch die Hydrokolloidschicht, vorzugsweise die Kollagenschicht, wird ein feucht-warmes Wundmilieu aufrechterhalten, welches die Wundheilung dahingehend unterstützt, dass insbesondere für enzymatische Vorgänge, wie sie für eine effiziente Wundheilung notwendig sind, eine beschleunigende und optimale Umgebung geschaffen wird. In diesem Zusammenhang wird unter anderem das autolytische (d. h. körpereigene) Debridement von bereits abgestorbenem Gewebe gefördert, was das Risiko des Erfordernisses der Durchführung eines beispielsweise chirurgischen Debridements signifikant herabsetzt. Weiterhin entfaltet das Hydrokolloid, vorzugsweise Kollagen, innerhalb der ersten Stunden nach eingetretener Verwundung seine Wirkung als Hämostat und bewirkt, indem sich über die Hydrokolloidfasern, insbesondere die Kollagenfasern, Blutplättchen an die Wunde anlagern, dass die Blutung schneller zum Stillstand kommt. In der resorptiven Phase, welche nach Bildung des Wundschorfes eintritt, wird die Bildung bzw. das Einwandern von Makrophagen durch das Kollagen unterstützt, so dass eingetretene Fremdkörper bzw. Keime in der Wunde schneller und effizienter unschädlich gemacht werden können.

Die Kombination von Aktivkohleschicht einerseits und Hydrokolloidschicht, vorzugsweise Kollagenschicht, andererseits führt in völlig überraschender Weise zu einer synergistischen Wirkung in Bezug auf die Wundheilung, da die beiden Komponenten - Aktivkohle einerseits und Hydrokolloid, insbesondere Kollagen, andererseits - sich in ihrer Wirkung im Rahmen der Wundheilung ergänzen und darüber hinaus verstärken: Während die Aktivkohle biostatisch bzw. biozid, insbesondere antimikrobiell bzw. bakteriostatisch wirkt und die zuvor ausgeführten Wirkweisen realisiert, wird durch die Hydrokolloid- bzw. Kollagenkomponente eine wundheilungsfördernde bzw. wundheilungsbeschleunigende Wirkung erreicht, d. h. aktiv der Wundheilungsprozess gefördert. In vollkommen überraschender Weise ergänzen sich auf diese Weise Aktivkohleschicht einerseits und Hydrokolloid, insbesondere Kollagenschicht, andererseits im Rahmen ihrer gemeinsamen Verwendung in der erfindungsgemäßen Wundauflage. Dabei ist die gegenseitige Wirkverstärkung dieser beiden Komponenten derart, dass z. B. auf eine Edelmetallimprägnierung, wie eine im Stand der Technik oftmals realisierte Silbermetallimprägnierung, der Aktivkohle im Rahmen der vorliegenden Erfindung gänzlich verzichtet werden kann, ohne dass hierunter die Wirkeffizienz der erfindungsgemäßen Wundauflage leidet; dies war in dieser Weise nicht zu erwarten, sondern wurde überraschend erst durch die Anmelderin im Rahmen ihrer Studien gefunden.

Die vorliegende Erfindung ist insgesamt mit zahlreichen Vorteilen, Verbesserungen und Besonderheiten verbunden, welche die vorliegende Erfindung gegenüber dem Stand der Technik auszeichnen und welche in nicht beschränkender Weise wie folgt zusammengefasst werden können:
Mit der vorliegenden Erfindung wird erstmals eine Wundauflage bereitgestellt, welche die Wundheilung, insbesondere die von komplexen bzw. kompliziert zu therapierenden oder chronischen Wunden, signifikant beschleunigt, darüber hinaus jedoch besonders gut verträglich ist und einen ausgezeichneten Kontaminationsschutz bietet.

Die Anwendung der erfindungsgemäßen Wundauflage führt zu einem beschleunigten Rückgang der Exsudation sowie zu einem schnellen Eintritt der Granulationsphase, in welcher bereits die Neubildung von Gewebe erfolgt, so dass ein schneller Wundverschluss erfolgen kann. Was die verbesserte Wundheilung anbelangt, kann bereits an dieser Stelle auch auf die von der Anmelderin durchgeführten Anwendungs- und Wirksamkeitsstudien verwiesen werden, welche die hervorragende Wirksamkeit belegen, worauf im weiteren Verlauf noch detailliert eingegangen wird.

Als besonders vorteilhaft hat sich im Rahmen der vorliegenden Erfindung die Kombination von einem Hydrokolloidmaterial, insbesondere Kollagenmaterial, mit Aktivkohle erwiesen, da sich beide Material in ihrer Wirkung sozusagen synergistisch ergänzen: Denn die Aktivkohle als solche unterstützt durch ihre biozide und/oder biostatische, insbesondere ihre antimikrobielle bzw. bakteriostatische Wirkung sowie ihre Fähigkeit, Feuchtigkeit kontrolliert zu speichern und mitunter toxische Abbauprodukte zu binden, die Wundheilung und verringert die Infektionsgefahr. In diesem Zusammenhang bindet die erfindungsgemäß eingesetzte Aktivkohle physikalisch Wundwasser und Mikroorganismen, wohingegen das Hydrokolloid, insbesondere das Kollagen, durch die Bereitstellung von für die Wundheilung optimalen physiologischen Bedingungen die Wundheilung in ergänzender Art und Weise stützt.

Insbesondere bietet die erfindungsgemäße Wundauflage einen ausgezeichneten Infektionsschutz auch vor typischen Hospitalkeimen, wie *Staphylococcus aureus, Staphylococcus epidermidis, Proteus mirabilis, Escherichia coli* und *Pseudomonas aerguginosa,* wie auch aus dem im Rahmen der erfindungsgemäßen Ausführungsbeispiele durchgeführten Hemmhoftest hervorgeht. Somit stellt die erfindungsgemäße Wundauflage eine ausgezeichnete Grundlage für die Therapie von mit multiresistenten Stämmen infizierten Wunden ohne den Einsatz von Antibiotika dar. Dies ist insbesondere im Hinblick auf Patienten mit bereits geschwächtem Immunsystem von Vorteil, da die Gabe zahlreicher Antibiotika das Immunsystem noch stärker belastet. Darüber hinaus trägt allgemein ein verringerter Einsatz von Antibiotika zu Therapiezwecken zu einer Eindämmung der Entstehung und Ausbreitung multiresistenter Keime bei.

Weiterhin bietet die erfindungsgemäße Wundauflage den Vorteil, dass sie aufgrund der Verwendung von Aktivkohle das Exsudat bzw. Wundwasser, wie es insbesondere aus großflächigen Wunden austritt, in großen Mengen aufnehmen kann. So wird sichergestellt, dass in der Wunde zwar das für eine gute Wundheilung erforderliche feuchte Milieu aufrechterhalten wird, die Bildung von Staunässe - welche die Wundheilung wiederum verzögern und das Infektionsrisiko steigern würde - jedoch unterbunden wird und toxische Abbauprodukte entfernt werden. Dabei erfolgt nicht nur die bloße Aufnahme von Exsudat und Abbauprodukten, vielmehr werden die Abbauprodukte mit Hilfe der Aktivkohle durch Immobilisieren bzw. Degradation auch unschädlich gemacht.

Neben der guten Adsorption von Wundwasser und Abbauprodukten während der Wundheilung werden bei Verwendung der erfindungsgemäßen Wundauflage zudem aufgrund der adsorptionsstarken Aktivkohle unangenehme Gerüche, wie sie insbesondere bei großflächigen bzw. nekrotisierenden Wunden auftreten, adsorbiert, was insbesondere für das Wohlbefinden des Patienten von Bedeutung ist, da Betroffene oftmals unter den mit einer solchen Wunde verbundenen Unannehmlichkeiten, wie einem starken Geruch, stärker leiden als unter der Wunde als solcher.

Die erfindungsgemäße Wundauflage zeichnet sich weiterhin durch ihre äußerst gute Verträglichkeit bei gleichzeitig gutem Kontaminationsschutz aus. Im Gegensatz zu erfindungsgemäßen Wundauflagen wird bei Wundauflagen des Standes der Technik oftmals eine gute desinfizierende bzw. antimikrobielle Wirkung erst durch den Einsatz von Edelmetallen, insbesondere Silber, erzielt. Der topische Einsatz solcher Metalle ist gesundheitlich jedoch äußerst bedenklich, da insbesondere Silber zellgängig ist und im Verdacht steht, an der Auslösung von Krankheiten, wie Alzheimer oder Parkinson, beteiligt zu sein. Erfindungsgemäß hingegen wird durch den Einsatz von Aktivkohle in Kombination mit einem Hydrokolloid, vorzugsweise Kollagen, bereits ein effizienter Kontaminationsschutz erzielt, welcher eine effektive Vorbeugung und Behandlung von Wundinfektionen ermöglicht.

Außerdem ist die erfindungsgemäße Wundauflage imstande, bei der Behandlung der Wunde ein feucht-warmes Milieu aufrechtzuerhalten, um die Versorgung des Gewebes mit Nährstoffen zu ermöglichen und ein Austrocknen zu verhindern (Trocknet die Wunde nämlich aus, wird der Gewebedefekt durch das Absterben von Zellen noch weiter vergrößert; zudem verlangsamt eine Austrocknung den Heilungsprozess, da durch die mangelhafte Versorgung die Abwehrzellen in ihrer Funktion beeinträchtigt sind und die Enzymaktivität bei der Neubildung von Gewebe gestört wird.). Auch die Temperatur wird mit der erfindungsgemäßen Wundauflage auf einer für die physiologischen Vorgänge der Wundheilung optimalen Temperatur gehalten.

Insbesondere ist die erfindungsgemäße Wundauflage für Gase durchlässig ausgebildet und ermöglicht darüber hinaus eine Entfernung von überschüssigen, krankhaften und giftigen Bestandteilen, wie sie teilweise im Wundwasser vorhanden sind. Von besonders hoher Relevanz ist zudem ein Schutz vor Kontaminationen durch die erfindungsgemäße Wundauflage, um Wundinfektionen vorzubeugen bzw. bereits infizierte Wunden vor dem Eindringen weiterer Mikroorganismen zu schützen.

Darüber hinaus weist die erfindungsgemäße Wundauflage eine gute Haftung in Bezug auf die Wunde auf, ohne jedoch dabei mit dem Wundgrund zu verkleben. Auch ist die erfindungsgemäße Wundauflage derart ausgebildet, dass keine Fasern oder andere Fremdstoffe an die Wunde abgegeben werden können (was andernfalls wiederum zu Entzündungsreaktionen führen könnte).

Im Ergebnis wird erfindungsgemäß also eine effiziente Wundauflage bereitgestellt, deren besondere Wirksamkeit insbesondere auf der gezielten, insbesondere synergistischen Kombination einer Hydrokolloidschicht einerseits und einer Aktivkohleschicht andererseits basiert.

Die erfindungsgemäße Wundauflage kann in vielfältiger Art und Weise ausgestaltet werden. Mögliche Formen und Ausgestastungen werden zum besseren Verständnis im Folgenden veranschaulicht:
Was das Hydrokolloid der Hydrokolloid enthaltenden Schicht anbelangt, so ist es erfindungsgemäß bevorzugt, wenn dieses ausgewählt ist aus der Gruppe von Polysacchariden und Proteinen, insbesondere pflanzlichen, tierischen oder bakteriellen Polysacchariden und Proteinen. Vorzugsweise ist das Hydrokolloid ausgewählt aus der Gruppe von Kollagen, Cellulose und Cellulosederivaten, Glykosaminoglykanen (insbesondere sauren Glykosaminoglykanen, vorzugsweise Hyaluronsäure und/oder deren Salzen), Pektinen, Gummi arabicum, Galactomannanen, Agar, Carrageen, Alginaten, Gelatine, Caseinaten, Xanthanen, Dextranen und Skleroglucanen. Besonders bevorzugt ist es, wenn das Hydrokolloid Kollagen, Hyaluronsäure und/oder deren Salze und/oder Gelatine, ganz besonders bevorzugt Kollagen, ist.
Was den Begriff "Hydrokolloid", wie er im Rahmen der vorliegenden Erfindung verwendet wird, anbelangt, so ist dieser sehr breit zu verstehen. Im Allgemeinen werden unter Hydrokolloiden zumindest teilweise wasserlösliche, natürliche, aber auch synthetische Polymere verstanden, welche in wässrigen Systemen Gele oder viskose Lösungen bzw. Suspensionen bilden. Dabei handelt es sich üblicherweise um Substanzen, welche den Stoffklassen der Proteine oder Polysaccharide angehören, wobei eine Vielzahl von Hydrokolloiden aus der Natur stammt, insbesondere aus Landpflanzen, Algen, Tieren sowie Bakterien. Hydrokolloide werden oftmals als Verdickungsmittel in Kosmetika und Produkten der Nahrungsmittelindustrie eingesetzt. Für weitergehende Einzelheiten zum Begriff des Hydrokolloids kann insbesondere verwiesen werden auf Römpp Chemielexikon, 10. Auflage, Georg Thieme Verlag, Stuttgart/New York, Stichwort: "Hydrokolloide", Seite 1837, einschließlich der dort referierten Literatur.

Wie aus den vorangehenden Ausführungen bereits deutlich wird, hat es sich als besonders vorteilhaft erwiesen, wenn das Hydrokolloid Gelatine und/oder Kollagen, insbesondere Kollagen, ist.

Bei Kollagen handelt es sich um langfaserige, linearkolloide und hochmolekulare Skleroproteine der extrazellulären Matrix, welche im Bindegewebe, insbesondere in der Haut, im Knorpel sowie in Sehnen, Bändern und Blutgefäßen sowie der proteinhaltigen Grundsubstanz der Knochen von Wirbeltieren, aber auch in phylogenetisch frühen Lebensformen, wie Schwämmen oder Seeanemonen, vorkommen. Die faserige Struktur des Kollagens wird insbesondere durch das Auftreten von Glycin an jeder dritten Position in der Aminosäuresequenz bedingt, da Glycin als sehr raumsparende Aminosäure eine spezielle, helikale Sekundärstruktur in Proteinen bedingt. Die auch als sogenannte *Helixbreaker* bekannten Aminosäuren Tryptophan und Tyrosin sowie die Disulfidbrücken ausbildende Aminosäure Cystein hingegen liegen in Kollagenen im Allgemeinen nicht vor. Für weitergehende Einzelheiten zum Begriff des Kollagens kann überdies verwiesen werden auf Römpp, Chemielexikon, 10. Auflage, Georg Thieme Verlag, Stuttgart/New York, Stichwort: "Collagene", Seiten 796 und 797, sowie die hierin referierte Literatur.

Was speziell die Verwendung von Kollagen im Rahmen der erfindungsgemäßen Wundauflage anbelangt, so ist dieses in der Lage, den Prozess der Wundheilung signifikant zu steigern. Insbesondere besitzt Kollagen eine proteasehemmende Wirkung, was zur Senkung des der Wundheilung abträglichen erhöhten Proteasespiegels im Wundgebiet dient. Ist der Proteasenspiegel im Wundgebiet nämlich erhöht, führt dies oftmals zu einer unkoordinierten Wundheilung und zur Zerstörung von Wachstumsfaktoren, da diese durch Proteasen, wie beispielsweise neutrophile Elastasen oder Matrix-Metallo-Proteasen (MMPs), degradiert werden. Weiterhin regt Kollagen die Bildung von Gefäßstrukturen und Bindegewebe an und unterstützt so die Wiederherstellung der strukturellen Stabilität des Gewebes. In diesem Sinne kann durch Verwendung von Kollagen als Hydrokolloid die Wundheilung in äußerst effizienter Art und Weise unterstützt werden.

Ähnliche Ausführungen treffen auch auf Gelatine zu, welche ebenfalls in bevorzugter Weise in der Wundauflage als Hydrokolloid eingesetzt werden kann: Unter dem Begriff "Gelatine" wird üblicherweise und im Rahmen der vorliegenden Erfindung ein Polypeptid, welches vornehmlich durch Hydrolyse des in Haut und Knochen von Tieren enthaltenen Kollagens unter sauren oder basischen Bedingungen gewonnen wird, verstanden. Dabei resultiert die Gewinnung von Gelatine unter sauren Bedingungen in der so genannten Typ A-Gelatine bzw. unter alkalischen Bedingungen in der so genannten Typ B-Gelatine. In Wasser, insbesondere unter gleichzeitigem Einfluss von Wärme, quillt Gelatine zunächst stark auf und löst sich darin unter Bildung einer viskosen Lösung, welche schließlich unterhalb von 35 °C gallertartig erstarrt. Für weitergehende Einzelheiten zum Begriff der Gelatine kann verwiesen werden auf Römpp Chemielexikon, 10. Auflage, Georg Thieme Verlag Stuttgart/New York, Stichwort: "Gelatine", Seite 1484, sowie die hierin referierte Literatur.

Was die Hydrokolloidschicht, insbesondere die Kollagenschicht, weiterhin anbelangt, so kann es erfindungsgemäß vorgesehen sein, dass die Hydrokolloid, vorzugsweise Kollagen, enthaltende Schicht auf einem Hydrokolloidvlies und/oder Hydrokolloidschaum, vorzugsweise einem Kollagenvlies und/oder Kollagenschaum, basiert. In diesem Zusammenhang kann es vorgesehen sein, dass die Hydrokolloidschicht auf Basis von Hydrokolloidvlies und/oder Hydrokolloidschaum, vorzugsweise Kollagenvlies und/oder Kollagenschaum, porcinen, bovinen und/oder equinen Ursprungs, besonders bevorzugt auf Basis von Hydrokolloidvlies und/oder Hydrokolloidschaum, vorzugsweise Kollagenvlies und/oder Kollagenschaum, porcinen Ursprungs, ausgebildet ist.

In erfindungsgemäß besonders bevorzugter Weise kann es vorgesehen sein, dass die das Hydrokolloid, vorzugsweise Kollagen, enthaltende Schicht durch ein Hydrokolloidvlies und/oder Hydrokolloidschaum, vorzugsweise Kollagenvlies und/oder einen Kollagenschaum, insbesondere durch ein Hydrokolloidvlies und/oder Hydrokolloidschaum, vorzugsweise Kollagenvlies und/oder einen Kollagenschaum, porcinen, bovinen und/oder equinen Ursprungs, bevorzugt durch ein Hydrokolloidvlies und/oder Hydrokolloidschaum, vorzugsweise Kollagenvlies und/oder einen Kollagenschaum, porcinen Ursprungs, gebildet ist.

Der Einsatz von Hydrokolloidvlies bzw. Hydrokolloidschaum, vorzugsweise Kollagenvlies bzw. Kollagenschaum, ist gegenüber herkömmlichen Materialien zur Herstellung von Wundauflagen insbesondere mit dem Vorteil verbunden, dass das Material mit dem Wundgrund bzw. der Wundoberfläche nicht verklebt, aber dennoch eine gute Haftung an der Oberfläche erzielt werden kann. Darüber hinaus ist es von besonderem Vorteil, dass Wundauflagen auf Basis von Hydrokolloidschaum bzw. Hydrokolloidvlies, insbesondere Kollagenschaum bzw. Kollagenvlies, keine Fasern bzw. keine festen Bestandteile bzw. Partikel bzw. Aktivkohle an die Wunde abgeben und somit das Eindringen bzw. ein zusätzliches Herantragen von Fremdkörpern verhindert wird.

Als besonders vorteilhaft hat sich in diesem Zusammenhang erwiesen, wenn die Wundauflage Hydrokolloidschaum, insbesondere Kollagenschaum, d. h. zu einem Schaum verfestigtes bzw. expandiertes Hydrokolloid bzw. Kollagen, aufweist, insbesondere da durch die im Hydrokolloidschaum bzw. Kollagenschaum enthaltenen Poren zudem in effizienter Weise große Mengen Wundwasser aus dem Wundgebiet abfließen können, so dass die Bildung von Staunässe sowie ein zu langer Kontakt von im Wundwasser enthaltenen und der Wundheilung abträglichen Substanzen mit der Wunde selbst verhindert wird. Dabei verhindern die chemischen und physikalischen Eigenschaften von verfestigtem und expandiertem Hydrokolloid bzw. Kollagen (d. h. Hydrokolloid- bzw. Kollagenschaum) dennoch, dass die Wunde austrocknen kann. Darüber hinaus sind derartige Schäume an die Form des Wundgrundes äußerst gut anpassbar, d. h. sie können die Wunde vollflächig bzw. flächendeckend abdecken, ohne dass Aufwölbungen oder dergleichen entstehen. Weiterhin wird unter Verwendung eines Hydrokolloidschaums bzw. eines Kollagenschaums eine besonders gute Gas- bzw. Luftdurchlässigkeit ermöglicht. Dies ist insbesondere mit dem Vorteil verbunden, dass die Wunde gut belüftet wird, insbesondere mit Sauerstoff, was einerseits die physiologischen Wundheilungsprozesse fördert, andererseits auch das Wachstum von Keimen mit anaerober Lebensweise, beispielsweise aus der Gattung *Clostridium,* verhindert.

Im Ergebnis wird also durch das Vorsehen der Kolloidschicht bzw. Kollagenschicht einerseits in effizienter Weise Wundwasser abgeführt und andererseits eine gute Gasdurchlässigkeit gewährleistet.

Was die Hydrokolloidschicht, insbesondere die Kollagenschicht, weiterhin anbelangt, so kann es erfindungsgemäß vorgesehen sein, dass diese erhältlich ist durch Aufbringen einer Dispersion oder Lösung eines Hydrokolloids, vorzugsweise eines Kollagens, auf einem Träger und nachfolgende Trocknung, insbesondere Lyophilisation (Gefriertrocknung), vorzugsweise unter Expansion des Hydrokolloids, vorzugsweise des Kollagens. Eine erfindungsgemäß geeignete Hydrokolloid-, vorzugsweise Kollagensuspension oder -lösung, ist insbesondere erhältlich durch Suspendieren bzw. Solubilisieren des Hydrokolloids, insbesondere Kollagens, in Wasser, insbesondere Reinstwasser bzw. in desinfiziertem oder entkeimtem bzw. sterilisiertem Wasser. Dabei kann das Hydrokolloid, insbesondere Kollagen, vorzugsweise in einer Menge im Bereich von 0,1 bis 5 Gew.-%, insbesondere 0,5 bis 4 Gew.-%, vorzugsweise 0,7 bis 3 Gew.-%, besonders bevorzugt 1 bis 2 Gew.-%, bezogen auf die Hydrokolloidsuspension oder -lösung, vorzugsweise Kollagensuspension oder -lösung, in der Suspension oder Lösung enthalten sein. Das getrocknete und expandierte Hydrokolloid, vorzugsweise Kollagen, kann schließlich von dem Träger entfernt werden und zur Herstellung der Wundauflage weiterverwendet werden. Zur Gewährleistung der gewünschten Eigenschaften kann das Hydrokolloid bzw. die entsprechende Schicht mit dem Hydrokolloid einen definierten Restfeuchtegehalt aufweisen, was dem Fachmann bekannt ist.

Das Hydrokolloid, vorzugsweise das Kollagen, der Hydrokolloidschicht, insbesondere Kollagenschicht, kann insbesondere porcinen, bovinen und/oder equinen Ursprungs, bevorzugt porcinen Ursprungs, insbesondere aus porciner Haut, sein. Ein Kollagenmaterial mit den vorgenannten Eigenschaften ist kommerziell erhältlich, insbesondere über die medichema^{®} GmbH, Chemnitz, Bundesrepublik Deutschland.

Weiterhin ist es erfindungsgemäß bevorzugt, wenn die mindestens ein Hydrokolloid, vorzugsweise Kollagen, enthaltende Schicht eine äußere Schicht der Wundauflage ist bzw. bildet. Insbesondere kann es in diesem Zusammenhang vorgesehen sein, dass die mindestens ein Hydrokolloid, vorzugsweise Kollagen, enthaltende Schicht im Applikations- bzw. Anwendungszustand der Wundauflage auf der der zu behandelnden Wunde zugewandten Seite der Wundauflage angeordnet ist.

Was die Abmessungen der mindestens ein Hydrokolloid, vorzugsweise Kollagen, enthaltenden Schicht anbelangt, so weist diese vorzugsweise eine Dicke im Bereich von 0,01 bis 100 mm, insbesondere 0,02 bis 50 mm, vorzugsweise 0,05 bis 10 mm, auf. Je nach Schweregrad der zu behandelnden Wunde und Stärke der Wundexsudation ist es vorteilhaft - insbesondere im Falle einer starken Sekretion von Wundwasser (insbesondere z. B. in der exsudativen Phase der Wundheilung) -, wenn die ein Hydrokolloid, vorzugsweise Kollagen, enthaltende Schicht besondern dick ausgebildet ist. Bei bereits im Heilungsprozess fortgeschrittenen Wunden ist es hingegen meist ausreichend, deutlich dünnere Hydrokolloid- bzw. Kollagenschichten einzusetzen. Erfindungsgemäß ist es somit möglich, die Dicke der Hydrokolloid- bzw. Kollagenschicht an die jeweiligen Erfordernisse anzupassen.

In diesem Zusammenhang kann es erfindungsgemäß vorgesehen sein, dass die ein Hydrokolloid, vorzugsweise Kollagen, enthaltende Schicht 5 % bis 95 %, insbesondere 10 % bis 80 %, vorzugsweise 20 % bis 60 %, der Gesamtdicke der Wundauflage ausmacht.

Auch die in der Wundauflage enthaltene Aktivkohle kann - wie im Folgenden noch erläutert wird - durch die sehr spezielle Auswahl an die jeweiligen an die erfindungsgemäße Wundauflage gestellten Anforderungen angepasst werden.

Was die physikalische Form bzw. körperliche Ausgestaltung der in der Wundauflage enthaltenen Aktivkohle anbelangt, so handelt es sich hierbei erfindungsgemäß um eine kugelförmige Aktivkohle. Hinsichtlich der bakteriostatischen bzw. antimikrobiellen Wirkung sowie der Aufnahme von Wundwasser hat sich die Verwendung von kugelförmiger Aktivkohle als besonders effizient erwiesen. Eine kugelförmige Aktivkohle bietet den Vorteil einer besonders guten Verarbeitbarkeit, insbesondere im Hinblick auf eine Fixierung an einem flächigen, vorzugsweise textilen Träger, und auf eine gute mechanische Belastbarkeit, so dass kein Staub und keine Verunreinigungen freigesetzt werden.

Gemäß einer bevorzugten Ausführungsform der vorliegenden Erfindung ist es vorgesehen, dass die Aktivkohle enthaltende Schicht eine kugelförmige Aktivkohle mit absoluten Teilchengrößen im Bereich von 0,01 bis 3 mm, insbesondere im Bereich von 0,02 bis 2 mm, bevorzugt im Bereich von 0,05 bis 1,5 mm, besonders bevorzugt im Bereich von 0,1 bis 0,8 mm, ganz besonders bevorzugt im Bereich von 0,2 bis 0,6 mm, umfasst. Gleichermaßen kann es vorgesehen sein, dass die Aktivkohle enthaltende Schicht eine kugelförmige Aktivkohle mit mittleren Teilchengrößen, insbesondere bestimmt nach ASTM D2862-97/04, im Bereich von 0,05 bis 2,5 mm, insbesondere im Bereich von 0,1 bis 2 mm, bevorzugt im Bereich von 0,15 bis 1 mm, besonders bevorzugt im Bereich von 0,2 bis 0,6 mm, umfasst.

Die im Folgenden genannten Parameterangaben der erfindungsgemäß eingesetzten Aktivkohle werden mit genormten oder explizit angegebenen Bestimmungsverfahren oder dem Fachmann an sich geläufigen Bestimmungsverfahren bestimmt bzw. vermittelt. Diese Parameterangaben ergeben sich, sofern nachfolgend nichts anderes angegeben ist, insbesondere aus den Stickstoffsorptionsisothermen der Aktivkohle.

Hinsichtlich der Beschaffenheit der eingesetzten Aktivkohle ist es weiterhin vorgesehen, dass die Aktivkohle der Aktivkohle enthaltenden Schicht einen durch Mikroporen mit Porendurchmessern von ≤ 200 nm (20 Å) gebildeten Mikroporenvolumenanteil von mindestens 60 %, insbesondere mindestens 65 %, bevorzugt mindestens 70 %, bezogen auf das Gesamtporenvolumen der Aktivkohle, aufweist. Insbesondere ist es von Vorteil, wenn die Aktivkohle der Aktivkohle enthaltenden Schicht einen durch Mikroporen mit Porendurchmessern von ≤ 200 nm (20 Å) gebildeten Mikroporenvolumenanteil, bezogen auf das Gesamtporenvolumen der Aktivkohle, im Bereich von 60 % bis 95 %, insbesondere im Bereich von 65 % bis 90 %, bevorzugt im Bereich von 70 % bis 85 %, aufweist. Was den übrigen Porenvolumenanteil der eingesetzten Aktivkohle anbelangt, so wird dieser durch Meso- und Makroporen gebildet.

Im Rahmen der vorliegenden Erfindung bezeichnet der Begriff der Mikroporen solche Poren mit Porendurchmessern bis zu 200 nm (20 Å) einschließlich, wohingegen der Begriff der Mesoporen solche Poren mit Porendurchmessern von > 200 nm (20 Å) bis 500 nm (50 Å) einschließlich bezeichnet und der Begriff der Makroporen solche Poren mit Porendurchmessern > 500 nm (50 Å) bezeichnet.

Durch den hohen Mikroporenanteil kann insbesondere eine bessere Sorption von Wundwasser sowie von Geruchsstoffen erzielt werden. Darüber hinaus ist die bakteriostatische bzw. antimikrobielle Wirkung gegenüber Aktivkohle mit hohem Meso- und Makroporenanteil signifikant verbessert. Zudem weist eine Aktivkohle mit hohem Mikroporenanteil den Vorteil auf, dass Mikroorganismen dauerhaft gebunden bzw. immobilisiert werden können.

Weiterhin kann es erfindungsgemäß vorgesehen sein, dass die Aktivkohle der Aktivkohle enthaltenden Schicht ein durch Mikroporen mit Porendurchmessern von ≤ 200 nm (20 Å) gebildetes Mikroporenvolumen, insbesondere ein Mikroporenvolumen nach Carbon Black, von mindestens 0,40 cm³/g, insbesondere mindestens 0,45 cm³/g, vorzugsweise mindestens 0,50 cm³/g, aufweist. Insbesondere kann es erfindungsgemäß vorgesehen sein, dass die Aktivkohle der Aktivkohle enthaltenden Schicht ein durch Mikroporen mit Porendurchmessern von ≤ 200 nm (20 Å) gebildetes Mikroporenvolumen, insbesondere ein Mikroporenvolumen nach Carbon Black, im Bereich von 0,40 cm³/g bis 2 cm³/g, insbesondere im Bereich von 0,45 cm³/g bis 1,5 cm³/g, vorzugsweise im Bereich von 0,50 cm³/g bis 1,2 cm³/g, aufweist.

Die Bestimmungsmethode nach Carbon Black ist dem Fachmann an sich bekannt, so dass es diesbezüglich keiner weitergehenden Einzelheiten bedarf. Zudem kann zu weitergehenden Einzelheiten der Bestimmung der Porenoberfläche und des Porenvolumens nach Carbon Black beispielsweise verwiesen werden auf R. W. Magee, Evaluation of the External Surface Area of Carbon Black by Nitrogen Adsorption, Presented at the Meeting of the Rubber Division of the American Chem. Soc., Oktober 1994, z. B. referiert in: Quantachrome Instruments, AUTOSORB-1, AS1 WinVersion 1.50, Operating Manual, OM, 05061, Quantachrome Instruments 2004, Florida, USA, Seiten 71 ff.

Was den Mikroporenoberflächenanteil der erfindungsgemäß eingesetzten Aktivkohle anbelangt, so kann es im Rahmen der vorliegenden Erfindung vorgesehen sein, dass die Aktivkohle der Aktivkohle enthaltenden Schicht einen spezifischen Mikroporenoberflächenanteil, insbesondere einen aus Poren mit Porendurchmessern von ≤ 200 nm (20 Å) gebildeten Mikroporenoberflächenanteil, von mindestens 50 %, insbesondere mindestens 60 %, bevorzugt mindestens 70 %, ganz besonders bevorzugt mindestens 75 %, bezogen auf die spezifische Gesamtoberfläche (BET) der Aktivkohle, aufweist.

Weiterhin ist es erfindungsgemäß bevorzugt, wenn die Aktivkohle der Aktivkohle enthaltenden Schicht eine innere Oberfläche (BET) im Bereich von 500 bis 3.000 m²/g, insbesondere im Bereich von 800 bis 2.000 m²/g, bevorzugt im Bereich von 900 bis 1.800 m²/g, besonders bevorzugt im Bereich von 1.000 bis 1.600 m²/g, aufweist.

Die Bestimmung der spezifischen Oberfläche gemäß BET ist dem Fachmann grundsätzlich als solche bekannt, so dass es diesbezüglich keiner weitergehenden Einzelheiten bedarf. Alle BET-Oberflächenangaben beziehen sich auf die Bestimmung gemäß ASTM D6556-04. Im Rahmen der vorliegenden Erfindung wird zur Bestimmung der BET-Oberfläche die so genannte MultiPoint-BET-Bestimmungsmethode (MP-BET) in einem Partialdruckbereich p/p0 von 0,05 bis 0,1 angewendet. In Bezug auf weitergehende Einzelheiten zur Bestimmung der BET-Oberfläche bzw. zu der BET-Methode kann auf die vorgenannte ASTM D6556-04 sowie auf Römpp Chemielexikon, 10. Auflage, Georg Thieme Verlag, Stuttgart/New York, Stichwort: "BET-Methode", einschließlich der dort referierten Literatur, und auf Winnacker-Küchler (3. Auflage), Band 7, Seiten 93 ff. sowie auf Z. Anal. Chem. 238, Seiten 187 bis 193 (1968) verwiesen werden.

Um eine gute Gesamteffizienz von Adsorptionsleistung, insbesondere in Bezug auf die Adsorption von Wundwasser und Geruchsstoffen sowie die antimikrobielle bzw. bakteriostatische Wirkung, zu erzielen, ist es erfindungsgemäß zudem von Vorteil, wenn die Aktivkohle der Aktivkohle enthaltenden Schicht ein Gesamtporenvolumen, insbesondere ein Gesamtporenvolumen nach Gurvich, im Bereich von 0,1 bis 4 cm³/g, insbesondere im Bereich von 0,2 bis 3 cm³/g, vorzugsweise im Bereich von 0,3 bis 2,5 cm³/g, besonders bevorzugt im Bereich von 0,5 bis 2 cm³/g, aufweist.

Was die Bestimmung des Gesamtporenvolumens nach Gurvich anbelangt, so handelt es sich um eine dem Fachmann auf diesem Gebiet an sich bekannte Mess- bzw. Bestimmungsmethode. Zu weitergehenden Einzelheiten bezüglich der Bestimmung des Gesamtporenvolumens nach Gurvich kann beispielsweise verwiesen werden auf L. Gurvich (1915), J. Phys. Chem. Soc. Russ. 47, 805, sowie auf S. Lowell et al., Characterization of Porous Solids and Powders: Surface Area Pore Size and Density, Kluwer Academic Publishers, Article Technology Series, Seiten 111 ff.

Um zu verhindern, dass Teile der Aktivkohle selbst als Fremdkörper in die Wunde eindringen, ist es von besonderem Vorteil, wenn die Aktivkohle dergestalt ist, dass zumindest im Wesentlichen keine Partikel bzw. Staub an die Umgebung abgegeben werden. In diesem Zusammenhang ist es erfindungsgemäß bevorzugt, wenn die Aktivkohle eine Druck- und/oder Berstfestigkeit, insbesondere eine Gewichtsbelastbarkeit pro Aktivkohleteilchen, insbesondere pro Aktivkohlekorn oder Aktivkohlekugel, von mindestens 10 Newton, insbesondere mindestens 15 Newton, vorzugsweise mindestens 20 Newton, aufweist. Gleichermaßen kann es erfindungsgemäß vorgesehen sein, wenn die Aktivkohle eine Druck- und/oder Berstfestigkeit, insbesondere eine Gewichtsbelastbarkeit, pro Aktivkohleteilchen, insbesondere pro Aktivkohlekorn oder Aktivkohlekugel, im Bereich von 10 bis 50 Newton, insbesondere im Bereich von 12 bis 45 Newton, vorzugsweise im Bereich von 15 bis 40 Newton, aufweist.

Im Rahmen der vorliegenden Erfindung ist es bevorzugt, wenn die Aktivkohle zumindest im Wesentlichen abriebfest und/oder zumindest im Wesentlichen staubfrei ausgebildet ist. Durch die hervorragende Abriebfestigkeit und die Staubfreiheit der eingesetzten Aktivkohle wird ermöglicht, dass die zu behandelnde Wunde nicht durch Materialien oder Verunreinigungen (wie z. B. Aktivkohlestaub) der Wundauflage kontaminiert wird.

Wie zuvor ausgeführt, sollte die Abriebhärte der erfindungsgemäß eingesetzten Aktivkohle extrem hoch ausgebildet sein: So liegt die Abriebfestigkeit der erfindungsgemäß eingesetzten Aktivkohle nach der Methode gemäß CEFIC (Conseil Européen des Federations des l'Industrie Chimique, Avenue Louise 250, Bte 71, B - 1050 Brüssel, November 1986, European Council of Chemical Manufacturers' Federations, Testmethoden für Aktivkohlen, Ziffer 1.6 "Mechanische Härte", Seiten 18/19) vorteilhafterweise bei 100 %. Auch nach ASTM D3802 sollten Abriebfestigkeiten der erfindungsgemäß eingesetzten Aktivkohle von 100 % vorliegen.

Insbesondere kann es erfindungsgemäß vorgesehen sein, dass die Aktivkohle der Aktivkohle enthaltenden Schicht eine fraktale Dimension der offenen Porosität von mindestens 2,3, insbesondere von mindestens 2,4, vorzugsweise von mindestens 2,5, besonders bevorzugt von mindestens 2,7, aufweist. Die fraktale Dimension der offenen Porosität kann insbesondere gemäß WO 2004/046033 A1 bzw. DE 102 54 241 A1 bestimmt werden und kennzeichnet insbesondere die Rauhigkeit, insbesondere Mikrorauhigkeit, der inneren Oberfläche der Aktivkohle. Dieser Wert ist somit als Maß für die Mikrostrukturierung der inneren Oberfläche der Aktivkohle zu werten. Je größer der Wert für den Parameter der fraktalen Dimension der offenen Porosität und somit die Oberflächenrauhigkeit der Aktivkohle ist, desto stärker ist die Fähigkeit der Aktivkohle ausgeprägt, vermehrt bindungsfähige oder zumindest attraktiv wirkende Unregelmäßigkeiten der elektronischen Zustanddichtefunktionen an der inneren Oberfläche der Aktivkohle auszubilden, verbunden mit der Folge einer gesteigerten bzw. verbesserten Bindung von zu sorbierenden, insbesondere zu adsorbierenden Spezies. Die Verbesserung der Bindung umfasst einerseits eine Erhöhung der Packungsdichte innerhalb einer adsorbierten Monolage (und somit einer Erhöhung der Adsorptionskapazität) und andererseits eine erhöhte Bindungsfestigkeit. Aufgrund der Auswahl einer Aktivkohle mit derartigen Werten für die fraktale Dimension der offenen Porosität können im Rahmen der erfindungsgemäßen Wundauflage Spezies, wie insbesondere Mikroorganismen, Toxine etc., in gesteigertem Maße, insbesondere mit verbesserter Beladung bzw. Kapazität und mit größerer Irreversibilität, sorptiv bzw. adsorptiv gebunden werden.

Um die gesamte Wirkweise, insbesondere in Bezug auf den Kontaminationsschutz und die Wundheilungsförderung, der erfindungsgemäßen Wundauflage noch weiter zu verbessern, hat es sich als ganz besonders vorteilhaft erwiesen, die bioziden und/oder biostatischen, insbesondere antimikrobiellen Eigenschaften der eingesetzten Aktivkohle in spezieller Art und Weise noch zu steigern. Was den Begriff "biozide Eigenschaften" anbelangt, so ist dieser so zu verstehen, dass durch die bioziden Eigenschaften insbesondere Mikroorganismen abgetötet und/oder degradiert werden. Im Rahmen der vorliegenden Erfindung werden unter Mikroorganismen sowohl Bakterien als auch Pilze, aber darüber hinaus auch Viren verstanden. Unter bioziden Eigenschaften im Sinne der vorliegenden Erfindung werden somit gleichermaßen bakterizide, fungizide und/oder viruzide Eigenschaften verstanden.

Durch "biostatische Eigenschaften" hingegen werden Mikroorganismen, insbesondere Bakterien, Pilze und Viren, vorrangig in ihrem Wachstum bzw. in ihrer Vermehrung gehemmt. Unter biostatischen Eigenschaften im Sinne der vorliegenden Erfindung werden somit gleichermaßen bakteriostatische, fungistatische und/oder virustatische Eigenschaften verstanden.

In diesem Zusammenhang ist es erfindungsgemäß besonders bevorzugt, wenn die Aktivkohle der Aktivkohle enthaltenden Schicht eine biozide und/oder biostatische, insbesondere antimikrobielle Wirkung und/oder Ausrüstung aufweist. Was die zu diesem Zweck zu verwendende Aktivkohle als solche anbelangt, so ist es vorteilhaft, synthetische bzw. synthetisch hergestellte Aktivkohle zu verwenden.

Was die biozide und/oder biostatische, insbesondere antimikrobielle Wirkung und/oder Ausrüstung der Aktivkohle weiterhin anbelangt, so kann es erfindungsgemäß vorgesehen sein, diese durch den Herstellungsprozess der Aktivkohle, insbesondere die Herstellung mittels Pyrolyse und nachfolgende Aktivierung organischer Polymere, zu erreichen. Die zuvor beschriebene Wirkung und/oder Ausrüstung der Aktivkohle resultiert insbesondere infolge der im Rahmen des Herstellungsprozesses generierten Oberflächenladung und/oder Hydrophobie und/oder texturellen Eigenschaften. Was die Ausgangspolymere zur Herstellung der Aktivkohle anbelangt, so kann es sich hierbei insbesondere um Polystyrole, vorzugsweise um divinylbenzolvernetzte Polystyrole, handeln.

In diesem Zusammenhang ist insbesondere hervorzuheben, dass die hervorragende antimikrobielle Wirksamkeit der erfindungsgemäß eingesetzten Aktivkohle darauf basiert, dass die zuvor beschriebenen Eigenschaften, insbesondere in Kombination mit einem hohen Mikroporenvolumen, insbesondere auf Polaritäten von (Bio-)Molekülen und (Bio-)Partikeln ansprechen. Was die Adsorption von Mikroorganismen, insbesondere Bakterien, anbelangt, so ist - ohne sich hierbei auf diese Theorie beschränken zu wollen - die erfindungsgemäß eingesetzte Aktivkohle insbesondere derart ausgebildet, dass insbesondere eine Affinität zu den in und/oder an der Zellwand der Mikroorganismen verankerten Molekülen besteht.

Was die biozide und/oder biostatische, insbesondere antimikrobielle Wirkung und/oder Ausrüstung der Aktivkohle im Speziellen anbelangt, so kann diese auch durch eine optimierte zusätzliche Ausrüstung, insbesondere Imprägnierung, der Aktivkohle mit mindestens einem bioziden und/oder biostatischen, insbesondere antimikrobiellen Wirkstoff, insbesondere wie im Nachfolgenden noch definiert, erfolgen bzw. hierdurch gesteigert werden.

Durch die zusätzliche Ausrüstung, insbesondere Imprägnierung, der Aktivkohle mit mindestens einem bioziden und/oder biostatischen, insbesondere antimikrobiellen Wirkstoff werden die inhärenten, insbesondere durch den Herstellungsprozess der Aktivkohle bedingten biostatischen bzw. bioziden, insbesondere antimikrobiellen Eigenschaften der Aktivkohle an sich zusätzlich durch die antimikrobiellen Eigenschaften des Wirkstoffs verstärkt. Die Ausrüstung, insbesondere Imprägnierung, der Aktivkohle erfolgt in dem Fachmann an sich bekannter Weise, beispielsweise durch Inkontaktbringen der Aktivkohle mit dem vorgesehenen Wirkstoff bzw. einer den Wirkstoff enthaltenden Lösung und/oder Dispersion. Ein solches Inkontaktbringen kann zum Beispiel durch Aufsprühen, Aufschlämmen, Tränken und dergleichen erfolgen.

Was die erfindungsgemäß eingesetzte Aktivkohle weiterhin anbelangt, so ist diese im Allgemeinen frei von Metallimprägnierungen. Bei der Ausrüstung und/oder Imprägnierung der erfindungsgemäß eingesetzten Aktivkohle sind also Metallimprägnierungen (z. B. auf Basis von Silber bzw. Silberionen) nicht vorgesehen. Auf diese Weise werden schädliche Nebenwirkungen in effizienter Weise verhindert. Insbesondere durch die Kombination mit der Hydrokolloidschicht, vorzugsweise Kollagenschicht, ist dennoch eine gute Wirkeffizienz gewährleistet.

Eine Aktivkohle mit den vorgenannten Eigenschaften ist kommerziell verfügbar, insbesondere über die Blücher GmbH, Erkrath / Bundesrepublik Deutschland, oder die Adsor-Tech GmbH, Premnitz / Bundesrepublik Deutschland.

Was die eingesetzten Mengen der Aktivkohle anbelangt, so können diese in weiten Bereichen variieren:
Um eine Wundauflage nach der Erfindung mit besonders hoher Wirkeffizienz bereitzustellen, kann es erfindungsgemäß vorgesehen sein, dass die Aktivkohle der Aktivkohle enthaltenden Schicht in einer Menge, insbesondere Auflagemenge, von 1 bis 1.000 g/m², insbesondere 5 bis 500 g/m², vorzugsweise 10 bis 400 g/m², bevorzugt 20 bis 300 g/m², besonders bevorzugt 25 bis 250 g/m², vorhanden ist.

Um eine sichere Fixierung der eingesetzten Aktivkohle sowie darüber hinaus einen Schutz vor mechanischer Belastung zu gewährleisten, ist es im Rahmen der vorliegenden Erfindung bevorzugt, wenn die Aktivkohle der Aktivkohle enthaltenden Schicht auf einem flächigen, vorzugsweise textilen Träger angeordnet ist, vorzugsweise hieran befestigt bzw. fixiert ist. Gleichermaßen kann es vorgesehen sein, dass die Aktivkohle der Aktivkohle enthaltenden Schicht auf einem dreidimensionalen, vorzugsweise porösen und/oder textilen Träger, bevorzugt einem Schaum oder Schaumstoff, angeordnet ist, vorzugsweise hieran befestigt bzw. fixiert bzw. hierin eingelagert ist. Insbesondere kann es in diesem Zusammenhang auch vorgesehen sein, dass der dreidimensionale Träger auf Basis eines Elastomerharzes bzw. auf Basis eines Polyurethans ausgebildet ist.

Der Vorteil der zuvor genannten (Träger-)Materialien ist insbesondere darin zu sehen, dass diese besonders luftdurchlässig sind, was dem Heilungsprozess zugute kommt. Wie oben bereits erwähnt, ist die Belüftung der Wunde insbesondere in Bezug auf die Bereitstellung von Sauerstoff im Wundgebiet sowie die Verhinderung des Wachstums anaerober Keime von Relevanz.

Gemäß einer besonders bevorzugten Ausführungsform der vorliegenden Erfindung kann es vorgesehen sein, dass die Aktivkohle der Aktivkohle enthaltenden Schicht zwischen einem ersten textilen Flächenmaterial und einem zweiten textilen Flächenmaterial angeordnet ist. Gleichermaßen kann es erfindungsgemäß vorgesehen sein, dass die Aktivkohle sozusagen in Form einer losen Schüttung in der Wundauflage vorliegt. In diesem Zusammenhang kann es beispielsweise vorgesehen sein, dass die Schüttung zwischen einem ersten textilen Flächenmaterial und einem zweiten textilen Flächenmaterial vorliegt bzw. eingebracht ist. Alternativ kann die lose Schüttung der Aktivkohle auch zwischen der Hydrokolloidschicht und einer äußeren Abdeckschicht vorliegen. Gemäß einer weiteren Ausführungsform kann es zudem vorgesehen sein, das die Aktivkohle in ein textiles Flächenmaterial eingebracht ist und sozusagen als "Aktivkohlekissen", welches Aktivkohle in loser Schüttung beinhaltet, in der Wundauflage vorliegt.

Was die Beschaffenheit des erfindungsgemäß eingesetzten textilen Flächenmaterials anbelangt, so ist es bevorzugt, wenn dieses als Gewebe, Gewirke, Gestricke, Gelege, Textilverbundstoff, Vlies oder Filamentfasermaterial, insbesondere als Filamentfasermaterial, ausgebildet ist.

Weiterhin kann es im Rahmen der vorliegenden Erfindung vorgesehen sein, dass das erste textile Flächenmaterial und/oder das zweite textile Flächenmaterial auf einer Faserart, ausgewählt aus der Gruppe von Polyestern (PES); Polyolefinen, insbesondere Polyethylen (PE) und/oder Polypropylen (PP), Polyvinylchlorid (PVC); Polyvinylidenchlorid (PVDC); Celluloseacetat (CA); Cellulosetriacetat (CTA); Polyacryl (PAN); Polyamid (PA); Polyvinylalkohol (PVAL); Polyurethanen (PU); Polyvinylestern; (Meth-)Acrylaten; sowie deren Mischungen, insbesondere Celluloseacetat und/oder Polyamid, basiert. Die vorgenannten Flächenmaterialien zeichnen sich insbesondere durch ihre hervorragende physiologische Verträglichkeit aus, so dass bei der Anwendung im Allgemeinen nicht mit allergischen und/oder toxischen Reaktionen zu rechnen ist.

Darüber hinaus ist es erfindungsgemäß bevorzugt, wenn das erste textile Flächenmaterial und/oder das zweite textile Flächenmaterial zumindest im Wesentlichen keine Fasern und/oder zumindest im Wesentlichen keine Aktivkohle freisetzen kann, so dass die Wunde durch das Fasermaterial nicht verunreinigt wird bzw. keine Fremdkörper in die Wunde eindringen.

Was speziell die Fixierung der Aktivkohle an der erfindungsgemäßen Wundauflage, insbesondere an dem textilen Flächenmaterial, anbelangt, so ist es erfindungsgemäß bevorzugt, wenn die Aktivkohle der Aktivkohle enthaltenden Schicht an dem ersten textilen Flächenmaterial und/oder an dem zweiten textilen Flächenmaterial befestigt ist, insbesondere mittels eines vorzugsweise medizinisch und/oder physiologisch kompatiblen Klebstoffs. In diesem Zusammenhang ist es weiterhin bevorzugt, wenn der Klebstoff diskontinuierlich und/oder punktförmig auf dem ersten und/oder zweiten textilen Flächenmaterial aufgetragen ist, so dass eine gute Gas- und Luftdurchlässigkeit des Flächenmaterials gewährleistet ist, und zudem die Aktivkohle nicht vollständig mit Klebstoff bedeckt ist und somit weiterhin gut zugänglich bleibt. Was den Einsatz des Klebstoffs anbelangt, so ist es bevorzugt, wenn dieser auf dem ersten und/oder zweiten textilen Flächenmaterial in einer Auflagemenge von 1 bis 100 g/m², insbesondere 5 bis 50 g/m², vorzugsweise 10 bis 40 g/m², aufgetragen ist. Gleichermaßen kann es erfindungsgemäß vorgesehen sein, dass der Klebstoff das erste und/oder zweite textile Flächenmaterial jeweils zu höchstens 70 %, insbesondere zu höchstens 60 %, vorzugsweise zu höchstens 50 %, bevorzugt zu höchstens 40 %, besonders bevorzugt zu höchstens 30 %, bedeckt; auf diese Weise wird eine sichere und stabile Fixierung der Aktivkohle bei dennoch guter Zugänglichkeit für die zu adsorbierenden Stoffe und hoher Gas- bzw. Luftdurchlässigkeit gewährleistet. Schließlich sollte der Klebstoff in einer solchen Menge und/oder mit einer solchen Beschaffenheit eingesetzt werden, dass die Oberfläche der Aktivkohle zu mindestens 50 %, insbesondere mindestens 60 %, vorzugsweise mindestens 70 % nicht mit Klebstoff bedeckt ist bzw. frei zugänglich ist; auf diese Weise wird, wie zuvor angeführt, eine sichere Fixierung bzw. Befestigung der Aktivkohle und eine hohe Wirksamkeit der Aktivkohle gewährleistet.

Auch kann es im Rahmen der vorliegenden Erfindung vorgesehen sein, die Aktivkohle in die Hydrokolloid, vorzugsweise Kollagen, enthaltende Schicht einzulagern und/oder an die Hydrokolloid, vorzugsweise Kollagen, enthaltende Schicht anzulagern und/oder zu fixieren.

Was die vorherigen Ausführungen im Zusammenhang mit der Aktivkohle enthaltenden Schicht anbelangt, so ist hierbei anzumerken, dass es sich nicht notwendigerweise um zusammenhängende Schichten handeln muss.

Zudem kann die erfindungsgemäße Wundauflage gemäß einer weiteren erfindungsgemäßen - wenngleich weniger bevorzugten - Ausführungsform derart realisiert werden, dass die Aktivkohle in eine wiederum weitere, insbesondere separate und/oder zusätzliche Schicht inkorporiert und/oder hieran fixiert ist. Diese separate und/oder zusätzliche Schicht, welche Bestandteil der erfindungsgemäßen Wundauflage ist, kann auch zusätzlich weitere Komponenten, insbesondere mindestens einen Wirkstoff, insbesondere wie er im Folgenden noch beschrieben wird, enthalten.

Um eine ausreichende Zugänglichkeit der Aktivkohle für die zu adsorbierenden Stoffe zu gewährleisten, ist es zudem erfindungsgemäß bevorzugt, wenn die Oberfläche der Aktivkohle der Aktivkohle enthaltenden Schicht zu mindestens 50 %, insbesondere zu mindestens 60 %, vorzugsweise zu mindestens 70 %, frei zugänglich ist und/oder nicht bedeckt ist. Dies wird - unabhängig davon, in welcher Form oder in welcher Schicht die Aktivkohle vorliegt - üblicherweise in der erfindungsgemäßen Wundauflage realisiert.

Was die erfindungsgemäße Ausgestaltung der Wundauflage weiterhin anbelangt, so ist es bevorzugt, wenn die einzelnen Schichten der Wundauflage jeweils miteinander verbunden sind bzw. wenn die einzelnen Schichten der Wundauflage einen Verbund ausbilden, so dass bei Anwendung und/oder Applikation der Wundauflage eine ausreichende Stabilität gewährleistet ist.

Um die Wirksamkeit der erfindungsgemäßen Wundauflage in Bezug auf die Beschleunigung der Wundheilung weiter zu verbessern und darüber hinaus einen verbesserten Kontaminationsschutz bereitzustellen, so kann es erfindungsgemäß vorgesehen sein, dass die Wundauflage weiterhin mindestens einen Wirkstoff enthält, welcher insbesondere ausgewählt sein kann aus der Gruppe von antimikrobiell wirkenden Wirkstoffen, desinfizierenden Wirkstoffen, entzündungshemmenden Wirkstoffen, blutstillenden Wirkstoffen und wundheilungsfördernden Wirkstoffen.

Somit ist es in diesem Zusammenhang erfindungsgemäß bevorzugt, dass die Wundauflage mit mindestens einem antimikrobiellen und/oder desinfizierenden und/oder entzündungshemmenden und/oder blutstillenden und/oder wundheilungsfördernden Wirkstoff ausgerüstet ist bzw. dass die Wundauflage mindestens einen antimikrobiellen und/oder desinfizierenden und/oder blutstillenden und/oder wundheilungsfördernden und/oder entzündungshemmenden Wirkstoff aufweist. Auf diese Weise wird ein verstärkter Schutz der zu behandelnden Wunde vor Kontaminationen, insbesondere auch in Bezug auf die häufig antibiotikaresistenten Krankenhaus- bzw. Hospitalkeime, ermöglicht. Darüber hinaus kann die Wundheilung durch den Einsatz dieser Wirkstoffe aktiv unterstützt werden.

In diesem Zusammenhang hat es sich als besonders vorteilhaft erwiesen, wenn der Wirkstoff biozide und/oder biostatische Wirkung, insbesondere bakterizide oder bakteriostatische und/oder fungizide oder fungistatische und/oder viruzide oder virustatische Wirkung, hat. Auf diese Weise können Krankheitserreger, wie Bakterien, Pilze oder Viren, nicht nur in ihrem Wachstum gehemmt werden, sondern darüber hinaus auch aktiv abgetötet werden.

Was die einzusetzenden Wirkstoffe als solche anbelangt, so hat es sich als besonders wirkungsvoll erwiesen, wenn der Wirkstoff ein Antiseptikum und/oder Desinfektionsmittel ist.

Unter einem Desinfektionsmittel werden solche insbesondere chemischen Mittel verstanden, welche der Abtötung pathogener Erreger an Organismen und Gegenständen dienen. Das Wirkspektrum von Desinfektionsmitteln umfasst im Allgemeinen pathogene Mikroorganismen, zu denen in diesem Zusammenhang Bakterien, Viren, Sporen, Klein- und Schimmelpilze gezählt werden. Was den Begriff "Antiseptikum" anbelangt, so bezeichnet dieser ebenfalls keimtötende Mittel, mit welchen insbesondere Wunden, Haut sowie Schleimhäute und medizinisch verwendete Gegenstände behandelt werden, um eine weitgehende Keimfreiheit zu erzielen. Für weitergehende Einzelheiten zu den Begriffen "Desinfektionsmittel" bzw. "Antiseptikum" kann verwiesen werden auf Römpp Chemielexikon, 10. Auflage, 1997, Georg Thieme Verlag, Stuttgart/New York, Stichwort: "Desinfektionsmittel", Seiten 905 und 906 sowie Stichwort: "Antiseptika", Seite 132, sowie die hierin referierte Literatur.

In diesem Zusammenhang ist es bevorzugt, wenn der Wirkstoff, insbesondere das Desinfektionsmittel, ausgewählt ist aus der Gruppe von Polyhexamethylenbiguanid (Polyhexanid), Taurolidin, Benzalkoniumchlorid, Chlorhexidin, Octenidin und deren physiologisch verträglichen Salzen und Derivaten sowie deren Mischungen, vorzugsweise aus Octenidin und/oder Polyhexamethylenbiguanid (Polyhexanid). Die zuvor genannten Wirkstoffe, insbesondere Octenidin und Polyhexanid, sind besonders gut verträglich und besitzen ein breites Wirkspektrum gegenüber zahlreichen Krankheitserregern. Darüber hinaus können insbesondere Nebenwirkungen, wie sie mit Silber oder anderen Edelmetallen unter Einsatz als Bakteriostatikum einhergehen, durch die Verwendung der zuvor genannten Wirksubstanzen verhindert werden. Der zusätzliche Einsatz eines Desinfektionsmittels geht insbesondere mit dem Vorteil einher, dass - ohne sich auf diese Theorie beschränken zu wollen - die Wundheilung durch eine weiterführende Reduktion der Infektionsrate bzw. durch eine Verringerung des Bakterienbefalls beschleunigt werden kann.

Das erfindungsgemäß verwendete Desinfektionsmittel Octenidin kann insbesondere in Form des Breitbandantiseptikums Octenidin-Dihydrochlorid eingesetzt werden. In chemischer Hinsicht gehört Octenidin zu der Gruppe der quartären Ammoniumverbindungen. Octenidin zeichnet sich bei der Anwendung auf der Haut insbesondere durch eine gute Verträglichkeit aus, was das Auftreten von Nebenwirkungen minimiert. Darüber hinaus besitzt Octenidin ein äußerst breites Wirkspektrum, welches sowohl Gram-positive als auch Gram-negative Bakterien und zudem eine Vielzahl von Viren und Pilzen umfasst. Für weitergehende Einzelheiten zu Octenidin kann verwiesen werden auf Römpp, Chemielexikon, 10. Auflage, Georg Thieme Verlag, Stuttgart/New York, Seite 2986, Stichwort: "Octenidin-Dihydrochlorid", sowie die darin referierte Literatur.

Gemäß einer weiteren bevorzugten Ausführungsform kann im Rahmen der vorliegenden Erfindung als Desinfektionsmittel Polyhexanid eingesetzt werden. Hierbei handelt es sich um ein Desinfektionsmittel aus der Gruppe der Biguanide, welche im Allgemeinen ein hydrophobes Rückgrat mit mehreren kationischen Biguanidgruppen aufweisen, wobei die Anzahl der Biguanidreste im Molekül variabel ist und dessen antimikrobielle bzw. bakteriostatische Wirksamkeit beeinflusst. Polyhexanid bzw. Polyhexanidlösungen liegt bzw. liegen somit in Form von Gemischen auf Basis von Polymeren mit unterschiedlichen Molekülgrößen vor. Die Anzahl der Biguanidreste pro Molekül liegt im Allgemeinen im Bereich von 2 bis 40. Die einzelnen Biguanidreste sind dabei über eine Hexamethylenkette voneinander getrennt.

Polyhexanid wirkt - ohne sich hierbei auf diese Theorie beschränken zu wollen - aufgrund der Protonierung der Biguanidreste im neutralen pH-Bereich als starke Base. Durch die stark basische Wirkung - ebenfalls ohne sich hierbei auf diese Theorie beschränken zu wollen - treten die Polyhexanid-Moleküle durch elektrostatische Wechselwirkungen mit der negativ geladenen Zellmembran der zugrundeliegenden pathogenen Keime in Wechselwirkung, was zu einer Destabilisierung bzw. Desintegration der zellulären Strukturen führt und einen Zelltod bewirken kann.

Insgesamt besitzt Polyhexanid als Desinfektionsmittel eine weitgehende unspezifische Wirkweise, so dass es auch schwer hemmbare Keime, wie beispielsweise *Staphylococcus aureus, Bacillus subtilis, Pseudomonas aeruginosa* und *Escherichia coli,* in effizienter Weise in ihrem Wachstum gehemmt werden können. Neben der vorgenannten antibakteriellen Wirkung ist Polyhexanid darüber hinaus auch antiviral und antifungizid wirksam.

Ein weiterer Vorteil, welcher mit dem Einsatz von Polyhexanid einhergeht, liegt zudem darin, dass aufgrund der unspezifischen Wirkweise im Gegensatz zu Antibiotika im Allgemeinen keine Resistenzbildung resultiert. Darüber hinaus zeichnet sich Polyhexanid bei breiter antimikrobieller Wirksamkeit auch durch eine hervorragende Toleranz und (Gewebe-)Verträglichkeit aus, so dass die Anwendung auch über einen längeren Zeitraum möglich ist.

Nicht zuletzt bei chronischen Wunden wird aufgrund des Einsatzes von Polyhexanid zudem die Wundheilung insbesondere aufgrund eines reduzierten Bakterienbefalls bzw. einer reduzierten Infektionsrate beschleunigt.

Gemäß einer weiteren erfindungsgemäßen Ausführungsform kann es zudem vorgesehen sein, dass das Desinfektionsmittel, insbesondere Polyhexanid, in Gegenwart mindestens einer viskositiätserhöhenden und/oder matrixbildenden Substanz, insbesondere auf Basis eines organischen Polymers, vorzugsweise eines Polyalkylenglykols, bevorzugt Polyethylenglykol und/oder Polypropylenglykol, eingesetzt ist. Bei einer derartigen Substanz kann es sich insbesondere um das kommerziell erhältliche Macrogolum^{®} 4000 handeln. Hierdurch kann die Wirkeffizienz des Desinfektionsmittels weiter gesteigert werden.

Gemäß einer weiteren erfindungsgemäßen Ausführungsform kann es auch vorgesehen sein, dass der Wirkstoff ein Wirkstoff mit wundheilungsfördernder Wirkung ist, welcher insbesondere ausgewählt sein kann aus der Gruppe von Alginaten, Chitosan, Hyaluronsäure und deren Salzen, Allantoin, beta-Sitosterol, Bromelain, Dexpanthenol, Pantothensäure, Harnstoff, Flavonoiden, Riboflavin, Saponinen, Cineol, Tocopherol und deren Mischungen.

Die eingesetzte Wirkstoffmenge kann in weiten Bereichen variieren. Im Rahmen der vorliegenden Erfindung hat sich überraschend gezeigt, dass eine besonders gute Wirksamkeit mit einer Wirkstoffmenge, insbesondere Auflagemenge, von 0,00001 bis 5 g/cm², insbesondere 0,0001 bis 2 g/cm², vorzugsweise 0,001 bis 1 g/cm², besonders bevorzugt 0,01 bis 0,5 g/cm², erzielt werden kann.

Gemäß einer bevorzugten Ausführungsform der vorliegenden Erfindung kann es vorgesehen sein, dass der Wirkstoff in der ein Hydrokolloid, vorzugsweise Kollagen, enthaltenden Schicht und/oder in der Aktivkohle enthaltenden Schicht vorliegt.

Der Wirkstoff kann jeweils entweder nur in der ein Hydrokolloid, vorzugsweise Kollagen, enthaltenden Schicht oder aber alternativ nur in der Aktivkohle enthaltenden Schicht inkorporiert sein. Die Einbringung des Wirkstoffs in die Hydrokolloid- bzw. Kollagenschicht führt dazu dass, eine direkte bzw. unmittelbare Freisetzung des Wirkstoffs aus der Hydrokolloid- bzw. Kollagenschicht in die Wunde erfolgt, während die Einbringung des Wirkstoffs in die Aktivkohleschicht mit dem Vorteil verbunden ist, dass der in der Aktivkohleschicht vorliegende Wirkstoff retardiert bzw. über einen verlängerten Zeitraum kontrolliert freigesetzt bzw. an die Wunde abgegeben wird (d. h. sozusagen eine Depotwirkung erreicht wird).

Erfindungsgemäß besonders bevorzugt ist jedoch die Einbringung des Wirkstoffs sowohl in die Hydrokolloid- bzw. Kollagenschicht als auch in die Aktivkohleschicht. Diese Ausführungsform ist insbesondere mit dem Vorteil verbunden, dass auf diese Art und Weise sozusagen eine doppelte Wirkung erzeugt werden kann, da die Freisetzung des Wirkstoffs aus der Hydrokolloid- bzw. Kollagenschicht direkt bzw. unmittelbar in die Wunde erfolgt, während die in der Aktivkohleschicht vorliegenden Wirkstoffe retardiert freigesetzt werden, wodurch auch eine Versorgung der Wunde mit dem jeweiligen Wirkstoff über einen längeren Zeitraum mit kontrollierter Abgabemenge sichergestellt werden kann.

Grundsätzlich ist es jedoch auch möglich, den Wirkstoff - zumindest teilweise - in andere Schichten der erfindungsgemäßen Wundauflage als die die Aktivkohle enthaltende Schicht oder die das Hydrokolloid, vorzugsweise Kollagen, enthaltende Schicht einzubringen, sofern solche Schichten vorhanden sind (z. B. in die optionalen textilen Träger bzw. Trägerschichten etc.). Des Weiteren ist es auch möglich, eine oder mehrere separate bzw. zusätzliche Schichten speziell für die Einbringung des oder der Wirkstoffe vorzusehen.

Was die Einbringung des Wirkstoffs in die Hydrokolloid- bzw. Kollagenschicht anbelangt, so kann dieser direkt bei der Herstellung der Hydrokolloid- bzw. Kollagenschicht in die Lösung bzw. Dispersion inkorporiert werden.

Das Einbringen des Wirkstoffs in die Aktivkohleschicht kann insbesondere durch Inkontaktbringen, vorzugsweise Imprägnieren, der Aktivkohle mit dem Wirkstoff bzw. einer Wirkstofflösung erfolgen.

Was die sprachliche Formulierung "dass der Wirkstoff in der Hydrokolloidschicht, vorzugsweise Kollagenschicht, und/oder Aktivkohleschicht vorliegt" anbelangt, so ist hierunter insbesondere zu verstehen, dass der Wirkstoff in die jeweilige Schicht eingebracht oder inkorporiert ist, insbesondere in der bzw. an die jeweilige Schicht fixiert, vorzugsweise reversibel fixiert ist und somit vorzugsweise bei Kontakt mit der Wunde bzw. mit Wasser bzw. Feuchtigkeit wieder freigesetzt bzw. an die Wunde abgegeben wird.

Darüber hinaus kann es gemäß einer besonderen Ausführungsform der vorliegenden Erfindung vorgesehen sein, dass die Wundauflage mit mindestens einer Substanz, welche Protease-Aktivität besitzt, ausgerüstet ist. Auch hier kann es vorgesehen sein, dass die Substanz mit Protease-Aktivität in der Hydrokolloid- bzw. Kollagenschicht und/oder in der mit Aktivkohle ausgerüsteten Schicht vorliegt. Durch den zweckgerichteten Einsatz insbesondere geringer Mengen von einer Substanz mit Protease-Aktivität kann erreicht werden, das Erfordernis eines Debridements zu verringern.

Weitere Vorteile, Eigenschaften und Merkmale der vorliegenden Erfindung ergeben sich aus der folgenden Beschreibung von in den Zeichnungen dargestellten, bevorzugten Ausführungsbeispielen. Es zeigt:
- Fig. 1: eine schematische Schnittdarstellung durch den Schichtaufbau einer Wundauflage gemäß einem ersten bevorzugten Ausführungsbeispiel der vorliegenden Erfindung entsprechend einer speziellen Ausführungsform;
- Fig. 2: eine schematische Schnittdarstellung durch den Schichtaufbau einer Wundauflage gemäß einem weiteren bevorzugten Ausführungsbeispiel der vorliegenden Erfindung entsprechend einer weiteren speziellen Ausführungsform.

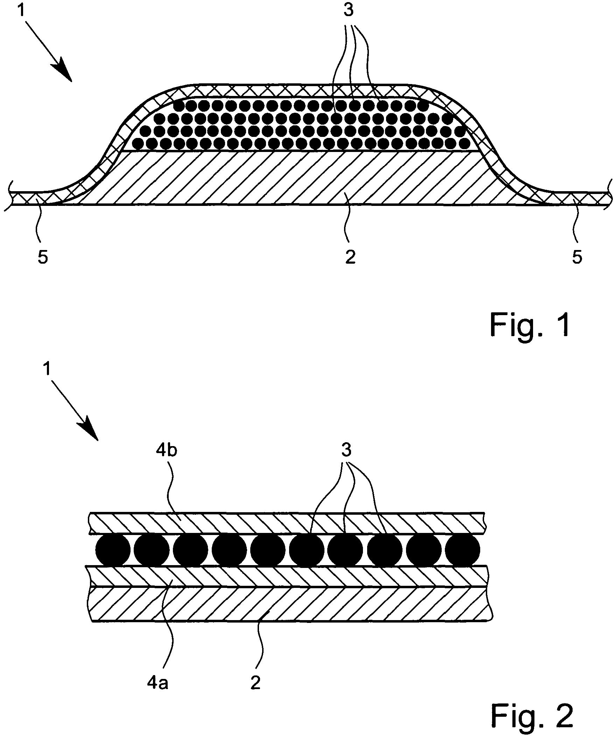

Fig. 1 zeigt eine schematische Schnittdarstellung durch den Schichtaufbau einer erfindungsgemäßen Wundauflage 1 entsprechend einer speziellen Ausgestaltung der vorliegenden Erfindung. Die erfindungsgemäße, insbesondere zur therapeutischen Wundversorgung geeignete Wundauflage 1 weist eine Aktivkohle enthaltende Schicht 3 sowie eine Kollagen enthaltende Schicht 2 auf, wobei die Kollagenschicht 2 im Anwendungszustand die der Wunde zugewandte Schicht darstellt. Gemäß dem dargestellten Ausführungsbeispiel kann die Wundauflage 1 einen Haftrand 5 aufweisen, welcher einerseits eine Fixierung der Wundauflage 1 bei ihrer Anwendung insbesondere auf der Haut ermöglicht und andererseits die einzelnen Schichten 2, 3 zusammenhält. Um eine ausreichende Stabilität der erfindungsgemäßen Wundauflage 1 zu gewährleisten, sind die zuvor beschriebenen Schichten als Verbund ausgebildet.

Fig. 2 zeigt eine schematische Schnittdarstellung durch den Schichtaufbau einer erfindungsgemäßen Wundauflage 1 entsprechend einer weiteren speziellen Ausgestaltung. Die erfindungsgemäße Wundauflage 1, welche insbesondere zur therapeutischen Wundversorgung geeignet ist, weist eine Aktivkohle enthaltende Schicht 3 auf, wobei die Aktivkohle zwischen zwei textilen Trägermaterialien 4a und 4b angeordnet, insbesondere hieran jeweils fixiert, ist und das textile Trägermaterial 4b eine erste Außenschicht der Wundauflage 1 bildet. Weiterhin weist die erfindungsgemäße Wundauflage 1 eine Kollagen enthaltende Schicht 2 auf, welche die zweite Außenschicht der erfindungsgemäßen Wundauflage 1 darstellt, wobei die Kollagenschicht 2 im Anwendungszustand die der Wunde zugewandte Schicht darstellt. Um eine ausreichende Stabilität der erfindungsgemäßen Wundauflage 1 zu gewährleisten, sind die zuvor beschriebenen Schichten als Verbund ausgebildet.

Im Rahmen der vorliegenden Erfindung wird somit eine Wundauflage mit bioziden und/oder biostatischen, insbesondere antimikrobiellen und wundheilungsfördernden Eigenschaften bereitgestellt, wobei die vorgenannten Eigenschaften insbesondere auch durch eine biostatisch oder biozid, insbesondere antimikrobiell wirksame Aktivkohle gewährleistet werden. Neben der Bindung von Geruchsstoffen und Toxinen kann die Aktivkohle Krankheitserreger (wie z. B. Pilze, Bakterien und Viren) inaktivieren bzw. abtöten, da diese ebenfalls an der Aktivkohle haften. Auf diese Art und Weise wird die Bakterienlast bzw. die Keimzahl in Wunden effektiv und dauerhaft minimiert. Aufgrund der hervorragenden bioziden oder biostatischen, insbesondere antimikrobiellen Eigenschaften der Aktivkohle kann die Konzentration von weiteren antimikrobiell wirksamen Mitteln gesenkt bzw. auf deren Einsatz gänzlich verzichtet werden, was entsprechend zu einer Verminderung des toxikologischen Potentials der Wundauflage führt. Insgesamt wird die Wundheilung insbesondere durch die Bindung der Toxine, durch das Exsudatmanagement zur Aufrecherhaltung der für die Wundheilung optimalen feucht-warmen Bedingungen und durch einen effektiven Gasaustausch gefördert. Darüber hinaus kann die Wirksamkeit noch gesteigert bzw. optimiert werden, indem auch noch weitere (Wirk-)Stoffe, z. B. wundheilungsfördernde Substanzen, in die Wundauflage integriert werden; aufgrund der hervorragenden Eigenschaften der Aktivkohle als solcher sind jedoch die hierzu gegebenenfalls einzusetzenden Mengen gegenüber den im Stand der Technik notwendigen Mengen deutlich geringer, so dass die Verträglichkeit der erfindungsgemäßen Wundauflage wesentlich besser ist.

Der vorliegende Erfindung stellt somit insbesondere eine Wundauflage mit einer bioziden oder biostatischen, insbesondere antimikrobiellen Aktivkohle bereit; letztere ist in der Lage - gegebenenfalls auch ohne zusätzlichen Einsatz eines antimikrobiell wirksamen Mittels - neben der Adsorption von Toxinen und Geruchsstoffen gleichermaßen Krankheitserreger (z. B. Pilze, Bakterien und Viren) dauerhaft zu inaktivieren bzw. abzutöten. Infolgedessen kann das toxikologische Risiko, welches durch die Verwendung von hohen Konzentrationen antimikrobieller Substanzen nach dem Stand der Technik zur Bereitstellung eines effektiven Kontaminationsschutzes notwendig wäre, gesenkt werden. Darüber hinaus wird die Wundheilung gefördert, da die Verwendung der Aktivkohle eine Reinigung des Exsudats durch die Adsorption von Toxinen ermöglicht. Zudem wirkt die Aktivkohle sozusagen als Sorptionsspeicher für das Exsudat, so dass dieses zur Aufrechterhaltung eines feuchten, aber nicht nassen Wundmilieus aufgenommen, aber auch wieder an die Wunde abgegeben werden kann. Darüber hinaus ist die gezielte Abgabe zusätzlicher Wirkstoffe aus der Aktivkohle möglich. Durch einen guten Gasaustausch über die Wundauflage werden die Wundheilungsprozesse weiterhin beschleunigt.

Die erfindungsgemäß einsetzte Aktivkohle mit biostatischen oder bioziden, insbesondere antimikrobiellen Eigenschaften ist somit in der Lage, einerseits Toxine und Geruchsstoffe zu binden und andererseits auch als Kontaminationsschutz zu wirken.

Die Wirkweise der Aktivkohle wird nun im Folgenden noch näher beschrieben, wobei die nachfolgenden Ausführungen die vorliegende Erfindung in keiner Weise beschränken sollen:
Die Aktivkohle ist in der Lage, Krankheitserreger (z. B. Pilze, Bakterien und Viren) abzutöten bzw. dauerhaft zu inaktivieren, da diese an ihr haften und somit immobilisiert werden. Die auf diese Weise erzeugte Bewegungsunfähigkeit bzw. Immobilisierung verhindert die Vermehrung der Krankheitserreger; zudem werden etwaigen Erregern durch die starken Anziehungskräfte der Aktivkohle Nährstoffe entzogen, die ebenfalls immobilisiert werden und für die Krankheitserreger nicht mehr zugänglich sind. Weiterhin verursacht die Aktivkohle aufgrund der starken Wechselwirkungen Schäden an der Zellmembran und der Zellwand der Erreger.

Die hervorragende Adsorptionsfähigkeit der Aktivkohle wird insbesondere durch die texturellen Eigenschaften ihrer (inneren) Oberfläche, insbesondere durch elektrostatische Wechselwirkungen und Van-der-Waals-Kräfte, bedingt. Die genannten Effekte bewirken eine langfristige Reduktion der Bakterienlast bzw. Keimzahl in der Wunde und im Ergebnis eine Minimierung der Kontaminationsgefahr.

Wie zuvor bereits ausgeführt, kann die biostatisch oder biozid, insbesondere antimikrobiell wirksame Aktivkohle in einer oder mehrerer Schicht(en) der Wundauflage enthalten sein. Gemäß einer besonderen Ausführungsform der vorliegenden Erfindung kann die Aktivkohle separat, z. B. als Appretur, auf einem textilen Flächenmaterial in der Wundauflage vorliegen. Das textile Flächenmaterial kann darüber hinaus über die Wahl der Polymere bzw. der daraus entstehenden Filamente, Fasern und Garne gezielt zur Modulierung bzw. Einstellung von Gas- und Flüssigkeitsdurchlässigkeit verwendet werden. Dies ist insbesondere in Bezug auf die verschiedenen Wundheilungsstadien von Bedeutung, da diese teilweise unterschiedliche Bedingungen an den Feuchtigkeitsgehalt sowie die Gaszusammensetzung im Wundgebiet stellen.

Gemäß einer weiteren Ausführungsform der vorliegenden Erfindung kann es vorgesehen sein, die Aktivkohle in Form eines Kissens vollständig von einem textilen Flächenmaterial zu umhüllen und an diesem mit einem Haftmittel zu fixieren. Dadurch wird ein Herauslösen der Aktivkohle verhindert. Durch den Einsatz eines textilen Flächenmaterials in Form eines Gewirks kann in diesem Zusammenhang gewährleistet werden, dass die Wundauflage weiterhin permeabel ist und dass Krankheitserreger mit dem Wundexsudat an die antimikrobiell wirksame Aktivkohle gelangen. Alternativ können auch Vliese oder Gewebe verwendet werden. Kommt das textile Flächenmaterial direkt mit der Verletzung in Berührung, wird erfindungsgemäß ein nicht an der Wunde anhaftendes Garn verwendet, um Verletzungen beim Verbandswechsel zu vermeiden. Eine solche Schicht kann mit verschiedenartigen Substanzen - welche für den Heilungsprozess von Bedeutung sind und wie sie zuvor explizit aufgeführt worden sind - ausgerüstet sein. Gemäß einer besonderen Ausführungsform der Erfindung handelt es sich um eine Schicht mit wundheilungsfördernden Substanzen, wie z. B. Alginat, Chitosan oder Hyaluronsäure. Möglich ist aber auch die Zugabe von anderen Substanzen, wie z. B. Allantoin, beta-Sitosterol, Harnstoff, Bromelain, Dexpanthenol, Flavonoiden, Riboflavin, Saponin, Cineol, Tocopherol und weiteren Substanzen dieser Art.

Gemäß einer besonderen Ausführungsform der vorliegenden Erfindung kann es zudem vorgesehen sein, dass das textile Flächenmaterial an einer Schicht aus resorbierbarem Hydrokolloid, insbesondere Kollagen, haftet, welches in diesem Zusammenhang mehrere Funktionen erfüllt: Durch eine solche Kollagenschicht werden aufgrund ihrer schaumig weichen Struktur Unebenheiten ausgeglichen. Darüber hinaus wird der Abstand zwischen der Wunde und der antimikrobiell wirksamen Aktivkohle verringert, was deren Effektivität erhöht. Außerdem entsteht durch die Porenstruktur der Kollagenmatrix eine hohe Kapillaraktivität, welche die Aufnahme und Weiterleitung von großen Flüssigkeitsmengen, insbesondere Wundwasser, ermöglicht. Infolgedessen wird ein feuchtes Wundmilieu bereitgestellt, welches eine der Wundheilung abträgliche Mazeration verhindert. Darüber hinaus wird der Exsudatfluss aus der Wunde heraus und hin zur Aktivkohle gewährleistet und überflüssige Feuchtigkeit in Form von Wasserdampf abgegeben. Verunreinigungen, Proteasen sowie freie Radikale werden sowohl von der Aktivkohle als auch von einer solchen Kollagenschicht auf unterschiedliche Weise gebunden und aus der Wunde entfernt.

Die biostatischen bzw. bioziden, insbesondere antimikrobiell wirksamen Eigenschaften der Aktivkohle verhindern zudem die Entstehung eines Biofilms bzw. einer Bakterienschicht und ermöglichen einen stabilen, lang anhaltenden Reinigungsprozess des Wundexsudats. Würde ein Biofilm entstehen, wäre dies auch insofern der Wundheilung abträglich, als der Kontakt der Flüssigkeit mit der Aktivkohle unterbunden und auf diese Weise der Gasaustausch verhindert wäre. Da die biostatische bzw. biozide, insbesondere antimikrobielle Wirkung durch die Aktivkohle als solche bereitgestellt wird, ist auch die Erzeugung eines Kontaminationsschutzes durch verminderte Sauerstoffzufuhr nicht erforderlich, wie es jedoch im Stand der Technik vielfach realisiert werden muss. Der im Rahmen der vorliegenden Erfindung verstärkte Gasaustausch sowie das verbesserte Exsudatmanagement gewährleisten insgesamt eine verbesserte Wundheilung.

Entsprechend einer weiteren Ausführungsform kann die erfindungsgemäße Wundauflage einen zusätzlichen, insbesondere antimikrobiellen Wirkstoff enthalten, um die Wirkweise der Aktivkohle in synergistischer Wirkweise zu unterstützen. Dabei kann es sich insbesondere um Polyhexamethylenbiguanid (PHMB), Chlorhexidin oder Octenidin handeln, wobei auch jedes andere Antiseptikum und/oder Desinfektionsmittel verwendet werden kann, wie beispielsweise Chitosan oder Triclosan.

Soll eine besonders starke antimikrobielle Wirkung erzielt werden, wie z. B. im Fall einer Infektion mit mehreren Bakterienstämmen, kann es auch vorgesehen sein, die Wundauflage mit Antibiotika auszurüsten. Im Rahmen der Anwendung der Wundauflage wird der jeweilige Wirkstoff aus der Wundauflage herausgelöst, diffundiert in die Wunde und entfaltet im gesamten Wundbereich seine Aktivität. Dies ermöglicht auch eine Inaktivierung von Mikroorganismen in Wundvertiefungen. Durch die erfindungsgemäß eingesetzte Aktivkohle wird jedoch sichergestellt, dass auch im Falle des vollständigen Herauslösens des Wirkstoffs aus der Wundauflage die biostatische oder biozide, insbesondere antimikrobielle Wirkung immer noch ausreichend ist, um eine Rekontamination der Wunde zu verhindern. Dies stellt bislang im Stand der Technik ein nicht gelöstes Problem dar.

In diesem Zusammenhang ist es mittels der erfindungsgemäßen Wundauflage insbesondere möglich, die Konzentration optional einzusetzender biostatischer oder biozider, insbesondere antimikrobieller Substanzen im Vergleich zum Stand der Technik zu senken bzw. gänzlich hierauf zu verzichten und infolgedessen eine Minimierung des toxikologischen Risikos bzw. der diesbezüglichen Nebenwirkungen zu erreichen. Zudem können von außen keine Krankheitserreger in die Wunde gelangen, da die Aktivkohleschicht für sie unpassierbar ist.

Weiterhin kann es erfindungsgemäß vorgesehen sein, dass die Wundauflage zusätzlich noch analgetische oder schmerzstillende Wirkstoffe enthält. Dabei kann es sich um entzündungshemmende Stoffe, wie z. B. Ibuprofen und Diclofenac, sowie um schmerzstillende Stoffe, wie z. B. Lidocain und Procain, handeln.

Auch es möglich, dass die Wundauflage einen blutstillenden Wirkstoff (Hämostyptikum) enthält. Möglich sind ein oder mehrere spezifisch lokale sowie systemisch wirksame Hämostyptika. In einer besonders bevorzugten Variante handelt es sich um den Einsatz von hydrophilen, hochmolekularen Polymeren, wie z. B. Cellulosederivaten, die durch Kontaktaktivierung des endogenen Gerinnungssystems die Blutstillung begünstigen. Zudem ermöglichen diese Mittel eine Wundbettadaptation und fungieren zugleich als Klebemittel, was wiederum die Haftung der Wundauflage begünstigt.

Die vorgenannten Wirkstoffe können selbst eine eigenständige Schicht bilden, aber auch in eine oder mehrere Schicht(en) der Wundauflage integriert bzw. inkorporiert werden. Zudem beeinflussen der Aufbau der Wundauflage, die jeweils eingesetzten Wirkstoffkonzentrationen und Partikelgrößen sowie die Art der Bindung der wirksamen Substanzen in den einzelnen Schichten deren Löslichkeitsverhalten. Eine gezielte zeitliche Abgabe zu verschiedenen Phasen der Wundheilung ist dementsprechend möglich.

In einer weiteren bevorzugten Ausführungsform wird die Fixierung der Wundauflage im Gebrauchszustand durch einen Haftrand verstärkt. Hierbei handelt es sich insbesondere um eine haftende Fläche, welche sich z. B. auf den textilen Flächenmaterialien, zwischen welchen die Aktivkohle angeordnet bzw. fixiert sein kann und welche sozusagen eine Art "Aktivkohlekissen" ausbilden, befinden kann. Die haftende Fläche geht über die Kanten des "Aktivkohlekissens" hinaus und gewährleistet eine klebende Umrandung, so dass die Wundauflage auf diese Art und Weise stabil auf der Haut des Patienten fixiert werden kann. Auch ist es erfindungsgemäß möglich, in diese Fläche eine Sperr- oder Wäscheschutzschicht zu integrieren. Als Haftmittel werden bei der vorliegenden Erfindung in einer erfindungsgemäßen Wundauflage insbesondere Substanzen, wie z. B. Polyacrylat, Siloxan oder Polyisobutadien, verwendet. Durch eine solche Haftschicht kann auf die Verwendung eines Sekundärverbandes oder eines Folienklebers verzichtet werden. Darüber hinaus wird eine seitliche Abdichtung gewährleistet. Zudem werden weder die Wunde noch die umgebende Haut bei Verwendung eines zuvor beschriebenen Haftrandes bzw. einer solchen Haftschicht gereizt. Auch wird durch den Haftrand die Gefahr einer falschen Anwendung minimiert.

Um die Gefahr einer falschen Anwendung zu minimieren, kann es ebenfalls vorgesehen sein, die einzelnen Schichten zu färben oder deren Oberfläche zur Kenntlichmachung zu strukturieren.

Im Ergebnis wird im Rahmen der vorliegenden Erfindung somit eine effiziente Wundauflage mit verbessertem Wundheilungsprofil bereitgestellt.

Was die Herstellung der erfindungsgemäßen Wundauflage anbelangt, so kann diese in einer dem Fachmann an sich geläufigen Art und Weise erfolgen:
Zu diesem Zweck wird vorteilhafterweise zunächst eine hydrokolloidhaltige, insbesondere kollagenhaltige Schicht erzeugt, bevorzugterweise auf einem nach Herstellung der Hydrokolloid- bzw. Kollagenschicht wieder ablösbaren Träger. Wie zuvor geschildert, ist die hydrokolloidhaltige, insbesondere kollagenhaltige Schicht insbesondere durch Aufbringung einer Dispersion oder Lösung eines Kollagens auf einem geeigneten Träger mit nachfolgender Trocknung, insbesondere Gefriertrocknung, vorzugsweise unter Expansion des Kollagens erhältlich, so dass auf diese Weise ein Hydrokolloidvlies bzw. ein Hydrokolloidschaum, insbesondere ein Kollagenvlies bzw. Kollagenschaum erzeugt wird.

Im Rahmen eines Verfahrens zur Herstellung der erfindungsgemäßen Wundauflage ist es bevorzugt, zur Herstellung der mindestens ein Hydrokolloid, vorzugsweise Kollagen, enthaltenden Schicht eine Hydrokolloid- bzw. Kollagensuspension, vorzugsweise mit einer Hydrokolloid- bzw. Kollagen-Konzentration im Bereich von 0,1 bis 5 Gew.-%, insbesondere im Bereich von 0,5 bis 4 Gew.-%, vorzugsweise im Bereich von 0,7 bis 3 Gew.-%, besonders bevorzugt im Bereich von 1 bis 2 Gew.-%, bezogen auf die Hydrokolloid- bzw. Kollagensuspension, herzustellen. Als bevorzugtes Lösungsmittel für das Hydrokolloid, insbesondere Kollagen, wird Reinstwasser eingesetzt und der pH-Wert der Suspension wird vorzugsweise auf den Bereich von 2,5 bis 5, insbesondere auf einen Bereich von 3 bis 4, eingestellt.

Gemäß einer besonders bevorzugten Ausführungsform der vorliegenden Erfindung kann es vorgesehen sein, dass in die Hydrokolloid- bzw. Kollagensuspension ein desinfizierender und/oder antimikrobieller und/oder blutstillender und/oder wundheilungsfördernder und/oder entzündungshemmender Wirkstoff (wie zuvor definiert) eingebracht wird. Was die Menge des eingebrachten Wirkstoffs anbelangt, so ist diese variabel; diesbezüglich kann auf obige Ausführungen verwiesen werden.

Zur Entfernung der Flüssigkeit kann es vorgesehen sein, die Hydrokolloid- bzw. Kollagensuspension auf einen Träger aufzutragen, wobei über die Auftragungshöhe der Hydrokolloid- bzw. Kollagensuspension auf dem Träger die Dicke der resultierenden, zumindest im Wesentlichen trockenen Hydrokolloid bzw. Kollagen enthaltenden Schicht modifiziert werden kann. Die Trocknung erfolgt vorzugsweise auf dem Träger, insbesondere durch Lyophilisation (Gefriertrocknung). Die zumindest im Wesentlichen trockene Hydrokolloid- bzw. Kollagenschicht kann nachfolgend vom Trägermaterial entfernt werden (alternativ aber auch erst nach Herstellung des Gesamtverbunds).

Auf die auf diese Weise erzeugte Hydrokolloid- bzw. Kollagenschicht kann anschließend eine aktivkohlehaltige Schicht aufgebracht werden, wobei die Aufbringung mittelbar oder unmittelbar erfolgen kann. Insbesondere kann zwischen der Hydrokolloid- bzw. Kollagenschicht und der eigentlichen Aktivkohleschicht mindestens eine textile Lage vorgesehen sein, wie nachfolgend beschrieben.

Was die Aktivkohle der Aktivkohle enthaltenden Schicht der erfindungsgemäßen Wundauflage anbelangt, so ist es erfindungsgemäß bevorzugt, wenn diese auf einem flächigen, vorzugsweise textilen Träger angeordnet wird, vorzugsweise hieran befestigt wird.

Gemäß einer besonders bevorzugten Ausführungsform kann es vorgesehen sein, dass die Aktivkohle der Aktivkohle enthaltenden Schicht zwischen einem ersten textilen Flächenmaterial und einem zweiten textilen Flächenmaterial angeordnet wird. Insbesondere kann es in diesem Zusammenhang vorgesehen sein, dass die Aktivkohle der Aktivkohle enthaltenden Schicht an dem ersten textilen Flächenmaterial und/oder an dem zweiten textilen Flächenmaterial befestigt wird, insbesondere mittels eines vorzugsweise medizinisch und/oder physiologisch kompatiblen Klebstoffs.

Im Rahmen einer besonderen Ausführungsform kann es zudem vorgesehen sein, dass die Aktivkohle der Aktivkohle enthaltenden Schicht mit einem wundheilungsfördernden und/oder blutstillenden und/oder antimikrobiellen und/oder entzündungshemmenden und/oder desinfizierenden Wirkstoff versehen wird. In diesem Zusammenhang ist es besonders bevorzugt, wenn die Aktivkohle mit dem Wirkstoff bzw. einer Wirkstofflösung imprägniert wird.

Um eine hinreichende Stabilität der erfindungsgemäßen Wundauflage zu ermöglichen, kann es zudem vorgesehen sein, dass die zuvor genannten Schichten, nämlich insbesondere die Aktivkohleschicht, die ein Hydrokolloid, vorzugsweise Kollagen, enthaltende Schicht sowie die textilen Flächenmaterialien, als Verbund zusammengeführt werden. Dies kann insbesondere dadurch erfolgten, dass die Schichten mit einer äußeren Abdeckschicht versehen werden. In diesem Zusammenhang kann es insbesondere vorgesehen sein, dass die äußere Abdeckschicht in ihrer Fläche über den Verbund bzw. die Schichten hinausragt, wobei die hinausragenden Abschnitte sozusagen als Haftrand ausgestaltet sind. Über den Haftrand ist es möglich, die Wundauflage auf der Haut des Patienten bzw. Anwenders zu fixieren. Insbesondere kann es erfindungsgemäß vorgesehen sein, dass die Wundauflage mit mindestens einer Haft-, Sperr- und/oder Wäscheschutzschicht ausgerüstet ist.

Gemäß einer besonders bevorzugten Ausführungsform resultiert ein mehrlagiger Verbund aus äußerer Hydrokolloidschicht/textilem Flächenmaterial/Aktivkohleschicht/textilem Flächenmaterial mit der vorbezeichneten Abfolge der einzelnen Schichten.

Weiterer Gegenstand der vorliegenden Erfindung ist - gemäß einem **zweiten** Aspekt der vorliegenden Erfindung - die Verwendung einer Wundauflage, wie sie zuvor beschrieben wurde, zur therapeutischen, insbesondere topischen Wundversorgung des menschlichen oder tierischen Körpers, insbesondere zur therapeutischen, vorzugsweise topischen Behandlung von Wunden und/oder Gewebsunterbrechungen (d. h. mit anderen Worten betrifft die vorliegende Erfindung eine wie zuvor beschriebene Wundauflage zur Verwendung bei der therapeutischen, insbesondere topischen Wundversorgung des menschlichen oder tierischen Körpers, insbesondere zur therapeutischen, vorzugsweise topischen Behandlung von Wunden bzw. Gewebsunterbrechungen).

Hinsichtlich des Begriffs "Wunden" bzw. "Gewebsunterbrechnungen", so kann zur Vermeidung unnötiger Wiederholungen auf die im Rahmen der Beschreibungseinleitung genannten Erklärungen und Definitionen verwiesen werden.

Insbesondere werden im Rahmen der vorliegenden Erfindung hierunter sämtliche Klassen bzw. Arten von Wunden verstanden, wie sie insbesondere auch im einleitenden Teil aufgeführt sind. Unter einer mechanischen Wunde werden insbesondere Stich-, Schnitt-, Quetsch-, Platz-, Ritz- bzw. Schürfwunden verstanden. Zur Klasse der thermischen Wunden gehören insbesondere solche Gewebsunterbrechungen, welche durch die Einwirkung von extremer Kälte oder Hitze ausgelöst werden. Unter chemischen Wunden hingegen werden solche Wunden verstanden, welche unter Einwirkung von chemischen Substanzen, insbesondere Verätzungen durch Laugen bzw. Säuren, ausgelöst werden. Zu strahlenbedingten Wunden kommt es insbesondere durch die Einwirkung aktinischer bzw. ionisierender Strahlung. Darüber hinaus kann sich die Wunde in physiologischen Zuständen befinden, welche besonders hohe Anforderungen an die Behandlung bzw. Therapie stellen. So kommt es bei nekrotisierenden Wunden zum Auflösen des Zellverbandes bzw. zum Absterben von Gewebe. Auch ist es möglich, dass Wunden durch Krankheitserreger, wie Bakterien, Pilze oder Viren, infiziert werden. Weiterhin wird eine Wunde, welche nach einem Zeitraum von circa acht Wochen noch nicht vollständig verheilt ist, als chronische Wunde definiert. Unter einer chronischen Wunde werden z. B. einerseits Druckgeschwüre, wie sie häufig bei bettlägerigen Patienten auftreten, sowie andererseits Wunden, wie sie häufig mit Durchblutungsstörungen verbundenen Krankheiten, z. B. Diabetes Mellitus Typ 2 oder der chronisch-venösen Insuffizienz, einhergehen, bezeichnet.

Erfindungsgemäß ist es beispielsweise möglich, die erfindungsgemäße Wundauflage zur therapeutischen Behandlung von mechanischen Wunden, insbesondere Schnitt-, Stich-, Quetsch-, Platz-, Ritz- und/oder Schürfwunden, zu verwenden.

Auch ist eine Verwendung der erfindungsgemäßen Wundauflage zur therapeutischen Behandlung von thermischen Wunden, insbesondere durch Kälte- oder Hitzeverbrennung ausgelösten Wunden, möglich.

Weiterhin kann es vorgesehen sein, die erfindungsgemäße Wundauflage zur therapeutischen Behandlung von chemischen Wunden, insbesondere durch Verätzung mit Laugen und/oder Säuren ausgelösten Wunden, zu verwenden. Erfindungsgemäß kann es insbesondere vorgesehen sein, die Wundauflage zur therapeutischen Behandlung von nekrotisierenden und/oder infizierten und/oder chronischen Wunden einzusetzen.

Gleichermaßen kann es erfindungsgemäß auch vorgesehen sein, die erfindungsgemäße Wundauflage zur therapeutischen Behandlung von akuten Wunden einzusetzen.

Schließlich kann es erfindungsgemäß gleichermaßen vorgesehen sein, die erfindungsgemäße Wundauflage zur therapeutischen Behandlung von Druckgeschwüren und/oder von durch Durchblutungsstörungen ausgelösten Wunden zu verwenden.

Für weitergehende Einzelheiten zu diesem Erfindungsaspekt kann zur Vermeidung unnötiger Wiederholungen auf obige Ausführungen zu dem ersten Erfindungsaspekt verwiesen werden, welche in Bezug auf diesen Erfindungsaspekt entsprechend gelten.

Die vorliegende Erfindung wird anhand der folgenden Ausführungsbeispiele veranschaulicht, welche die vorliegende Erfindung jedoch keinesfalls beschränken.

### 1. Bereitstellung von erfindungsgemäßen und nichterfindungsgemäßen Wundauflagen

Um die erfindungsgemäße Wundauflage mit nichterfindungsgemäßen Wundauflagen zu vergleichen, wurden verschiedene Ausführungsformen der erfindungsgemäßen Wundauflagen und Vergleichswundauflagen bereitgestellt:
Die erfindungsgemäßen Wundauflagen wiesen dabei die folgenden Merkmale auf:
   - Die Wundauflagen A und A' wiesen jeweils eine Kollagenschicht sowie eine zwischen zwei textilen Flächenmaterialien auf Basis von Polyamid angeordnete Aktivkohleschicht auf;
   - Die Wundauflagen B und B' wiesen jeweils ebenfalls eine Kollagenschicht sowie eine zwischen zwei textilen Trägermaterialien auf Basis von Polyamid angeordnete Aktivkohleschicht auf und darüber hinaus waren sowohl Kollagenschicht als auch Aktivkohleschicht mit Octenidin ausgerüstet.
   - Die Wundauflage C wies ebenfalls eine Kollagenschicht sowie eine zwischen zwei textilen Trägermaterialien auf Basis von Polyamid angeordnete Aktivkohleschicht auf und darüber hinaus waren sowohl Kollagenschicht als auch Aktivkohleschicht mit Polyhexanid ausgerüstet.
Die weiteren Wundauflagen wiesen dabei die folgenden Eigenschaften auf:
   - Wundauflage D basierte auf einem Standardaktivkohlevlies;
   - Wundauflage E basierte auf einem in Polyhexanid getränkten Polyurethanschaum;
   - Wundauflage F basierte ausschließlich auf Kollagenschaum.
Die Kollagenschicht der Wundauflagen wurde jeweils ausgehend von einer wässrigen Kollagensuspension mit nachfolgender Lyophilisation auf einem geeigneten Träger erzeugt, wobei ein entsprechender Kollagenschaum resultierte. Es wurde Kollagen auf Basis von porciner Haut eingesetzt.
Als Aktivkohle für die Wundauflagen A, B, und C wurde jeweils eine kugelförmige Aktivkohle der Adsor-Tech GmbH, Premnitz /Bundesrepublik Deutschland, eingesetzt, wobei die Aktivkohle durch Carbonisierung und nachfolgende Aktivierung organischer Polymere auf Basis von Polystyrol, insbesondere divinylbezolvernetztem Polystyrol, erhalten wurde (absolute Partikelgröße: ca. 0,2 bis 0,6 mm; Mikroporenanteil: ca. 76 %; BET-Oberfläche: ca. 1.775 m²/g; Gesamtporenvolumen nach Gurvich: ca. 2,2 m²/g; Druck-/Berstfestigkeit: > 15 Newton/Aktivkohlekugel; fraktale Dimension der offenen Porosität: 2,55; Abriebfestigkeit: 100 %).
Als Aktivkohle für die Wundauflagen A' und B' wurde jeweils eine handelsübliche kugelförmige Aktivkohle auf Basis von Phenolharz eingesetzt (absolute Partikelgröße: ca. 0,2 bis 0,6 mm; Mikroporenanteil: ca. 57 %; BET-Oberfläche: ca. 1.375 m²/g; Gesamtporenvolumen nach Gurvich: ca. 1,5 m²/g; Druck-/Berstfestigkeit: < 10 Newton/Aktivkohlekugel; fraktale Dimension der offenen Porosität: 2,15; Abriebfestigkeit: 87 %).

### 2. In-vitro-Untersuchungen der erfindungsgemäßen Wundauflagen

Um die Wirksamkeit der verschiedenen Wundauflagen gegenüber Hospitalkeimen bzw. Krankenhauskeimen, welche insbesondere bei chronischen Wunden ein drastisches Problem darstellen, zu testen, wurden die Wundauflagen im Rahmen eines Hemmhoftests auf ihre antimikrobielle Leistung untersucht.

Die durchgeführte Hemmstoffprüfung wurde im Rahmen eines nach Bauer-Kirby (DIN 58940-3) modifizierten Tests durchgeführt. Im Rahmen des Tests wurde untersucht, in welchem Maße die Wundauflagen imstande sind, das Wachstum der Hospitalkeime *Staphylococcus aureus, Staphylococcus epidermidis, Escherichia coli, Pseudomonas aeruginosa* und *Proteus mirabilis* auf Festmedien zu hemmen.

Dazu wurde nach beendeter Inkubationszeit der Teststämme die Fläche [mm²] des Hemmhofs um die Wundauflage - d. h. der Bereich, in welchem kein Bakterienwachstum erfolgt war - gemessen und als Maß für die antimikrobielle Wirksamkeit der jeweiligen Wundauflage gewertet.

Die Hemmstoffprüfung wurde auf Festmedien auf Basis von Blutagar mit 5 Gew.-% Schafsblut durchgeführt. Vor Animpfen des Festmediums wurden jeweils Verdünnungen der Testkeime derart angesetzt, dass zählbare koloniebildende Einheiten auf den Blutplatten entstanden. Die jeweiligen Verdünnungen der Testkeimsuspension wurden unter sterilen Bedingungen ausplattiert. Im Anschluss wurden die Wundauflagen mit einem Skalpell aseptisch in 1 cm x 1 cm große Stücke geschnitten, unter sterilen Bedingungen auf die Kulturplatten aufgelegt und nach 24 Stunden Auflegezeit wieder entfernt. Es schloss sich eine aerobe Kultivierung bei 37 °C an. Die Fläche der Hemmhöfe um die jeweiligen Wundauflagen wurde nach insgesamt 48 Stunden Inkubationszeit digital gemessen. Anschließend erfolgte die Auswertung durch Vergleich der um die jeweilige Wundauflage entstandenen Hemmhöfe.

Im Rahmen der Hemmstoffprüfung konnten die Hospitalkeime *Staphylococcus aureus, Staphylococcus epidermidis, Escherichia coli, Pseudomonas aeruginosa* und *Proteus mirabilis* mit sämtlichen erfindungsgemäßen Wundauflagen in hervorragender Weise in ihrem Wachstum gehemmt werden. Die stärkste Wachstumshemmung wurde unter Einsatz der erfindungsgemäßen Wundauflage C, welche zudem Polyhexanid als antimikrobiellen Wirkstoff aufwies, erzielt werden. Ebenfalls hervorragende Ergebnisse wurden unter Einsatz der Wundauflagen B und B' erzielt, welche anstelle von Polyhexanid als antimikrobiellen Wirkstoff Octenidin enthielten. Auch mit den erfindungsgemäßen Wundauflagen A und A', welche auf einer Kombination von Kollagenschaum und Aktivkohle basierten, ließ sich das Wachstum der oben aufgeführten Mikroorganismen im Rahmen der Hemmstoffprüfung zufriedenstellend hemmen, allerdings weniger effizient als mit den Wundauflagen B, B' und C.

Mit den Vergleichswundauflagen D, E und F hingegen konnte keine derart effiziente bzw. zufriedenstellende Hemmung des Wachstums der oben genannten Keime beobachtet werden.

In Bezug auf die erfindungsgemäßen Wundauflagen konnte im Rahmen des von der Anmelderin durchgeführten Hemmhoftests insbesondere gezeigt werden, dass der Einsatz spezieller Aktivkohlen zu einer effizienten zusätzlichen Wirkungssteigerung von Wundauflagen auf Basis von Aktivkohle und Kollagenschaum hinsichtlich einer Wachstumshemmung von Hospitalkeimen führt, welche durch den Einsatz eines zusätzlichen antimikrobiellen Wirkstoffs noch weiter gesteigert werden kann.

Die diesbezüglichen Ergebnisse sind im Folgenden detailliert erläutert:
Ein Vergleich der Resultate für die Hemmstoffprüfung der erfindungsgemäßen Wundauflagen A bzw. A' ohne zusätzliches Desinfektionsmittel mit denjenigen der erfindungsgemäßen Wundauflagen B bzw. B' und C, welche jeweils ein zusätzliches Desinfektionsmittel enthielten, zeigen, dass durch die Kombination von Aktivkohle und Kollagen einerseits mit einem weiteren Wirkstoff mit desinfizierender Wirkung andererseits Mikroorganismen in ihrem Wachstum besonders effizient gehemmt werden können. Mit den Wundauflagen A bzw. A' wurde jeweils eine zufriedenstellende Hemmung des mikrobiellen Wachstums erzielt, der Einsatz der Wundauflagen B, B' und C, welche zusätzlich Octenidin bzw. Polyhexanid aufwiesen, führte jedoch insgesamt zu signifikant größeren Hemmhöfen.
Was die Wirkung der Aktivkohle anbelangt, so zeigt der Vergleich der erfindungsgemäßen Wundauflagen A bzw. B mit den erfindungsgemäßen Wundauflagen A' bzw. B', dass insbesondere Aktivkohlen auf Basis von Polystyrol mit einer hohen Mikroporosität, einer großen BET-Oberfläche sowie einer großen fraktalen Dimension der offenen Porosität, wie sie insbesondere von der Adsor-Tech GmbH vertrieben wird, besonders gute antimikrobielle Eigenschaften aufweisen. Denn mit den Wundauflagen A und B ließen sich im Vergleich zur Wundauflage A' bzw. Wundauflage B', welche jeweils eine handelsübliche phenolharzbasierte Aktivkohle mit geringerer Mikroporosität, kleinerer BET-Oberfläche und kleinerem Wert für die fraktale Dimension der offenen Porosität aufwiesen, im Rahmen des Hemmstoffstest signifikant größere Hemmhöfe erzeugen.

Die im Rahmen des Hemmhoftests ermittelten Werte für die jeweiligen Wundauflagen sowie die eingesetzten Keime können darüber hinaus auch Tabelle 1 entnommen werden:

**Tabelle 1: Ergebnisse des Hemmhoftests**

| | Testkeim | Hemmhof [mm²] |
|---|---|---|
| Wundauflage A | *Pseudomonas aeruginosa* | 92 |
| | *Staphylococcus aureus* | 254 |
| | *Staphylococcus epidermidis* | 241 |
| | *Escherichia coli* | 83 |
| | *Proteus mirabilis* | 56 |
| Wundauflage A' | *Pseudomonas aeruginosa* | 85 |
| | *Staphylococcus aureus* | 205 |
| | *Staphylococcus epidermidis* | 199 |
| | *Escherichia coli* | 87 |
| | *Proteus mirabilis* | 41 |
| Wundauflage B | *Pseudomonas aeruginosa* | 124 |
| | *Staphylococcus aureus* | 371 |
| | *Staphylococcus epidermidis* | 340 |
| | *Escherichia coli* | 243 |
| | *Proteus mirabilis* | 112 |
| Wundauflage B' | *Pseudomonas aeruginosa* | 104 |
| | *Staphylococcus aureus* | 339 |
| | *Staphylococcus epidermidis* | 327 |
| | *Escherichia coli* | 238 |
| | *Proteus mirabilis* | 95 |
| Wundauflage C | *Pseudomonas aeruginosa* | 137 |
| | *Staphylococcus aureus* | 397 |
| | *Staphylococcus epidermidis* | 373 |
| | *Escherichia coli* | 289 |
| | *Proteus mirabilis* | 119 |
| Wundauflage D | *Pseudomonas aeruginosa* | 79 |
| | *Staphylococcus aureus* | 152 |
| | *Staphylococcus epidermidis* | 176 |
| | *Escherichia coli* | 76 |
| | *Proteus mirabilis* | 19 |
| Wundauflage E | *Pseudomonas aeruginosa* | 82 |
| | *Staphylococcus aureus* | 191 |
| | *Staphylococcus epidermidis* | 182 |
| | *Escherichia coli* | 81 |
| | *Proteus mirabilis* | 34 |
| Wundauflage F | *Pseudomonas aeruginosa* | 69 |
| | *Staphylococcus aureus* | 112 |
| | *Staphylococcus epidermidis* | 107 |
| | *Escherichia coli* | 56 |
| | *Proteus mirabilis* | 0 |

Insgesamt geht aus den vorstehenden Ergebnissen hervor, dass sich mit einer Kombination von Kollagen und Aktivkohle das Wachstum von Mikroorganismen im Vergleich zu Wundauflagen des Standes der Technik deutlich stärker hemmen lässt. Darüber hinaus wird deutlich, dass sich dieser Effekt zusätzlich durch a) den Einsatz eines weiteren desinfizierenden Wirkstoffs sowie b) den Einsatz spezieller Aktivkohlen steigern lässt.

Der besondere Vorteil der erfindungsgemäßen Wundauflagen ist insbesondere auch darin zu sehen, dass durch deren Einsatz auch solche Mikroorganismen im Wachstum gehemmt werden können, welche für das vermehrte Auftreten von Antibiotikaresistenzen bekannt sind, insbesondere die sogenannten Hospitalkeime.

### 3. Anwendungs- und Wirksamkeitsstudien

Um die Wirksamkeit der erfindungsgemäßen Wundauflagen mit den nichterfindungsgemäßen Wundauflagen zu vergleichen, wurden jeweils Probanden im Alter von 70 bis 85, welche an chronischen bzw. nekrotisierenden Wunden litten, über einen Zeitraum von vier Wochen behandelt. Dazu wurde die jeweilige Wundauflage auf die betroffene Partie des Körpers appliziert. Innerhalb der ersten Woche des Behandlungszeitraumes wurde die Wundauflage morgens und abends gewechselt; ab der zweiten Woche erfolgte ein Wechseln der Wundauflagen in Abhängigkeit vom Zustand der jeweiligen Wunde. Je weiter die Wundheilung bereits vorangeschritten war, desto länger war der Zeitraum zwischen den Verbandswechseln, wobei in allen Fällen spätestens nach zwei Tagen ein Verbandswechsel durchgeführt wurde.

Im Rahmen der durchgeführten Studien wurde in Bezug auf die Wundauflagen A und B jeweils eine Probandengruppe von 15 Personen untersucht. 15 Personen, von denen 11 weiblich und 4 männlich waren, erhielten die Wundauflage A, welche auf einer Kombination von Aktivkohle und Kollagen basierte. Weitere 15 Personen, von denen 8 weiblich und 7 männlich waren, erhielten zur Verbesserung der Wundheilung ihrer chronischen Wunden Wundauflage B, welche sowohl in der Kollagenschicht als auch in der Aktivkohleschicht mit dem antimikrobiellen Wirkstoff Octenidin ausgerüstet war.

Nach vierwöchiger Therapie bzw. Behandlung der chronischen Wunden war bei allen Probanden eine deutliche Verbesserung zu erkennen. Die Wundsekretion sowie Entzündungssymptome waren vollkommen abgeklungen und die Wundumgebung war bei allen Probanden nach der Behandlung intakt. Insgesamt waren nach der vierwöchigen Therapie die Wunden bei 9 Probanden der ersten Gruppe (Wundauflage A) sowie nach dreiwöchiger Therapie bei 13 Probanden der zweiten Gruppe (Wundauflage B) vollkommen geschlossen und weitgehend epithelisiert. Bei den übrigen Probanden waren die Wunden teilweise noch nicht vollständig geschlossen, allerdings erschien der Wundgrund rosig und granulierend und die Wundumgebung war intakt, was auf eine baldige Verheilung der Wunde schließen lässt. Anhand der Beurteilung des Zustandes der Wunden nach drei bzw. vier Wochen konnte insgesamt ein zufriedenstellendes Ergebnis hinsichtlich des Heilungsfortschrittes bei den chronischen Wunden verzeichnet werden. Was die Wirksamkeit der Wundauflagen A und B anbelangt, so beschleunigt die zusätzliche Ausrüstung der Wundauflage mit einem antimikrobiellen Wirkstoff, wie Octenidin, die Wundheilung. Insbesondere konnte bei den mit der Wundauflage B behandelten Probanden ein schnellerer Rückgang von Entzündungssymptomen, insbesondere bei Vorliegen von Infektionen, beobachtet werden.

Hinsichtlich der Wundauflagen D, E und F wurde ebenfalls eine Probandengruppe untersucht, wobei der Behandlungszeitrum jeweils vier Wochen betrug. Von der Probandengruppe erhielten 15 Personen, von denen 8 weiblich und 7 männlich waren, Wundauflage D. Weitere 15 Probanden, von denen 6 weiblich und 9 männlich waren, wurden mit der Wundauflage E behandelt. Wiederum weitere 15 Probanden, von denen 9 weiblich und 6 männlich waren, wurden mit der Wundauflage F behandelt.

Bei den mit der Wundauflage D behandelten Probanden wurde im Behandlungszeitraum eine Verbesserung der Wundheilung beobachtet, wobei jedoch nur bei 5 der insgesamt 15 Probanden ein Verschluss bzw. eine vollständige Epithelisierung der Wunde beobachtet werden konnte; bei den übrigen 10 Probanden waren die Wunden noch nicht vollständig verschlossen, besaßen aber zumindest einen granulierenden Wundgrund. Was die Geruchsadsorption der Wundauflage D anbelangte, so war diese jedoch weitgehend zufriedenstellend. Darüber hinaus waren bei 8 der insgesamt 15 Probanden leichte bis mittlere Entzündungsreaktionen bzw. Wundinfektionen aufgetreten, welche eine zusätzliche Therapie mit antimikrobiellen Substanzen erforderlich machten. Gegenüber den Wundauflagen A und B lässt sich somit mit der Wundauflage D insgesamt kein optimaler Schutz vor Kontaminationen mit pathogenen Keimen erzielen. Insbesondere führt der geringere Kontaminationsschutz auch zu einer verzögerten Wundheilung.

Mit der Wundauflage E konnten ebenfalls keine mit den Wundauflagen A und B vergleichbaren Ergebnisse erzielt werden. Nur bei 2 der insgesamt 15 Probanden, die mit Wundauflage E behandelt wurden, war die Wunde vollständig geschlossen bzw. epithelisiert. Bei insgesamt 8 der 15 Probanden erschien der Wundgrund rosig und granulierend, was auf ein Fortschreiten des Heilungsprozesses hindeutet. Bei 2 Probanden war der Wundgrund immer noch mit Fibrin belegt, was ein Charakteristikum der frühen Phasen des Heilungsprozesses darstellt. Darüber hinaus hatte sich bei 2 der 15 Probanden die Wunde infiziert, so dass mitunter starke Entzündungssymptome auftraten. Wundauflagen auf Basis von Polyurethanschaum, welcher mit einem Desinfektionsmittel getränkt wurde, bieten insgesamt weder einen optimalen Kontaminationsschutz noch eine zufriedenstellende Geruchsadsorption.

Mit der Wundauflage F ausschließlich auf Basis von Kollagen wurden die schlechtesten Ergebnisse erhalten. Nur bei einem der Probanden war die Wunde vollständig geschlossen bzw. epithelisiert; bei nur 4 Probanden erschien der Wundgrund rosig und granulierend, und bei 10 Probanden war der Wundgrund immer noch mit Fibrin belegt. Darüber hinaus bemängelten die Probanden die unzureichende Geruchsadsorption. Wundauflagen ausschließlich auf Basis von Kollagen führen im Ergebnis somit weder zu einer beschleunigten Wundheilung noch zu einer hinreichenden Geruchsadsorption.

Die getesteten Wundauflagen wurden nach einem Schulnotensystem, d. h. mit Bewertungen variierend im Bereich von 1 = sehr gut bis 6 = ungenügend, im Hinblick auf die Beschleunigung der Wundheilung, den Kontaminationsschutz und die Eindämmung von Infektions- und Entzündungssymptomen sowie die Geruchsadsorption bewertet. Die diesbezüglichen Ergebnisse für die getesteten Wundauflagen sind nachfolgend Tabelle 2 zu entnehmen:

**Tabelle 2: Bewertung der Wundauflagen mit dem Schulnotensystem**

| | Beschleunigung Wundheilung | Kontaminationsschutz / Eindämmung von Infektions- und Entzündungssymptomen | Geruchsadsorption |
|---|---|---|---|
| Wundauflage A | 1,9 ± 0,2 | 2,2 ± 0,1 | 1,6 ± 0,3 |
| Wundauflage B | 1,8 ± 0,1 | 1,7 ± 0,2 | 1,7 ± 0,4 |
| Wundauflage D | 3,5 ± 0,3 | 3,1 ± 0,2 | 2,0 ± 0,3 |
| Wundauflage E | 3,2 ± 0,5 | 2,7 ± 0,1 | 3,5 ± 0,2 |
| Wundauflage F | 4,3 ± 0,3 | 3,8 ± 0,2 | 3,4 ± 0,2 |

Die durchgeführten Anwendungs- und Wirksamkeitsbeobachtungen zeigen die hervorragende Wirksamkeit der Wundauflagen A und B bei der Behandlung von chronischen Wunden, insbesondere im Zusammenhang mit Druckgeschwüren sowie Wunden, welche im Rahmen von mit Durchblutungsstörungen einhergehenden Grunderkrankungen verbunden sind.

Aus den Ergebnissen geht deutlich hervor, dass durch den kombinierten Einsatz von Aktivkohle einerseits sowie Kollagen andererseits die Wundheilung signifikant beschleunigt werden kann und darüber hinaus Entzündungssymptome und Infektionen gelindert werden. Weiterhin ist es möglich, durch den Einsatz der aktivkohlehaltigen Wundauflagen unangenehme Gerüche, wie sie häufig im Zusammenhang mit chronischen Wunden entstehen, zu adsorbieren.

Die besten Ergebnisse wurden insgesamt mit den Wundauflagen A und B erzielt, wobei die Wundauflage B, welche neben Aktivkohle und Kollagen mit der bakteriziden Komponente Octenidin ausgerüstet war, die besten Ergebnisse lieferte. Durch die zusätzliche Ausrüstung mit einem desinfizierenden bzw. bakteriziden Wirkstoff konnten Entzündungssymptome noch besser eingedämmt werden bzw. es bestand ein verbesserter Kontaminationsschutz, so dass insgesamt auch der Heilungsprozess noch stärker beschleunigt werden konnte.

Tabelle 3 zeigt einen Vergleich der grundlegenden Eigenschaften der erfindungsgemäßen Wundauflagen einerseits mit denjenigen der Wundauflagen des Standes der Technik andererseits, wie unter Ziffer 1.) beschrieben.

**Tabelle 3: Eigenschaften erfindungsgemäßer und nichterfindungsgemäßer Wundauflagen**

| **Parameter** | **Wundauflage** | | | |
|---|---|---|---|---|
| | Erfindungsgemäße Wundauflagen (A, A', B, B' und C) | Wundauflage mit einer handelsüblichen Aktivkohle (D) | in Polyhexanid getränkter Polyurethanschaum (E) | Kollagenschaum (F) |
| Beschreibung der Wundauflage | oben beschriebene erfindungsgemäße Wundauflagen A bis C | Textiler Träger auf Basis eines Viskose/Polyamid-Gemischs mit dazwischen liegender Aktivkohleschicht | Schaumverband aus Polyurethan, wobei das Polyurethan mit Polyhexanid imprägniert wurde | Wundauflage aus einem resorbierbaren Kollagenschaum |
| Antimikrobielle bzw. biozide Eigenschaften | sehr hoch | nicht vorhanden | nachweisbar, jedoch nicht für alle Testkeime | äußerst gering, kaum nachweisbar |
| Adsorption von Keimen und Bestandteilen von Keimen aus der Wunde | stark | schwach | schwach | schwach |
| Förderung der Wundheilung | sehr stark | schwach | schwach | stark |
| Initiierung der Wundheilung bei stagnierenden Wunden | stark | nicht vorhanden | nicht vorhanden | schwach |
| Räumliche Anpassung an den Wundgrund | sehr stark | schwach | mittel | stark |
| Mazerationsschutz | stark | schwach | mittel | mittel |
| Exsudatmanagement | stark | schwach | schwach | mittel |
| Gasdurchlässigkeit | sehr stark | stark | schwach | stark |
| Geruchs- und Toxinadsorption | Stark | Mittel | Schwach | Schwach |
| Barrierewirkung vor äußeren Einflüssen | sehr stark | mittel | stark | mittel |
| Kühlender Effekt | sehr stark | schwach | schwach | stark |
| Verbandswechsel | schmerzfrei | schmerzhaft | schmerzhaft | weitgehend schmerzfrei |
| Toxikologische Risiken | gering, da die erforderliche Polyhexanidmenge reduziert ist | gering | erhöht aufgrund relativ großer Mengen an Polyhexanid | gering |

### 4. Fazit

Insgesamt geht aus den Ausführungsbeispielen deutlich hervor, dass die erfindungsgemäßen Wundauflagen in vielfacher Hinsicht den gegenüber nichterfindungsgemäßen Wundauflagen - insbesondere durch die biostatische bzw. biozide Ausrüstung bzw. Eigenschaften und/oder durch die Kombination von Aktivkohle einerseits und Kollagen andererseits - verbessert sind. So wird bei Einsatz der erfindungsgemäßen Wundauflagen zur therapeutischen Wundversorgung der Heilungsprozess der Wunde signifikant beschleunigt. Darüber hinaus besteht ein ausgezeichneter Kontaminationsschutz, insbesondere vor Krankenhaus- bzw. Hospitalkeimen, welche gegenüber Antibiotika häufig resistent sind und deren Auftreten im Wundgebiet besondere Anforderungen an die Therapie stellt. Weiterhin besitzen die erfindungsgemäßen Wundauflagen gute Geruchsadsorptionseigenschaften, was vor allem dem Wohlbefinden der Patienten zugute kommt.

## Patentansprüche

1. Wundauflage (1), insbesondere zur therapeutischen Wundversorgung, wobei die Wundauflage (1) einen mehrschichtigen Aufbau aufweist, wobei der mehrschichtige Aufbau mindestens eine mindestens ein Hydrokolloid, vorzugsweise Kollagen, enthaltende Schicht (2) ("Hydrokolloidschicht" oder "Kollagenschicht") und mindestens eine Aktivkohle enthaltende Schicht (3) ("Aktivkohleschicht") umfasst,
wobei die Aktivkohle enthaltende Schicht (3) eine kugelförmige Aktivkohle umfasst und wobei die Aktivkohle der Aktivkohle enthaltenden Schicht (3) einen durch Mikroporen mit Porendurchmessern von ≤ 200 nm (20 Å) gebildeten Mikroporenvolumenanteil, bezogen auf das Gesamtporenvolumen der Aktivkohle, von mindestens 60 % aufweist.

2. Wundauflage nach Anspruch 1,
wobei das Hydrokolloid der Hydrokolloid enthaltenden Schicht (2) ausgewählt ist aus der Gruppe von Polysacchariden und Proteinen, insbesondere pflanzlichen, tierischen oder bakteriellen Polysacchariden und Proteinen, vorzugsweise aus der Gruppe von Kollagen, Cellulose und Cellulosederivaten, Glykosaminoglykanen, Pektinen, Gummi arabicum, Galactomannanen, Agar, Carrageen, Alginaten, Gelatine, Caseinaten, Xanthanen, Dextranen und Skleroglucanen, und besonders bevorzugt Kollagen, Hyaluronsäure und/oder deren Salze und/oder Gelatine, ganz besonders bevorzugt Kollagen, ist;
und/oder
wobei die Hydrokolloid, vorzugsweise Kollagen, enthaltende Schicht (2) auf Hydrokolloidvlies und/oder Hydrokolloidschaum, vorzugsweise Kollagenvlies und/oder Kollagenschaum, basiert und insbesondere auf Basis von Hydrokolloidvlies und/oder Hydrokolloidschaum, vorzugsweise Kollagenvlies und/oder Kollagenschaum, porcinen, bovinen und/oder equinen Ursprungs, besonders bevorzugt auf Basis von Hydrokolloidvlies und/oder Hydrokolloidschaum, vorzugsweise Kollagenvlies und/oder Kollagenschaum, porcinen Ursprungs, ausgebildet ist und/oder wobei die Hydrokolloid, vorzugsweise Kollagen, enthaltende Schicht (2) durch ein Hydrokolloidvlies und/oder Hydrokolloidschaum, vorzugsweise Kollagenvlies und/oder einen Kollagenschaum, insbesondere durch ein Hydrokolloidvlies und/oder Hydrokolloidschaum, vorzugsweise Kollagenvlies und/oder einen Kollagenschaum, porcinen, bovinen und/oder equinen Ursprungs, bevorzugt durch ein Hydrokolloidvlies und/oder Hydrokolloidschaum, vorzugsweise Kollagenvlies und/oder einen Kollagenschaum, porcinen Ursprungs, gebildet ist.

3. Wundauflage nach Anspruch 1 oder 2,
wobei die Hydrokolloid, vorzugsweise Kollagen, enthaltende Schicht (2) erhältlich ist durch Aufbringen einer Dispersion oder Lösung eines Hydrokolloids, vorzugsweise Kollagens, auf einem Träger und nachfolgende Trocknung, insbesondere Lyophilisation (Gefriertrocknung), vorzugsweise unter Expansion des Hydrokolloids, vorzugsweise Kollagens; und/oder
wobei das Hydrokolloid, vorzugsweise Kollagen, der Hydrokolloid, vorzugsweise Kollagen, enthaltenden Schicht (2) porcinen, bovinen und/oder equinen Ursprungs, bevorzugt porcinen Ursprungs, insbesondere aus porciner Haut, ist; und/oder
wobei die Hydrokolloid, vorzugsweise Kollagen, enthaltende Schicht (2) eine äußere Schicht der Wundauflage (1) bildet und/oder wobei die Hydrokolloid, vorzugsweise Kollagen, enthaltende Schicht (2) im Anwendungszustand der Wundauflage (1) auf der der zu behandelnden Wunde zugewandten Seite der Wundauflage angeordnet ist; und/oder
wobei die Hydrokolloid, vorzugsweise Kollagen, enthaltende Schicht (2) eine Dicke im Bereich von 0,01 bis 100 mm, insbesondere 0,02 bis 50 mm, vorzugsweise 0,05 bis 10 mm, aufweist und/oder wobei die Hydrokolloid, vorzugsweise Kollagen, enthaltende Schicht (2) 5 % bis 95 %, insbesondere 10 % bis 80 %, vorzugsweise 20 % bis 60 %, der Gesamtdicke der Wundauflage ausmacht.

4. Wundauflage nach einem der vorangehenden Ansprüche,
wobei die Aktivkohle enthaltende Schicht (3) eine kugelförmige Aktivkohle mit absoluten Teilchengrößen im Bereich von 0,01 bis 3 mm, insbesondere im Bereich von 0,02 bis 2 mm, bevorzugt im Bereich von 0,05 bis 1,5 mm, besonders bevorzugt im Bereich von 0,1 bis 0,8 mm, ganz besonders bevorzugt im Bereich von 0,2 bis 0,6 mm, umfasst und/oder wobei die Aktivkohle enthaltende Schicht (3) eine kugelförmige Aktivkohle mit mittleren Teilchengrößen, insbesondere bestimmt nach ASTM D2862-97/04, im Bereich von 0,05 bis 2,5 mm, insbesondere im Bereich von 0,1 bis 2 mm, bevorzugt im Bereich von 0,15 bis 1 mm, besonders bevorzugt im Bereich von 0,2 bis 0,6 mm, umfasst; und/oder
wobei die Aktivkohle der Aktivkohle enthaltenden Schicht (3) einen durch Mikroporen mit Porendurchmessern von ≤ 200 nm (20 Å) gebildeten Mikroporenvolumenanteil, bezogen auf das Gesamtporenvolumen der Aktivkohle, von mindestens 65 %, bevorzugt mindestens 70 %, aufweist und/oder wobei die Aktivkohle der Aktivkohle enthaltenden Schicht (3) einen durch Mikroporen mit Porendurchmessern von ≤ 200 nm (20 Å) gebildeten Mikroporenvolumenanteil, bezogen auf das Gesamtporenvolumen der Aktivkohle, im Bereich von 60 % bis 95 %, insbesondere im Bereich von 65 % bis 90 %, bevorzugt im Bereich von 70 % bis 85 %, aufweist; und/oder
wobei die Aktivkohle der Aktivkohle enthaltenden Schicht (3) ein durch Mikroporen mit Porendurchmessern von ≤ 200 nm (20 Å) gebildetes Mikroporenvolumen, insbesondere ein Mikroporenvolumen nach Carbon Black, von mindestens 0,40 cm³/g, insbesondere mindestens 0,45 cm³/g, vorzugsweise mindestens 0,50 cm³/g, aufweist und/oder wobei die Aktivkohle der Aktivkohle enthaltenden Schicht (3) ein durch Mikroporen mit Porendurchmessern von ≤ 200 nm (20 Å) gebildetes Mikroporenvolumen, insbesondere ein Mikroporenvolumen nach Carbon Black, im Bereich von 0,40 cm³/g bis 2 cm³/g, insbesondere im Bereich von 0,45 cm³/g bis 1,5 cm³/g, vorzugsweise im Bereich von 0,50 cm³/g bis 1,2 cm³/g, aufweist.

5. Wundauflage nach einem der vorangehenden Ansprüche,
wobei die Aktivkohle der Aktivkohle enthaltenden Schicht (3) einen spezifischen Mikroporenoberflächenanteil, insbesondere einen aus Poren mit Porendurchmessern von ≤ 200 nm (20 Å) gebildeten Mikroporenoberflächenanteil, von mindestens 50 %, insbesondere mindestens 60 %, bevorzugt mindestens 70 %, ganz besonders bevorzugt mindestens 75 %, bezogen auf die spezifische Gesamtoberfläche (BET) der Aktivkohle, aufweist; und/oder
wobei die Aktivkohle der Aktivkohle enthaltenden Schicht (3) eine innere Oberfläche (BET) im Bereich von 500 bis 3.000 m²/g, insbesondere im Bereich von 800 bis 2.000 m²/g, bevorzugt im Bereich von 900 bis 1.800 m²/g, besonders bevorzugt im Bereich von 1.000 bis 1.600 m²/g, aufweist; und/oder
wobei die Aktivkohle der Aktivkohle enthaltenden Schicht (3) ein Gesamtporenvolumen, insbesondere ein Gesamtporenvolumen nach Gurvich, im Bereich von 0,1 bis 4 cm³/g, insbesondere im Bereich von 0,2 bis 3 cm³/g, vorzugsweise im Bereich von 0,3 bis 2,5 cm³/g, besonders bevorzugt im Bereich von 0,5 bis 2 cm³/g, aufweist.

6. Wundauflage nach einem der vorangehenden Ansprüche,
wobei die Aktivkohle der Aktivkohle enthaltenden Schicht (3) eine Druck- und/oder Berstfestigkeit, insbesondere eine Gewichtsbelastbarkeit pro Aktivkohleteilchen, insbesondere pro Aktivkohlekorn oder Aktivkohlekugel, von mindestens 10 Newton, insbesondere mindestens 15 Newton, vorzugsweise mindestens 20 Newton, aufweist und/oder wobei die Aktivkohle der Aktivkohle enthaltenden Schicht (3) eine Druck- und/oder Berstfestigkeit, insbesondere eine Gewichtsbelastbarkeit, pro Aktivkohleteilchen, insbesondere pro Aktivkohlekorn oder Aktivkohlekugel, im Bereich von 10 bis 50 Newton, insbesondere im Bereich von 12 bis 45 Newton, vorzugsweise im Bereich von 15 bis 40 Newton, aufweist; und/oder
wobei die Aktivkohle der Aktivkohle enthaltenden Schicht (3) eine fraktale Dimension der offenen Porosität von mindestens 2,3, insbesondere von mindestens 2,4, vorzugsweise von mindestens 2,5, besonders bevorzugt von mindestens 2,7, aufweist und/oder wobei die Aktivkohle der Aktivkohle enthaltenden Schicht (3) zumindest im Wesentlichen abriebfest und/oder zumindest im Wesentlichen staubfrei ausgebildet ist.

7. Wundauflage nach einem der vorangehenden Ansprüche,
wobei die Aktivkohle der Aktivkohle enthaltenden Schicht (3) eine biozide und/oder biostatische, insbesondere antimikrobielle Wirkung und/oder Ausrüstung aufweist,
insbesondere wobei die biozide und/oder biostatische, insbesondere antimikrobielle Wirkung und/oder Ausrüstung der Aktivkohle durch den Herstellungsprozess der Aktivkohle, insbesondere die Herstellung mittels Pyrolyse und nachfolgende Aktivierung organischer Polymere, erreicht wird, insbesondere infolge der hierdurch generierten Oberflächenladung und/oder Hydrophobie und/oder texturellen Eigenschaften, und/oder
insbesondere wobei die biozide und/oder biostatische, insbesondere antimikrobielle Wirkung und/oder Ausrüstung der Aktivkohle durch eine Ausrüstung, insbesondere Imprägnierung, der Aktivkohle mit mindestens einem bioziden und/oder biostatischen, insbesondere antimikrobiellen Wirkstoff erfolgt.

8. Wundauflage nach einem der vorangehenden Ansprüche,
wobei die Aktivkohle der Aktivkohle enthaltenden Schicht (3) in einer Menge, insbesondere Auflagemenge, von 1 bis 1.000 g/m², insbesondere 5 bis 500 g/m², vorzugsweise 10 bis 400 g/m², bevorzugt 20 bis 300 g/m², besonders bevorzugt 25 bis 250 g/m², vorhanden ist; und/oder
wobei die Aktivkohle der Aktivkohle enthaltenden Schicht (3) auf einem flächigen, vorzugsweise textilen Träger (4a, 4b) angeordnet ist, vorzugsweise hieran befestigt ist, und/oder wobei die Aktivkohle der Aktivkohle enthaltenden Schicht (3) auf einem dreidimensionalen, vorzugsweise porösen und/oder textilen Träger (4a, 4b), bevorzugt einem Schaum oder Schaumstoff, angeordnet ist, vorzugsweise hieran befestigt und/oder hierin eingelagert ist, insbesondere wobei der dreidimensionale Träger (4a, 4b) auf Basis eines Elastomerharzes und/oder auf Basis eines Polyurethans ausgebildet ist.

9. Wundauflage nach einem der vorangehenden Ansprüche,
wobei die Aktivkohle der Aktivkohle enthaltenden Schicht (3) zwischen einem ersten textilen Flächenmaterial (4a) und einem zweiten textilen Flächenmaterial (4b) angeordnet ist;
insbesondere wobei das erste textile Flächenmaterial (4a) und/oder das zweite textile Flächenmaterial (4b) als Gewebe, Gewirke, Gestricke, Gelege, Textilverbundstoff, Vlies oder Filamentfasermaterial, insbesondere als Filamentfasermaterial, ausgebildet ist; und/oder
wobei das erste textile Flächenmaterial (4a) und/oder das zweite textile Flächenmaterial (4b) auf einer Faserart, ausgewählt aus der Gruppe von Polyestern (PES); Polyolefinen, insbesondere Polyethylen (PE) und/oder Polypropylen (PP), Polyvinylchlorid (PVC); Polyvinylidenchlorid (PVDC); Celluloseacetat (CA); Cellulosetriacetat (CTA); Polyacryl (PAN); Polyamid (PA); Polyvinylalkohol (PVAL); Polyurethanen (PU); Polyvinylestern; (Meth-)Acrylaten; sowie deren Mischungen, insbesondere Celluloseacetat und/oder Polyamid, basiert; und/oder
wobei das erste textile Flächenmaterial (4a) und/oder das zweite textile Flächenmaterial (4b), derart ausgebildet ist, dass es zumindest im Wesentlichen keine Fasern und/oder zumindest im Wesentlichen keine Aktivkohle freisetzen kann; und/oder insbesondere wobei die Aktivkohle der Aktivkohle enthaltenden Schicht (3) an dem ersten textilen Flächenmaterial (4a) und/oder an dem zweiten textilen Flächenmaterial (4b) befestigt ist, insbesondere mittels eines vorzugsweise medizinisch und/oder physiologisch kompatiblen Klebstoffs, insbesondere wobei der Klebstoff diskontinuierlich und/oder punktförmig auf dem ersten und/oder zweiten textilen Flächenmaterial (4a, 4b) aufgetragen ist und/oder insbesondere wobei der Klebstoff auf dem ersten und/oder zweiten textilen Flächenmaterial (4a, 4b) in einer Auflagemenge von 1 bis 100 g/m², insbesondere 5 bis 50 g/m², vorzugsweise 10 bis 40 g/m², aufgetragen ist und/oder insbesondere wobei der Klebstoff das erste und/oder zweite textile Flächenmaterial (4a, 4b) jeweils zu höchstens 70 %, insbesondere zu höchstens 60 %, vorzugsweise zu höchstens 50 %, bevorzugt zu höchstens 40 %, besonders bevorzugt zu höchstens 30 %, bedeckt und/oder insbesondere wobei die Oberfläche der Aktivkohle zu mindestens 50 %, insbesondere zu mindestens 60 %, vorzugsweise zu mindestens 70 %, nicht mit Klebstoff bedeckt ist und/oder frei zugänglich ist.

10. Wundauflage nach einem der vorangehenden Ansprüche,
wobei die Aktivkohle der Aktivkohle enthaltenden Schicht (3) als selbsttragende Schicht, insbesondere als Aktivkohlefaserflächengebilde oder als selbsttragende, flächige oder dreidimensionale, vorzugsweise zusammenhängende Struktur von miteinander verbundenen und/oder aneinander befestigten kornförmigen, insbesondere kugelförmigen Aktivkohlepartikeln, vorliegt; und/oder
wobei die Aktivkohle der Aktivkohle enthaltenden Schicht (3) in die Hydrokolloid, vorzugsweise Kollagen, enthaltende Schicht (2) eingelagert und/oder an die Hydrokolloid, vorzugsweise Kollagen, enthaltende Schicht (2) angelagert und/oder fixiert ist.

11. Wundauflage nach einem der vorangehenden Ansprüche,
wobei die Oberfläche der Aktivkohle der Aktivkohle enthaltenden Schicht (3) zu mindestens 50 %, insbesondere zu mindestens 60 %, vorzugsweise zu mindestens 70 %, frei zugänglich ist und/oder nicht bedeckt ist; und/oder
wobei die einzelnen Schichten (2, 3, 4a, 4b) der Wundauflage (1) jeweils miteinander verbunden sind und/oder wobei die einzelnen Schichten (2, 3, 4a, 4b) der Wundauflage (1) einen Verbund ausbilden.

12. Wundauflage nach einem der vorangehenden Ansprüche,
wobei die Wundauflage (1) weiterhin mindestens einen Wirkstoff enthält, insbesondere ausgewählt aus der Gruppe von antimikrobiell wirkenden Wirkstoffen, desinfizierenden Wirkstoffen, entzündungshemmenden Wirkstoffen, blutstillenden Wirkstoffen und wundheilungsfördernden Wirkstoffen; und/oder
wobei die Wundauflage (1) weiterhin mit mindestens einem antimikrobiellen und/oder desinfizierenden und/oder entzündungshemmenden und/oder blutstillenden und/oder wundheilungsfördernden Wirkstoff ausgerüstet ist und/oder wobei die Wundauflage (1) mindestens einen antimikrobiellen und/oder desinfizierenden und/oder entzündungshemmenden und/oder blutstillenden und/oder wundheilungsfördernden Wirkstoff aufweist.

13. Wundauflage nach Anspruch 12,
wobei der Wirkstoff biozide und/oder biostatische Wirkung, insbesondere bakterizide oder bakteriostatische und/oder fungizide oder fungistatische und/oder viruzide oder virustatische Wirkung, hat; und/oder
wobei der Wirkstoff ein Antiseptikum und/oder Desinfektionsmittel ist und/oder wobei der Wirkstoff ausgewählt ist aus der Gruppe von Polyhexamethylenbiguanid (Polyhexanid), Taurolidin, Benzalkoniumchlorid, Chlorhexidin, Octenidin und deren physiologisch verträglichen Salzen sowie deren Mischungen, vorzugsweise aus Octenidin und/oder Polyhexamethylenbiguanid (Polyhexanid).

14. Wundauflage nach Anspruch 12 oder 13,
wobei der Wirkstoff ein Wirkstoff mit wundheilungsfördernder Wirkung ist, insbesondere ausgewählt aus der Gruppe von Alginaten, Chitosan, Hyaluronsäure und deren Salzen, Allantoin, beta-Sitosterol, Bromelain, Dexpanthenol, Pantothensäure, Harnstoff, Flavonoiden, Riboflavin, Saponinen, Cineol, Tocopherol und deren Mischungen; und/oder
wobei der Wirkstoff in einer Menge, insbesondere Auflagemenge, von 0,00001 bis 5 g/cm², insbesondere 0,0001 bis 2 g/cm², vorzugsweise 0,001 bis 1 g/cm², besonders bevorzugt 0,01 bis 0,5 g/cm², in der Wundauflage (1) vorhanden ist; und/oder
wobei der Wirkstoff in der Hydrokolloid enthaltenden Schicht (2) und/oder in der Aktivkohle enthaltenden Schicht (3) vorliegt, vorzugsweise der Wirkstoff in der Hydrokolloid enthaltenden Schicht (2) und in der Aktivkohle enthaltenden Schicht (3) vorliegt.

15. Wundauflage nach einem der vorangehenden Ansprüche zur Verwendung bei der therapeutischen, insbesondere topischen Wundversorgung, insbesondere zur Verwendung bei der therapeutischen, vorzugsweise topischen Behandlung von Wunden und/oder Gewebsunterbrechungen;
insbesondere zur Verwendung bei der therapeutischen Behandlung von mechanischen Wunden, insbesondere Schnitt-, Stich-, Quetsch-, Platz-, Ritz- und/oder Schürfwunden; oder
insbesondere zur Verwendung bei der therapeutischen Behandlung von thermischen Wunden, insbesondere durch Kälte- oder Hitzeverbrennung ausgelöste Wunden; oder insbesondere zur Verwendung bei der therapeutischen Behandlung von chemischen Wunden, insbesondere durch Verätzung mit Laugen und/oder Säuren ausgelöste Wunden; oder
insbesondere zur Verwendung bei der therapeutischen Behandlung von strahlenbedingten Wunden, insbesondere durch aktinische und/oder ionisierende Strahlung ausgelöste Wunden; oder
insbesondere zur Verwendung bei der therapeutischen Behandlung von nekrotisierenden und/oder infizierten und/oder chronischen Wunden oder aber von akuten Wunden; oder
insbesondere zur Verwendung bei der therapeutischen Behandlung von Druckgeschwüren und/oder durch Durchblutungsstörungen ausgelösten Wunden.

## Claims

1. A wound dressing (1), in particular for therapeutic wound care, wherein the wound dressing (1) has a multilayered structure, wherein the multilayered structure at least comprises at least one layer (2) including a hydrocolloid, preferably collagen ("hydrocolloid layer" or "collagen layer") and at least one layer (3) containing active charcoal ("active charcoal layer"),
wherein the active charcoal layer (3) comprises spherical active charcoal, and wherein the active charcoal of the active charcoal layer (3) has a micropore volume proportion of at least 60 % based on the total pore volume of the active charcoal, formed by micropores having pore diameters ≤ 200 nm (20 Ǻ).

2. The wound dressing according to claim 1,
wherein the hydrocolloid of the hydrocolloid layer (2) is selected from the group of polysaccharides and proteins, in particular vegetable, animal, or bacterial polysaccharides and proteins, preferably from the group of collagen, cellulose and cellulose derivatives, glycosaminoglycans, pectins, gum arabic, galactomannans, agar-agar, carrageenan, alginates, gelatins, caseinates, xanthans, dextrans, and scleroglucans, and more preferably collagen, hyaluronic acid, and/or salts thereof, and/or gelatin, most preferably collagen; and/or
wherein the hydrocolloid - preferably collagen - layer (2) is formed on hydrocolloid fleece and/or hydrocolloid foam, preferably collagen fleece, and in particular on hydrocolloid fleece and/or hydrocolloid foam, preferably collagen fleece and/or collagen foam of porcine, bovine and/or equine origin, and wherein the hydrocolloid - preferably collagen - layer is formed with a hydrocolloid fleece and/or hydrocolloid foam, preferably collagen fleece and/or collagen foam, in particular a hydrocolloid fleece and/or hydrocolloid foam, preferably collagen fleece and/or collagen foam of porcine, bovine, and/or equine origin, more preferably a hydrocolloid fleece and/or hydrocolloid foam, preferably colloid fleece and/or colloid foam of porcine origin.

3. The wound dressing according to claim 1 or 2,
wherein the hydrocolloid - preferably collagen - layer (2) may be obtained by applying a dispersion or solution of a hydrocolloid, preferably a collagen, onto a support, followed by drying, in particular lyophilization (freeze-drying), preferably whilst expanding the hydrocolloid, preferably collagen; and/or
wherein the hydrocolloid - preferably collagen - of the hydrocolloid - preferably collagen - layer (2) is of porcine, bovine, and/or equine origin, preferably porcine origin, in particular of porcine skin, and/or
wherein the hydrocolloid - preferably collagen - layer (2) forms an outer layer of the wound dressing (1), and/or wherein the hydrocolloid - preferably collagen - layer (2) is arranged on the side of the wound dressing facing the wound to be treated during the application of the wound dressing (1); and/or
wherein the hydrocolloid - preferably collagen - layer (2) has a thickness in the range of 0.01 - 100 mm, in particular 0.02 - 50 mm, preferably 0.05 - 10 mm, and/or wherein the hydrocolloid - preferably collagen -layer (2) constitutes 5 .% - 95 %, in particular 10 % - 80 %, preferably 20 % - 60 % of the total thickness of the wound dressing.

4. The wound dressing according to any of the preceding claims,
wherein the active charcoal layer (3) comprises spherical active charcoal with absolute particle sizes in the range of 0.01 - 3 mm, in particular in the range of 0.02 - 2 mm, preferably 0.05 - 1.5 mm, more preferably 0.1 - 0.8 mm, most preferably 0.2 - 0.6 mm, and/or wherein the active charcoal layer (3) comprises spherical active charcoal with average particle sizes, determined in particular according to ASTM D2862-97/04, in the range of 0.05 - 2.5 mm, in particular in the range of 0.1 - 2 mm,
preferably 0.15 - 1 mm, more preferably 0.2 - 0.6 mm; and/or
wherein the active charcoal of the active charcoal layer (3) has a micropore volume proportion of at least 65 %, preferably at least 70 %, relative to the total pore volume of the active charcoal, formed by micropores having pore diameters ≤ 200 nm (20 Ǻ), and/or wherein the active charcoal of the active charcoal layer (3) has a micropore volume proportion in the range of 60 % - 95 %, in particular 65 % - 90 % preferably 70 % - 85 %, relative to the total pore volume of the active charcoal, formed by micropores having pore diameters ≤200 nm (20 A), and/or
wherein the active charcoal of the active charcoal layer (3) has a micropore volume proportion, in particular a micropore volume of carbon black, of at least 0.40 cm³/g, in particular at least 0.45 cm³/g, preferably at least 0.50 cm³/g, formed by micropores having pore diameters ≤ 200 nm (20 A), wherein the active charcoal of the active charcoal layer (3) has a micropore volume proportion, in particular a micropore volume of carbon black, in the range of 0.40 cm³/g - 2 cm³/g, in particular 0.45 cm³/g - 1.5 cm³/g, preferably 0.50 cm³/g - 1.2 cm³/g, formed by micropores having pore diameters ≤ 200 nm (20 Ǻ),

5. The wound dressing according to any of the preceding claims,
wherein the active charcoal of the active charcoal layer (3) has a micropore surface proportion of at least 50 %, in particular at least 60 %, preferably at least 70 %, most preferably at least 75 %, relative to the specific total surface area (BET) of the active charcoal, in particular one formed by micropores having pore diameters ≤ 200 nm (20 Ǻ),
wherein the active charcoal layer (3) has an internal surface area (BET) in the range of 500 - 3,000 m²/g, in particular in the range of 800 - 2,000 m²/g, preferably 900 - 1,800 m²/g, more preferably 1,000 - 1,600 m²/g; and/or
wherein the active charcoal of the active charcoal layer (3) has a total pore volume, in particular a Gurvich total pore volume, in the range of 0.1 - 4 cm³/g, in particular 0.2 - 3 cm³/g, preferably 0.3 - 2.5 cm³/g, more preferably 0.5 - 2 cm³/g.

6. The wound dressing according to any of the preceding claims,
wherein the active charcoal of the active charcoal layer (3) has a compressive and/or bursting strength, in particular a weight loading capacity per active charcoal particle, in particular per active charcoal grain or active charcoal bead, of at least 10 Newton, in particular at least 15 Newton, preferably at least 20 Newton, and/or wherein the active charcoal of the active charcoal layer (3) has a compressive and/or bursting strength, in particular a weight loading capacity per active charcoal particle, in particular per active charcoal grain or active charcoal bead, in the range of 10 - 50 Newton, in particular 12 - 45 Newton, preferably 15 - 40 Newton, and/or
wherein the active charcoal of the active charcoal layer (3) has a fractal open porosity dimension of at least 2.3, in particular at least 2.4, preferably at least 2.5,
more preferably at least 2.7, and/or wherein the active charcoal of the active charcoal layer (3) is at least substantially abrasion-resistant, and/or at least substantially dust-free,

7. The wound dressing according to any of the preceding claims,
wherein the active charcoal of the active charcoal layer (3) has a biocidal and/or biostatic, in particular antimicrobial effect and/or component,
in particular wherein the biocidal and/or biostatic, in particular antimicrobial, effect and/or component of the active charcoal is obtained by the production process for the active charcoal, in particular production by means of pyrolysis and subsequent activation of organic polymers, in particular due to the resultant surface charge and/or hydrophobicity and/or textural properties, and/or
in particular wherein the biocidal and/or biostatic, in particular antimicrobial effect and/or component of the active charcoal is obtained by providing the active charcoal,
in particular by impregnation, with at least one biocidal and/or biostatic, in particular antimicrobial active ingredient.

8. The wound dressing according to any of the preceding claims,
wherein the active charcoal of the active charcoal layer (3) is present in an amount, in particular a coating quantity, of 1 - 1,000 g/m², in particular 5 - 500 g/m², preferably 10 - 400 g/m², preferably 20 - 300 g/m², more preferably 25 - 250 g/m²; and/or wherein the active charcoal of the active charcoal layer (3) is arranged on a flat, preferably textile, support (4a, 4b), preferably attached thereto, and/or wherein the active charcoal of the active charcoal layer (3) is arranged on a three-dimensional,
preferably porous and/or textile support (4a, 4b), preferably a foam or foam material, preferably attached thereto or embedded therein, in particular wherein the three-dimensional support (4a, 4b) is based on an elastomeric resin and/or a polyurethane.

9. The wound dressing according to any of the preceding claims,
wherein the active charcoal of the active charcoal layer (3) is arranged between a first textile surface material (4a) and a second textile surface material (4b);
in particular wherein the first textile surface material (4a) and/or the second textile surface material (4b) is formed as a fabric, mesh, knit, mat, textile composite, fleece, or filament fiber material, in particular as a filament fiber material; and/or
wherein the first textile surface material (4a) and/or the second textile surface material (4b) based on a fiber type selected from the group of polyesters (PES); polyolefins, in particular polyethylene (PE) and/or polypropylene (PP), polyvinyl chloride (PVC); polyvinylidene chloride (PVDC); cellulose acetate (CA); cellulose triacetate (CTA); polyacrylic (PAN); polyamide (PA); polyvinyl alcohol (PVAL); polyurethanes (PU); polyvinylesters; (meth-)acrylates, as well as mixtures thereof, in particular cellulose acetate and/or polyamide; and/or
wherein the first textile surface material (4a) and/or the second textile surfaces material (4b) is formed such that it may release at least substantially no fibres and/or at least substantially no active charcoal; and/or
in particular wherein the active charcoal of the active charcoal layer (3) is attached to the first textile surface material (4a) and/or the second textile surface material (4b), in particular by means of a preferably medically and/or physiologically compatible adhesive, in particular wherein the adhesive is applied discontinuously and/or in a point-like manner on the first and/or second textile surface material (4a, 4b); and/or in particular wherein the adhesive is applied to the first and/or second textile surface material (4a, 4b) in an amount of 1 - 100 g/m², in particular 5 - 50 g/m², preferably 10 - 40 g/m²; and/or in particular wherein the adhesive covers the first and/or second textile surface material (4a, 4b) respectively in an amount of no more than 70 %, in particular no more than 60 %, preferably no more than 50 %, more preferably no more than 40 %, most preferably no more than 30 %, and/or wherein at least 50 %, in particular at least 60 %, preferably at least 70 % of the surface of the active charcoal is not covered with adhesive and/or is freely accessible.

10. The wound dressing according to any of the preceding claims,
wherein the active charcoal of the active charcoal layer (3) is a self-supporting layer, in particular an active charcoal fibre fabric or a self-supporting, flat or three-dimensional, preferably cohesive structure of granular, in particular spherical, active charcoal particles that are connected and/or attached to one another; and/or wherein the active charcoal of the active charcoal layer (3) is embedded in the hydrocolloid - preferably collagen -layer (2), or is deposited on and/or affixed to the hydrocolloid - preferably collagen - layer (2).

11. The wound dressing according to any of the preceding claims,
wherein at least 50 %, in particular at least 60 %, preferably at least 70 % of the surface of the active charcoal of the active charcoal layer (3) is not covered with adhesive and/or is freely accessible,
wherein the individual layers (2, 3, 4a, 4b) of the wound dressing (1) are each connected with one another and/or wherein the individual layers (2, 3, 4a, 4b) of the wound dressing (1) form a composite.

12. The wound dressing according to any of the preceding claims,
wherein the wound dressing (1) additionally contains at least one active ingredient, in particular one selected from the group of antimicrobials, disinfectants, anti-inflammatory agents, styptic agents, and wound-healing agents; and/or
wherein the wound dressing (1) is additionally provided with at least one antimicrobial and/or disinfectant and/or anti-inflammatory and/or styptic and/or wound-healing agent, and/or wherein the wound dressing (1) has at least one antimicrobial and/or disinfectant and/or anti-inflammatory and/or styptic and/or wound-healing agent.

13. The wound dressing according to claim 12,
wherein the active ingredient has a biocidal and/or biostatic effect, in particular bactericidal or bacteriostatic and/or fungicidal or fungistatic and/or antiviral effect; and/or
wherein the active ingredient is an antiseptic and/or disinfectant, and/or wherein the active ingredient is selected from the group of polyhexamethylene biguanide (polyhexanide), taurolidine, benzalkonium chloride, chlorhexidine, octenidine, and their physiologically acceptable salts, as well as mixtures thereof, preferably octenidine and/or polyhexamethylene biguanide (polyhexanide).

14. The wound dressing according to claim 12 or 13,
wherein the active ingredient is an active ingredient with wound-healing effects, in particular one selected from the group of alginates, chitosan, hyaluronic acid and salts thereof, allantoin, beta-sitosterol, bromelain, dexpanthenol, pantothenic acid, urea, flavonoids, riboflavin, saponins, cineol, tocopherol, and mixtures thereof; and/or wherein the active ingredient is present in the wound dressing (1) in an amount, in particular a coating quantity, of 0.00001 - 5 g/cm², in particular 0.0001 - 2 g/cm², preferably 0.001 - 1 g/cm², more preferably 0.01 - 0.5 g/cm²; and/or
wherein the active ingredient is present in the hydrocolloid layer (2) and/or the active charcoal layer (3), preferably present in the hydrocolloid layer (2) and the active charcoal layer (3).

15. The wound dressing according to any of the preceding claims for use in therapeutic, in particular topical, wound care, in particular for use in therapeutic, preferably topical, treatment of wounds and/or tissue disruptions;
in particular for use in the therapeutic treatment of mechanical wounds, in particular cuts, punctures, contusions, lacerations, scratches, and/or abrasions, or
in particular for use in the therapeutic treatment of thermal wounds, in particular wounds caused by cryogenic or thermal burns, or
in particular for use in the therapeutic treatment of chemical wounds, in particular wounds caused by burns with lyes and/or acids, or
in particular for use in the therapeutic treatment of wounds caused by radiation, in particular wounds caused by actinic and/or ionizing radiation, or
in particular for use in the therapeutic treatment of necrotizing and/or infected and/or chronic wounds, or acute wounds, or
in particular for use in the therapeutic treatment of bedsores or wounds caused circulatory disorders.

## Revendications

1. Pansement (1), notamment pour le soin thérapeutique de plaies, le pansement (1) présentant une structure multicouche, la structure multicouche comprenant au moins une couche (2) contenant au moins un hydrocolloïde, de préférence du collagène (« couche d'hydrocolloïde » ou « couche de collagène »), et au moins une couche (3) contenant du charbon actif (« couche de charbon actif »), dans lequel la couche (3) contenant du charbon actif comprend un charbon actif sphérique et
dans lequel le charbon actif de la couche (3) contenant du charbon actif présente une proportion en volume de micropores formés par les micropores de diamètres de pores ≤ 200 nm (20 A), par rapport au volume poreux total du charbon actif, d'au moins 60 %.

2. Pansement selon la revendication 1, dans lequel l'hydrocolloïde de la couche (2) contenant un hydrocolloïde est choisi dans le groupe des polysaccharides et des protéines, notamment des polysaccharides et des protéines végétaux, animaux ou bactériens, de préférence dans le groupe constitué par le collagène, la cellulose et les dérivés de cellulose, les glycosaminoglycanes, les pectines, la gomme arabique, les galactomannanes, l'agar-agar, le carraghénane, les alginates, la gélatine, les caséinates, les xanthanes, les dextranes et les scléroglucanes, et est de manière particulièrement préférée le collagène, l'acide hyaluronique et/ou ses sels et/ou la gélatine, de manière tout particulièrement préférée le collagène ; et/ou la couche (2) contenant un hydrocolloïde, de préférence du collagène, est à base d'un non-tissé d'hydrocolloïde et/ou d'une mousse d'hydrocolloïde, de préférence d'un non-tissé de collagène et/ou d'une mousse de collagène, et notamment à base d'un non-tissé d'hydrocolloïde et/ou d'une mousse d'hydrocolloïde, de préférence d'un non-tissé de collagène et/ou d'une mousse de collagène, d'origine porcine, bovine et/ou équine, de manière particulièrement préférée à base d'un non-tissé d'hydrocolloïde et/ou d'une mousse d'hydrocolloïde, de préférence d'un non-tissé de collagène et/ou d'une mousse de collagène, d'origine porcine, et/ou la couche (2) contenant un hydrocolloïde, de préférence du collagène, est formée par un non-tissé d'hydrocolloïde et/ou une mousse d'hydrocolloïde, de préférence un non-tissé de collagène et/ou une mousse de collagène, notamment par un non-tissé d'hydrocolloïde et/ou une mousse d'hydrocolloïde, de préférence un non-tissé de collagène et/ou une mousse de collagène, d'origine porcine, bovine et/ou équine, de préférence par un non-tissé d'hydrocolloïde et/ou une mousse d'hydrocolloïde, de préférence un non-tissé de collagène et/ou une mousse de collagène, d'origine porcine.

3. Pansement selon la revendication 1 ou 2, dans lequel la couche (2) contenant un hydrocolloïde, de préférence du collagène, peut être obtenue par application d'une dispersion ou d'une solution d'un hydrocolloïde, de préférence de collagène, sur un support, et séchage ultérieur, notamment lyophilisation (séchage par congélation), de préférence avec expansion de l'hydrocolloïde, de préférence du collagène ; et/ou
dans lequel l'hydrocolloïde, de préférence le collagène, de la couche (2) contenant un hydrocolloïde, de préférence du collagène, est d'origine porcine, bovine et/ou équine, de préférence d'origine porcine, notamment issu de peau porcine ; et/ou dans lequel la couche (2) contenant un hydrocolloïde, de préférence du collagène, forme une couche extérieure du pansement (1), et/ou dans lequel la couche (2) contenant un hydrocolloïde, de préférence du collagène, est agencée à l'état d'utilisation du pansement (1) sur le côté du pansement orienté vers la plaie à traiter ; et/ou
dans lequel la couche (2) contenant un hydrocolloïde, de préférence du collagène, présente une épaisseur dans la plage allant de 0,01 à 100 mm, notamment de 0,02 à 50 mm, de préférence de 0,05 à 10 mm, et/ou dans lequel la couche (2) contenant un hydrocolloïde, de préférence du collagène, représente 5 % à 95 %, notamment 10 % à 80 %, de préférence 20 % à 60 %, de l'épaisseur totale du pansement.

4. Pansement selon l'une quelconque des revendications précédentes,
dans lequel la couche (3) contenant du charbon actif comprend un charbon actif sphérique de tailles de particules absolues dans la plage allant de 0,01 à 3 mm, notamment dans la plage allant de 0,02 à 2 mm, de préférence dans la plage allant de 0,05 à 1,5 mm, de manière particulièrement préférée dans la plage allant de 0,1 à 0,8 mm, de manière tout particulièrement préférée dans la plage allant de 0,2 à 0,6 mm, et/ou dans lequel la couche (3) contenant du charbon actif comprend un charbon actif sphérique de tailles de particules moyennes, notamment déterminées selon ASTM D2862-97/04, dans la plage allant de 0,05 à 2,5 mm, notamment dans la plage allant de 0,1 à 2 mm, de préférence dans la plage allant de 0,15 à 1 mm, de manière particulièrement préférée dans la plage allant de 0,2 à 0,6 mm ; et/ou dans lequel le charbon actif de la couche (3) contenant du charbon actif présente une proportion en volume de micropores formés par les micropores de diamètres de pores ≤ 200 nm (20 A), par rapport au volume poreux total du charbon actif, d'au moins 65 %, de préférence d'au moins 70 %, et/ou dans lequel le charbon actif de la couche (3) contenant du charbon actif présente une proportion en volume de micropores formés par les micropores de diamètres de pores ≤ 200 nm (20 A), par rapport au volume poreux total du charbon actif, dans la plage allant de 60 % à 95 %,
notamment dans la plage allant de 65 % à 90 %, de préférence dans la plage allant de 70 % à 85 % ; et/ou
le charbon actif de la couche (3) contenant du charbon actif présente un volume de micropores formés par les micropores de diamètres de pores ≤ 200 nm (20 Å),
notamment un volume de micropores selon Carbon Black, d'au moins 0,40 cm³/g, notamment d'au moins 0,45 cm³/g, de préférence d'au moins 0,50 cm³/g, et/ou dans lequel le charbon actif de la couche (3) contenant du charbon actif présente un volume de micropores formés par les micropores de diamètres de pores ≤ 200 nm (20 A), notamment un volume de micropores selon Carbon Black, dans la plage allant de 0,40 cm³/g à 2 cm³/g, notamment dans la plage allant de 0,45 cm³/g à 1,5 cm³/g, de préférence dans la plage allant de 0,50 cm³/g à 1,2 cm³/g.

5. Pansement selon l'une quelconque des revendications précédentes,
dans lequel le charbon actif de la couche (3) contenant du charbon actif présente une proportion de surface de micropores spécifique, notamment une proportion de surface de micropores formés par les pores de diamètres de pores ≤ 200 nm (20 A), d'au moins 50 %, notamment d'au moins 60 %, de préférence d'au moins 70 %, de manière tout particulièrement préférée d'au moins 75 %, par rapport à la surface spécifique totale (BET) du charbon actif ; et/ou
le charbon actif de la couche (3) contenant du charbon actif présente une surface intérieure (BET) dans la plage allant de 500 à 3 000 m²/g, notamment dans la plage allant de 800 à 2 000 m²/g, de préférence dans la plage allant de 900 à 1 800 m²/g, de manière particulièrement préférée dans la plage allant de 1 000 à 1 600 m²/g ; et/ou
le charbon actif de la couche (3) contenant du charbon actif présente un volume poreux total, notamment un volume poreux total selon Gurvich, dans la plage allant de 0,1 à 4 cm³/g, notamment dans la plage allant de 0,2 à 3 cm³/g, de préférence dans la plage allant de 0,3 à 2,5 cm³/g, de manière particulièrement préférée dans la plage allant de 0,5 à 2 cm³/g.

6. Pansement selon l'une quelconque des revendications précédentes,
dans lequel le charbon actif de la couche (3) contenant du charbon actif présente une résistance à la compression et/ou à l'éclatement, notamment une capacité portante en poids par particule de charbon actif, notamment par grain de charbon actif ou bille de charbon actif, d'au moins 10 Newton, notamment d'au moins 15 Newton, de préférence d'au moins 20 Newton, et/ou dans lequel le charbon actif de la couche (3) contenant du charbon actif présente une résistance à la compression et/ou à l'éclatement, notamment une capacité portante en poids par particule de charbon actif, notamment par grain de charbon actif ou bille de charbon actif, dans la plage allant de 10 à 50 Newton, notamment dans la plage allant de 12 à 45 Newton, de préférence dans la plage allant de 15 à 40 Newton ; et/ou dans lequel le charbon actif de la couche (3) contenant du charbon actif présente une dimension fractale de la porosité ouverte d'au moins 2,3, notamment d'au moins 2,4, de préférence d'au moins 2,5, de manière particulièrement préférée d'au moins 2,7, et/ou dans lequel le charbon actif de la couche (3) contenant du charbon actif est configuré au moins essentiellement sous forme résistante à l'abrasion et/ou au moins essentiellement sous forme sans poussière.

7. Pansement selon l'une quelconque des revendications précédentes,
dans lequel le charbon actif de la couche (3) contenant du charbon actif présente un effet et/ou un apprêt biocide et/ou biostatique, notamment antimicrobien, notamment dans lequel l'effet et/ou l'apprêt biocide et/ou biostatique, notamment antimicrobien, du charbon actif est obtenu par le procédé de fabrication du charbon actif, notamment la fabrication par pyrolyse et activation ultérieure de polymères organiques, notamment en conséquence de la charge de surface et/ou de l'hydrophobie et/ou des propriétés de texture ainsi générées, et/ou notamment dans lequel l'effet et/ou l'apprêt biocide et/ou biostatique, notamment antimicrobien, du charbon actif est obtenu par un apprêt, notamment par imprégnation, du charbon actif avec au moins un agent actif biocide et/ou biostatique, notamment antimicrobien.

8. Pansement selon l'une quelconque des revendications précédentes,
dans lequel le charbon actif de la couche (3) contenant du charbon actif est présent en une quantité, notamment une quantité de revêtement, de 1 à 1 000 g/m², notamment de 5 à 500 g/m², de préférence de 10 à 400 g/m², de préférence de 20 à 300 g/m², de manière particulièrement préférée de 25 à 250 g/m²; et/ou dans lequel le charbon actif de la couche (3) contenant du charbon actif est agencé sur un support textile plat, de préférence textile (4a, 4b), de préférence fixé sur celui-ci, et/ou dans lequel le charbon actif de la couche (3) contenant du charbon actif est agencé sur un support tridimensionnel, de préférence poreux et/ou textile (4a, 4b),
de préférence une mousse ou une matière moussée, de préférence fixé sur celui-ci et/ou incorporé dans celui-ci, le support tridimensionnel (4a, 4b) étant notamment à base d'une résine élastomère et/ou à base d'un polyuréthane.

9. Pansement selon l'une quelconque des revendications précédentes,
dans lequel le charbon actif de la couche (3) contenant du charbon actif est agencé entre un premier matériau de surface textile (4a) et un second matériau de surface textile (4b) ;
notamment dans lequel le premier matériau de surface textile (4a) et/ou le second matériau de surface textile (4b) sont configurés sous la forme d'un tissu, d'un tricot, d'une nappe, d'un composite textile, d'un non-tissé ou d'un matériau fibreux filamentaire, notamment sous la forme d'un matériau fibreux filamentaire ; et/ou dans lequel le premier matériau de surface textile (4a) et/ou le second matériau de surface textile (4b) sont à base d'un type de fibre choisi dans le groupe constitué par les polyesters (PES) ; les polyoléfines, notamment le polyéthylène (PE) et/ou le polypropylène (PP), le polychlorure de vinyle (PVC) ; le polychlorure de vinylidène (PVDC) ; l'acétate de cellulose (CA) ; le triacétate de cellulose (CTA) ; le polyacryle (PAN) ; le polyamide (PA) ; l'alcool polyvinylique (PVAL) ; les polyuréthanes (PU) ; les polyesters de vinyle ; les (méth)acrylates ; ainsi que leurs mélanges, notamment l'acétate de cellulose et/ou le polyamide ; et/ou
dans lequel le premier matériau de surface textile (4a) et/ou le second matériau de surface textile (4b) sont configurés de manière à ce qu'au moins essentiellement aucune fibre et/ou au moins essentiellement aucun charbon actif ne puisse être libéré ; et/ou
notamment dans lequel le charbon actif de la couche contenant du charbon actif (3) est fixé au premier matériau de surface textile (4a) et/ou au second matériau de surface textile (4b), notamment au moyen d'un adhésif de préférence médicalement et/ou physiologiquement compatible, l'adhésif étant notamment appliqué de manière discontinue et/ou ponctuelle sur le premier et/ou le second matériau de surface textile (4a, 4b), et/ou l'adhésif étant appliqué sur le premier et/ou le second matériau de surface textile (4a, 4b) en une quantité de revêtement de 1 à 100 g/m², notamment de 5 à 50 g/m², de préférence de 10 à 40 g/m², et/ou l'adhésif recouvrant respectivement le premier et/ou le second matériau de surface textile (4a, 4b) à hauteur d'au plus 70 %, notamment à hauteur d'au plus 60 %, de préférence à hauteur d'au plus 50 %, de préférence à hauteur d'au plus 40 %, de manière particulièrement préférée à hauteur d'au plus 30 %, et/ou la surface du charbon actif étant de préférence non recouverte avec l'adhésif et/ou étant librement accessible à hauteur d'au moins 50 %, notamment à hauteur d'au moins 60 %, de préférence à hauteur d'au moins 70 %.

10. Pansement selon l'une quelconque des revendications précédentes,
dans lequel le charbon actif de la couche (3) contenant du charbon actif se présente sous la forme d'une couche autoportante, notamment sous la forme d'une structure plate fibreuse de charbon actif ou sous la forme d'une structure autoportante, plate ou tridimensionnelle, de préférence cohérente, de particules de charbon actif granuleuses, notamment sphériques, reliées les unes aux autres et/ou fixées les unes aux autres ; et/ou
dans lequel le charbon actif de la couche (3) contenant du charbon actif est incorporé dans la couche (2) contenant un hydrocolloïde, de préférence du collagène, et/ou superposé et/ou fixé sur la couche (2) contenant un hydrocolloïde, de préférence du collagène.

11. Pansement selon l'une quelconque des revendications précédentes,
dans lequel la surface du charbon actif de la couche (3) contenant du charbon actif est librement accessible et/ou est non recouverte à hauteur d'au moins 50 %, notamment à hauteur d'au moins 60 %, de préférence à hauteur d'au moins 70 % ; et/ou
dans lequel les couches individuelles (2, 3, 4a, 4b) du pansement (1) sont chacune reliées les unes avec les autres, et/ou dans lequel les couches individuelles (2, 3, 4a, 4b) du pansement (1) forment un composite.

12. Pansement selon l'une quelconque des revendications précédentes,
dans lequel le pansement (1) contient en outre au moins un agent actif, notamment choisi dans le groupe constitué par les agents actifs à effet antimicrobien, les agents actifs désinfectants, les agents actifs anti-inflammatoires, les agents actifs hémostatiques et les agents actifs favorisant la cicatrisation ; et/ou dans lequel le pansement (1) est en outre apprêté avec au moins un agent actif antimicrobien et/ou désinfectant et/ou anti-inflammatoire et/ou hémostatique et/ou favorisant la cicatrisation, et/ou dans lequel le pansement (1) comprend au moins un agent actif antimicrobien et/ou désinfectant et/ou anti-inflammatoire et/ou hémostatique et/ou favorisant la cicatrisation.

13. Pansement selon la revendication 12,
dans lequel l'agent actif a un effet biocide et/ou biostatique, notamment un effet bactéricide ou bactériostatique et/ou fongicide ou fongistatique et/ou virucide ou virustatique ; et/ou
dans lequel l'agent actif est un antiseptique et/ou un désinfectant, et/ou dans lequel l'agent actif est choisi dans le groupe constitué par le polyhexaméthylène-biguanide (polyhexanide), la taurolidine, le chlorure de benzalkonium, la chlorhexidine, l'octénidine et leurs sels physiologiquement acceptables, ainsi que leurs mélanges, de préférence parmi l'octénidine et/ou le polyhexaméthylène-biguanide (polyhexanide).

14. Pansement selon la revendication 12 ou 13,
dans lequel l'agent actif est un agent actif à effet favorisant la cicatrisation, notamment choisi dans le groupe constitué par les alginates, le chitosan, l'acide hyaluronique et ses sels, l'allantoïne, le bêta-sitostérol, la bromélaïne, le dexpanthénol, l'acide pantothénique, l'urée, les flavonoïdes, la ribflavine, les saponines, le cinéol, le tocophérol et leurs mélanges ; et/ou
dans lequel l'agent actif est présent dans le pansement (1) en une quantité, notamment une quantité de revêtement, de 0,00001 à 5 g/cm², notamment de 0,0001 à 2 g/cm², de préférence de 0,001 à 1 g/cm², de manière particulièrement préférée de 0,01 à 0,5 g/cm² ; et/ou
dans lequel l'agent actif est présent dans la couche (2) contenant un hydrocolloïde et/ou dans la couche (3) contenant du charbon actif, l'agent actif étant de préférence présent dans la couche (2) contenant un hydrocolloïde et dans la couche (3) contenant du charbon actif.

15. Pansement selon l'une quelconque des revendications précédentes, destiné à une utilisation pour le soin thérapeutique des plaies, notamment topique, notamment destiné à une utilisation pour le traitement thérapeutique de plaies et de coupures de tissus, de préférence topique ;
notamment destiné à une utilisation pour le traitement thérapeutique de plaies mécaniques, notamment d'entailles, de blessures pénétrantes, de contusions, de plaies par rupture, d'écorchures et/ou d'abrasions ; ou
notamment destiné à une utilisation pour le traitement thérapeutique de plaies thermiques, notamment de plaies causées par une brûlure à froid ou à chaud ; ou notamment destiné à une utilisation pour le traitement thérapeutique de plaies chimiques, notamment de plaies causées par brûlure avec des bases et/ou des acides ; ou
notamment destiné à une utilisation pour le traitement thérapeutique de plaies causées par des rayonnements, notamment de plaies causées par un rayonnement actinique et/ou ionisant ; ou
notamment destiné à une utilisation pour le traitement thérapeutique de plaies nécrosantes et/ou infectées et/ou chroniques, ou de plaies aigues ;
notamment destiné à une utilisation pour le traitement thérapeutique d'escarres et/ou de plaies causées par des troubles de l'irrigation sanguine.
